# EUROPÄISCHE PATENTANMELDUNG

(11) **EP 0 567 997 A1**
(43) Veröffentlichungstag der Anmeldung: **03.11.1993**
(21) Anmeldenummer: 93106797.9
(22) Anmeldetag: 27.04.1993
(51) Int. Cl.: C07D 213/76, A61K 31/44, C07D 213/81

(54) **4- oder 5-(Sulf)imido- und (Sulfon)amidopyridine, sowie ihre Pyridin-N-oxide, Verfahren zu ihrer Herstellung und ihre Verwendung als Arzneimittel**

(30) Priorität: 30.04.1992 DE 4214465; 24.07.1992 DE 4224440
(71) Anmelder: HOECHST AKTIENGESELLSCHAFT, D-65926 Frankfurt am Main (DE)
(72) Erfinder: Weidmann, Klaus, Dr., W-6242 Kronberg/Ts. (DE); Bickel, Martin, Dr., W-6380 Bad Homburg (DE); Günzler-Pukall, Volkmar, Dr., W-3550 Marburg (DE)

(57) **Zusammenfassung**

Es werden neue 4- oder 5-(Sulf)imido- und (Sulfon)amidopyridine und die entsprechenden Pyridin-N-oxide gemäß Formel I beschrieben. Ebenso umfaßt die Erfindung ein Verfahren zur Herstellung dieser Verbindungen sowie ihre Verwendung als Arzneimittel gegen fibrotische Erkrankungen.

## Beschreibung

Die Erfindung betrifft 4 - oder 5-(Sulf)imido - und (Sulfon)amidopyridine und ihre Pyridin-N-oxide sowie ihre Verwendung als Arzneimittel gegen fibrotische Erkrankungen.

Verbindungen, die die Enzyme Proin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird proteingebundenes Proin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazallulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des ertrazellulär abgelagerten Kollagens.

Inhibitoren der Prolylhydroxylase sind deshalb geeignete Substanzen in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma) sowie die Atherosklerose.

Es ist bekannt, daß das Enzym Prolinhydroxylase durch Pyridin-2,4- und -2,5-dicarbonsäure effektiv gehemmt wird (K. Majamaa et al., Eur. J. Biochem. 138 (1984)239-245). Diese Verbindungen sind in der Zellkultur allerdings nur in sehr hohen Konzentrationen als Hemmstoffe wirksam (Tschank, G. et al., Biochem. J. 238 (1987) 625-633). Im J. Med. Chem. 1992, 35, Seiten 800-804 werden 5-Sulfonylimide der Pyridin-2,5-dicarbonsäure beschrieben.

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Sauren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4- und -2,5-dicarbonsäure, die die Kollagenbiosynthese im Tiermodell wirksam hemmen.

Es bestand nunmehr die Aufgabe nach Verbindungen zu suchen, die stärker antifibrotisch sind als die bisher bekannten Verbindungen.

Gelöst wird die Aufgabe durch das Bereitstellen von 4- oder 5-(Sulf)imido- und (Sulfon)amidopyridinen und verwandten Verbindungen sowie ihrer Pyridin-N-oxide der allgemeinen Formel I 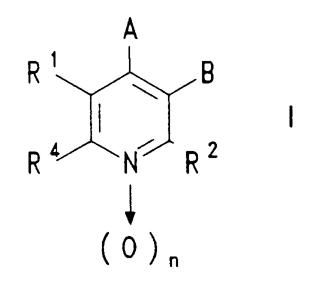
worin
A = R³ und B -XNR⁶R⁷ oder
B = R³ und A -XNR⁶R⁷ und
X eine Einfachbindung oder -CO-,
bedeutet und
- R¹, R² und R³: gleich oder verschieden sind und Wasserstoff, unsubstituiertes oder substituiertes (C₁-C₆)-Akyl, (C₁-C₆)-Alkoxy, Halogen, insbesondere Fluor, Chlor oder Brom, Nitril, Hydroxy, Amino,
- R⁶: Wasserstoff, (C₁-C₂₀)-Alkyl oder eine N-Schutzgruppe wie Acyl, (C₁-C₂₀)-Alkanoyl, (C₁-C₂₀)-Alkenoyl, (C₁-C₂₀)-Alkinoyl, (C₇-C₂₀)-Aralkanoyl, (C₆-C₁₈)-Aroyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein physiologisch verwendbares Kation, wie Na⊕, K⊕, Mg²⊕, Ca²⊕, Al³⊕ oder ein Ammoniumion, ggf. 1-3 fach substituiert mit (C₁-C₁₆)Alkyl, (C₁-C₁₆)-Hydroxyalkyl, (C₁-C₈)-Alkoxy-(C₁-C₁₆)-alkyl, Phenyl, Benzyl oder (C₁-C₁₆)-Alkyl, welches 1 bis 3-fach substituiert sein kann mit Hydroxy oder (C₁-C₈)-Alkoxy, oder ein Kation eines basischen Aminosäurederivates, wobei ein vorhandener Arylteil gegebenenfalls substituiert sein kann,
- R⁷: einen Rest Y R⁸ bedeutet,
in welchem
- Y: -SO₂-, -CO-, -CONR⁹ oder -SO₂NR⁹
und
- R⁸ und R⁹: [C-U]ᵣ-D-W bedeuten, worin
- C: eine Bindung oder
einen verzweigten oder unverzweigten aliphatischen (C₁-C₁₆)-Alkandiyl oder cycloaliphatischen (C₃-C₁₀)-Alkandiylrest oder
einen verzweigten oder unverzweigten (C₂-C₁₆)-Alkendiyl- oder
Cycloalkendiyl-Rest, oder einen (C₂-C₁₆)-Alkindiyl-Rest oder einen (C₂-C₁₆)-Alkenindiyl-Rest bedeutet, der jeweils eine oder mehrere C-C-Mehrfachbindungen enthalten kann,
- U: eine Bindung oder
Wasserstoff oder
einen Rest aus der Reihe folgender Heteroatomgruppierungen -CO-, -O(CO)-, -(CO)-O-, -(CO)NR-, -NR(CO)-, -O-, -SO-, -SO₂-, -NR, worin R (C₁-C₃)Alkyl, (C₁-C₈)-Alkanoyl, (C₇-C₁₆)-Aralkanoyl, (C₆-C₁₂)-Aroyl oder Wasserstoff bedeutet,
- r: 1, 2, 3 oder 4 ist,
- D: eine Bindung oder Wasserstoff oder
einen verzweigten oder unverzweigten aliphatischen (C₁-C₁₀)-Alkandiyl-Rest, oder
einen verzweigten oder unverzweigten (C₁-C₁₀)-Alkendiyl-Rest, einen (C₂-C₁₀)-Alkindiyl-Rest oder einen (C₂-C₁₀)-Alkenindiyl-Rest, der jeweils eine oder mehrere C-C-Mehrfachbindungen enthalten kann,
- W: eine Bindung oder
Wasserstoff oder
einen (C₃-C₁₀) cycloaliphatischen Alkyl-, Alkenyl-, Alkinyl- oder Alkeninyl-Rest oder
einen (C₆-C₁₆)-Arylrest oder einen 5- oder 6-gliedrigen Heteroarylrest,
wobei mindestens eine der Variablen C oder D oder W nicht eine Bindung bedeuten und U nur dann in der Bedeutung einer Heteratomgruppierung steht, wenn C nicht eine Bindung bedeutet und
- C, D und/oder W,: sofern diese keine Bindung oder Wasserstoff bedeuten und
vorzugsweise ihrerseits substituiert sind
oder
- R⁸ und R⁹: auch über 3-6 C-Atome verknüpft sind, wobei -NR⁸R⁹ einen heterocyclischen Ring bilden, mit N als Heteroatom,
oder
- R⁹: in der Bedeutung von R⁶ steht,
und
für [C-U]ᵣ-D-W die Bedeutung -SO₂H ausgenommen ist, sowie
- R⁴: einen Rest bedeutet, der physiologisch, insbesondere in der Leber, in eine Carboxylatgruppe oder deren Salze umgewandelt werden kann, wobei Ester und Amide ausgenommen sind, und
- n: 0 oder 1,
- f: 1 bis 8, vorzugsweise 1 bis 5,
- g: 0, 1 bis (2f+ 1) und
- x: 0 bis 8, vorzugsweise 0 oder 1 bedeuten.

Bevorzugt sind Verbindungen der Formel I, in denen
- X: eine Einfachbindung oder -CO-,
- R¹, R² und R³: gleich oder verschieden sind und Wasserstoff, (C₁-C₆)-Alkyl, (C₁-C₆)-Alkoxy, Halogen, insbesondere Fluor, Chlor oder Brom, Nitril, Hydroxy, Amino, ggf. mono oder disubstituiert mit (C₁-C₄)-Alkyl, Hydroxy-(C₁-C₄)-alkyl, (C₁-C₆)-Alkylcarbonyloxy,
- R⁶: Wasserstoff, (C₁-C₂₀)-Alkyl oder eine N-Schutzgruppe wie Acyl, (C₁-C₂₀)-Alkanoyl, (C₁-C₂₀)-Alkenoyl, (C₁-C₂₀)-Alkinoyl, (C₇-C₂₀)-Aralkanoyl, (C₆-C₁₈)-Aroyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein physiologisch verwendbares Kation, wie Na⊕, K⊕, Mg²⊕, Ca²⊕, Al³⊕ oder ein Ammoniumion, ggf. 1-3fach substituiert mit (C₁-C₁₆)Alkyl, (C₁-C₁₆)-Hydroxyalkyl, (C₁-C₈)-Alkoxy-(C₁-C₁₆)-alkyl, Phenyl, Benzyl oder (C₁-C₁₆)-Alkyl, welches 1 bis 3-fach substituiert sein kann mit Hydroxy oder (C₁-C₈)-Alkoxy, oder ein Kation eines basischen Aminosäurederivates, wobei ein vorhandener Arylteil gegebenenfalls substituiert sein kann,
- R⁷: einen Rest Y R⁸ bedeutet,
in welchem
- Y: -SO₂-, -CO-, -CONR⁹ oder -SO₂NR⁹
und
- R⁸ und R⁹: [C-U]ᵣ-D-W bedeuten, worin
- C: eine Bindung oder
einen verzweigten oder unverzweigten aliphatischen (C₁-C₁₆)-Alkandiyl oder cycloaliphatischen (C₃-C₁₀)-Alkandiylrest oder
einen verzweigten oder unverzweigten (C₂-C₁₆)-Alkendiyl- oder Cycloalkendiyl-Rest, oder einen (C₂-C₁₆)-Alkindiyl-Rest oder einen (C₂-C₁₆)-Alkenindiyl-Rest bedeutet, der jeweils eine oder mehrere C-C-Mehrfachbindungen enthalten kann,
- U: eine Bindung oder
Wasserstoff oder
einen Rest aus der Reihe folgender Heteroatomgruppierungen
-CO-, -O(CO)-, -(CO)-O-, -(CO)NR-, -NR(CO)-, -O-, -SO-, -SO₂-, -NR, worin R (C₁-C₃)Alkyl, (C₁-C₈)-Alkanoyl, (C₇-C₁₆)-Aralkanoyl, (C₆-C₁₂)-Aroyl oder Wasserstoff bedeutet,
- r: 1, 2, 3 oder 4 ist,
- D: eine Bindung oder Wasserstoff oder
einen verzweigten oder unverzweigten aliphatischen (C₁-C₁₀)-Alkandiyl-Rest, oder
einen verzweigten oder unverzweigten (C₁-C₁₀)-Alkendiyl-Rest, einen (C₂-C₁₀)-Alkindiyl-Rest oder einen (C₂-C₁₀)-Alkenindiyl-Rest, der jeweils eine oder mehrere C-C-Mehrfachbindungen enthalten kann,
- W: Wasserstoff oder
einen (C₃-C₁₀) cycloaliphatischen Alkyl-, Alkenyl-, Alkinyl- oder Alkeninyl-Rest oder
einen (C₆-C₁₆)-Arylrest oder einen 5- oder 6-gliedrigen Heteroarylrest, wobei mindestens eine der Variablen C oder D oder W nicht eine Bindung bedeuten und U nur dann in der Bedeutung einer Heteratomgruppierung steht, wenn C nicht eine Bindung bedeutet und
- C, D und/oder W,: sofern diese keine Bindung oder Wasserstoff bedeuten, vorzugsweise ihrerseits substituiert sind durch eine Kombination von bis zu 5 gleichen oder verschiedenen Substituenten aus der Reihe Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, - OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂) Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂)-Alkinyloxycarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)alkyl)carbamoyl,
N-((C₆-C₁₆)-Aryloxy-(C₁-C₁₀)alkyl)carbamoyl,
N-((C₇-C₁₆)-Aralkyloxy-(C₁-C₁₀)alkyl)carbamoyl,
N-(C₁-C₁₀)-Alkyl-N-((C₁-C₁₀)-Alkoxy-(C₁-C₁₀)alkyl)carbamoyl,
N-(C₁-C₁₀)-Alkyl-N-((C₆-C₁₆)-Aryloxy-(C₁-C₁₀)alkyl)carbamoyl,
N-(C₁-C₁₀)-Alkyl-N-((C₇-C₁₆)-Aralkyloxy-(C₁-C₁₀)alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)arylcarbamoyloxy, N-(C₁-C₁₀)-Alkyl-N-(C₇-C₁₆)-Aralkylcarbamoyloxy
N-((C₁-C₁₀)-Alkoxy-(C₁-C₁₀)alkyl)carbamoyloxy,
N-((C₆-C₁₆)-Aryloxy-(C₁-C₁₀)alkyl)carbamoyloxy,
N-((C₇-C₁₆)-Aralkyloxy-(C₁-C₁₀)alkyl)carbamoyloxy,
N-(C₁-C₁₀)-Alkyl-N-((C₁-C₁₀)Alkoxy-(C₁-C₁₀)alkyl)carbamoyloxy,
N-(C₁-C₁₀)-Alkyl-N-((C₆-C₁₆)-Aryloxy-(C₁-C₁₀)alkyl)carbamoyloxy,
N-(C₁-C₁₀)-Alkyl-N-((C₇-C₁₆)-Aralkyloxy-(C₁-C₁₀)alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-(C₁-C₁₀)Alkyl-(C₇-C₁₀)Aralkylamino, N-(C₁-C₁₀)Alkyl-N(C₆-C₁₂)Arylamino, (C₁-C₁₂)-Alkoxy-amino, (C₁-C₁₂)-Alkoxy-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)alkylamino-(C₁-C₁₀)alkyl, N,N-Di(C₁-C₁₀)alkylamino-(C₁-C₁₀)alkyl, (C₃-C₈)cycloalkylamino-(C₁-C₁₀)alkyl,
(C₁-C₁₂)-Alkylmercapto, (C₁-C₁₂)-Alkylsulfinyl, (C₁-C₁₂)-Alkylsulfonyl,
(C₆-C₁₆)-Arylmercapto, (C₆-C₁₆)-Arylsulfinyl, (C₆-C₁₆)-Arylsulfonyl,
(C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl,(C₃-C₈)-cycloalkylsulfamoyl,
N-(C₆-C₁₆)-Arylsulfamoyl,
N-(C₇-C₁₆)-Aralkylsulfamoyl,
N-(C₁-C₁₀)-Alkyl-N-(C₆-C₁₆)-Arylsulfamoyl,
N-(C₁-C₁₀)-Alkyl-N-(C₇-C₁₆)-Aralkylsulfamoyl,
(C₁-C₁₀)-Alkyl-sulfonamido,
N-((C₁-C₁₀)alkyl)-(C₁-C₁₀)alkylsulfonamido, (C₇-C₁₆)-Aralkylsulfonamido,
N-((C₁-C₁₀)alkyl-(C₇-C₁₆)-Aralkylsulfonamido,
wobei die Reste, die einen Arylrest enthalten, ihrerseits am Aryl substituiert sein können mit 1, 2, 3, 4 oder 5 gleichen oder verschiedenen Substituenten aus der Reihe, Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂) Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂)-Alkinyloxycarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)alkyl)carbamoyl,
N-((C₆-C₁₆)-Aryloxy-(C₁-C₁₀)alkyl)carbamoyl,
N-((C₇-C₁₆)Aralkyloxy-(C₁-C₁₀)alkyl)carbamoyl,
N-(C₁-C₁₀)-Alkyl-N-((C₁-C₁₀)-Alkoxy-(C₁-C₁₀)alkyl)carbamoyl,
N-(C₁-C₁₀)-Alkyl-N-((C₆-C₁₆)-Aryloxy-(C₁-C₁₀)alkyl)carbamoyl,
N-(C₁-C₁₀)-Alkyl-N-((C₇-C₁₆)-Aralkyloxy-(C₁-C₁₀)alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)arylcarbamoyloxy, N-(C₁-C₁₀)-Alkyl-N-(C₇-C₁₆)-Aralkylcarbamoyloxy N-((C₁-C₁₀)-Alkoxy-(C₁-C₁₀)alkyl)carbamoyloxy,
N-((C₆-C₁₆)-Aryloxy-(C₁-C₁₀)alkyl)carbamoyloxy,
N-((C₇-C₁₆)-Aralkyloxy-(C₁-C₁₀)alkyl)carbamoyloxy,
N-(C₁-C₁₀)-Alkyl-N-((C₁-C₁₀)-Alkoxy-(C₁-C₁₀)alkyl)carbamoyloxy,
N-(C₁-C₁₀)-Alkyl-N-((C₆-C₁₆)-Aryloxy-(C₁-C₁₀)alkyl)carbamoyloxy,
N-(C₁-C₁₀)-Alkyl-N-((C₇-C₁₆)-Aralkyloxy-(C₁-C₁₀)alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-(C₁-C₁₀)-Alkyl-N-(C₇-C₁₆)-Aralkylamino, N-(C₁-C₁₀)-Alkyl-N-(C₆-C₁₂)-Arylamino, (C₁-C₁₂)-Alkoxy-amino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)alkylamino-(C₁-C₁₀)alkyl, N,N-Di(C₁-C₁₀)alkylamino-(C₁-C₁₀)alkyl, (C₃-C₈)cycloalkylamino-(C₁-C₁₀)alkyl,
(C₁-C₁₂)-Alkylmercapto, (C₁-C₁₂)-Alkylsuffinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl,(C₃-C₈)-cycloalkylsulfamoyl,
N-(C₆-C₁₆)-Arylsulfamoyl,
N-(C₇-C₁₆)-Aralkylsulfamoyl,
N-(C₁-C₁₀)-Alkyl-N-(C₆-C₁₆)-Arylsulfamoyl,
N-(C₁-C₁₀)-Alkyl-N-(C₇-C₁₆)-Aralkylsulfamoyl,
(C₁-C₁₀)-Alkyl-sulfonamido,
N-((C₁-C₁₀)alkyl)-(C₁-C₁₀)alkylsulfonamido, (C₇-C₁₆)-Aralkylsulfonamido,
N-((C₁-C₁₀)alkyl-(C₇-C₁₆)-Aralkylsulfonamido,
oder
R⁸ und R⁹ auch über 3 - 6 C-Atome verknüpft sind, wobei -NR⁸R⁹ einen heterocyclischen Ring bilden, mit N als Heteroatom,
oder
- R⁹: in der Bedeutung von R⁶ steht,
und für [C-U]ᵣ-D-W die Bedeutung -SO₂H ausgenommen ist, sowie
- R⁴: eine (C₁-C₁₀)-Alkylgruppe, die gegebenenfalls mit Hydroxy, (C₁-C₆)-Alkoxy substituiert sein kann, Aldehyd-, Imino-, N(C₁-C₈)-Alkylimino-, Acetal-, ein Thioacetal, eine cyclische Acetalgruppe, eine cyclische Thioacetalgruppe oder eine andere der Aldehydgruppe äquivalente Gruppe oder - CH₂OR⁵ bedeutet, worin
- R⁵: Wasserstoff oder den Rest einer physiologisch vertraglichen, vorzugsweise in der Leber unter physiologischen Bedingungen abspaltbaren Alkoholschutzgruppe bedeutet,
und
- n: 0 oder 1,
- f: 1 bis 8, vorzugsweise 1 bis 5,
- g: 0, 1 bis (2f+ 1) und
- x: 0 bis 8, vorzugsweise 0 oder 1 bedeuten.

Unter Aryl-, Aryloxy-, Heteroaryl- bzw. Heteroaryloxyverbindungen versteht man insbesondere Phenyl-, Biphenyl- oder Naphthyl- bzw. unsubstituierte 5- und 6-gliedrige heteroaromatische Ringe mit 1, 2 oder 3 Stickstoff- und/oder Sauerstoff und/oder Schwefelatomen, wie Pyridyl-, Pyridazyl-, Pyrimidyl-, Pyrazyl-, Imidazolyl-, Triazolyl-, Thienyl-, Oxazolyl-, und Thiazolyl-Derivate, und deren benzoannellierte Derivate.

Als Alkohol-Schutzgruppe kommen insbesondere substituierte oder unsubstituierte Methylether, Ethylether, Benzylether, Silylether, Ester, Carbonate oder Sulfonate in Frage.

Hierunter fallen folgende Verbindungen:
Als substituierte Methylether:
Methoxymethyl, Methylthiomethyl, t-Butylthiomethyl,
(Phenyldimethylsilyl)methoxymethyl, Benzyloxymethyl, p-Methoxybenzyloxymethyl, (4-Methoxyphenoxy)-methyl, Guaiacolmethyl, t-Butoxymethyl, 4-Pentenyloxymethyl, Siloxymethyl, 2-Methoxyethoxymethyl, 2,2,2-Trichlorethoxymethyl, Bis(2-chlorethoxy)methyl, 2-(Trimethylsilyl)ethoxymethyl, Tetrahydropyranyl, 3-Bromotetrahydropyranyl, Tetrahydrothiopyranyl, 1-Methoxycyclohexyl, 4-Methoxytetrahydropyranyl, 4-Methoxytetrahydrothiopyranyl, 4-Methoxytetrahydrothiopyranyl-S,S-Dioxo, 1-[2-Chlor-4-methyl)-phenyl]-4-methoxypiperidin-4-yl, 1 ,4-Dioxan-2yl, Tetrahydrofuranyl, Tetrahydrothiofuranyl.

Als substituierte Ethylether:
1-Ethoxyethyl, 1-(2-Chlorethoxy)ethyl, 1-Methyl-1-methoxyethyl, 1-Methyl-1-benzyloxyethyl, 1-Methyl-1-benzyloxy-2-fluoroethyl, 2,2,2-Trichlorethyl, 2-Trimethylsilylethyl, 2-(Phenylselenyl)ethyl, t-Butyl, Allyl, p-Chlorphenyl, p-Methoxyphenyl, 2,4-Dinitrophenyl, Benzyl.

Als substituierte Benzylether:
p-Methoxybenzyl, 3,4-Dimethoxybenzyl, o-Nitrobenzyl, p-Nitrobenzyl, p-Halogenbenzyl, 2,6-Dichlorbenzyl, p-Cyanobenzyl, p-Phenylbenzyl, 2- und 4-Picolyl, 3-Methyl-2-picolyl-N-oxido, Diphenylmethyl, p,p'-Dinitrobenzhydryl, Triphenylmethyl, α-Naphthyldiphenylmethyl, p-Methoxyphenyldiphenylmethyl, Di(p-methoxyphenyl)-phenylmethyl, Tri(p-methoxyphenyl)methyl, 4-(4'-Bromphenacyloxy)phenyldiphenylmethyl, 4,4',4''-Tris (4,5-dichlorphthalimidophenyl)methyl, 4,4',4''-Tris(levulinooxyphenyl)methyl, 4,4',4''-Tris(benzoyloxyphenyl)methyl, 3-(Imidazol-1,4'-methyl)bis (4',4''-dimethoxyphenyl)-methyl, 1,1-Bis(4-methoxyphenyl)-1'-pyrenylmethyl, 9-Anthryl, 9-(9-Phenyl)xanthenyl, 9-(9-Phenyl-10-oxo)anthryl.

Als Silylether:
Trimethylsilyl, Triethylsilyl, Triisopropylsilyl, Dimethylisopropylsilyl, Diethylisopropylsilyl, Dimethylthexylsilyl, t-Butyldimethylsilyl, t-Butyldiphenylsilyl, Tribenzylsilyl, Tri-p-xylylsilyl, Triphenylsilyl, Diphenylmethylsilyl, t-Butylmethoxyphenylsilyl.

Als Ester:
Format, Benzoylformiat, Acetate, Propionat, Butyrat, Valeroat, Capronat, Heptanoat, Caprylat, Caprinat, Laurinat, Palimitat, Stearat, Chloroacetat, Dichloroacetat, Trichloroacetat, Trifluoroacetat, Methoxyacetat, Triphenylmethoxyacetat, Phenoxyacetat, p-Chlorphenoxyacetat, p-Cl-Phenylacetat, 3-Phenylpropionat, 4-Oxopentanoat (Levulinat), 4,4-(Ethylendithio)pentanoat, Pivaloat, Adamantoat, Crotonat, 4-Methoxycrotonat, Benzoat, p-Phenylbenzoat, 2,4,6-Trimethylbenzoat (Mesitoat), 2-Methylbenzoat, 2-Methylpropionat, 3-Methylbutyrat, 4-Methylvaleroat, 2-Ethylbutyrat, weitere Ester mit ein- oder mehrfach ungesättigten Fettsäuren, wie z.B. Ölsäure, Sorbinsäure, Linolsäure, Linolensäure, Ester mit Trolox, Gallensäuren, wie z.B. Cholsäure, Lithocholsäure, Chenodesoxycholsäure und Ursodesoxycholsäure.

Die Carbonsäure-Komponente kann auch weitere Carboxylgruppen enthalten. In diesem Fall sind ein oder mehrere Carboxylgruppen mit Resten der Formel I, in welcher R₄ = CH₂OH bedeutet, verestert. Beispiele für diese Di- und Polycarbonsäuren sind beispielsweise Oxalsäure, Malonsäure, Bernsteinsäure, Maleinsäure, Fumarsäure, Zitronensäure, Phthalsäure, Isophthalsäure, Terephthalsäure und Adipinsäure.

Als Carbonate:
Methyl, 9-Fluorenylmethyl, Ethyl, 2,2,2,-Trichlorethyl, 2-(Trimethylsilyl)ethyl, 2-(Phenylsulfonyl)ethyl, 2-(Triphenylphosphonio)ethyl, Isobutyl, Vinyl, Allyl, p-Nitrophenyl, Benzyl, p-Methoxybenzyl, 3,4-Dimethoxybenzyl, o-Nitrobenzyl, p-Nitrobenzyl, S-Benzyl Thioxarbonate, 4-Ethoxy-1-naphthyl, Methyl Dithiocarbonate.

Weitere Ester:
2,6-Dichlor-4-methylphenoxyacetat, 2,6-Dichlor-4-(1,1,3,3-tetramethylbutyl)phenoxyacetat, 2,4-Bis(1,1-dimethylpropyl)phenoxyacetat, Chlordiphenylacetat, Isobutyrat, Monosuccinat, (E)-2-Methyl-2-butenoat (Tigloat), O-(Methoxycarbonyl)benzoat, p-P-Benzoat, α-Naphthoat, Nitrat, Alkyl N,N,N', N'-Tetramethylphosphorodiamidat, N-Phenylcarbamat, Borate, Dimethylphosphinothioyl, 2,4-Dinitrophenylsulfenat.

Als Sulfonate:
Sulfate, Methansulfonat (Mesylat), Benzylsulfonat, Tosylate.

Insbesondere bevorzugt sind folgende Schutzgruppen:
(C₁-C₂₀)-Alkanoyl, (C₁-C₂₀)-Alkenoyl, (C₁-C₂₀)-Alkinoyl, (C₁-C₁₈)-Alkylcarbamoyl, Di-(C₁-C₁₈)-alkylcarbamoyl, N-(C₃-C₈)-cycloalkylcarbamoyl, (C₁-C₁₈)-Alkoxycarbonyl, (C₆-C₁₂)-Aryloxycarbonyl, (C₇-C₁₁)Aralkyloxycarbonyl, insbesondere Benzyloxycarbonyl, (C₆-C₁₂)-Arylcarbonyl, (C₇-C₁₁)-Aralkylcarbonyl, (C₁-C₁₈)-Alkyl, gegebenenfalls substituiertes (C₇-C₁₈)-Aralkyl, (C₁-C₁₂)-Alkoxy-(C₁-C₁₂)-alkyl, Carbamoyl-(C₁-C₆)-alkylester, (C₁-C₁₈)-Acyloxy-(C₁-C₆)-alkyl, vorzugsweise (C₁-C₁₈)-Alkanoyloxy-(C₁-C₆)-alkyl, Benzyloxy-(C₁-C₆)-alkyl, Benzyloxycarbonyloxy(C₁-C₆)-alkyl, (C₁-C₁₈)-Alkoxycarbonyloxy-(C₁-C₆)-alkyl, wobei diese Gruppen gegebenenfalls weitere Substituenten tragen können, Tetrahydropyranyl, Aminosäureester oder Ester mit Polypeptiden/Polypeptid-Derivaten, vorzugsweise Di- und Tripeptiden, die im Besonderen über ihre terminale Carboxylgruppe verestert sind.

In den Komponenten, die Aminosäuren beinhalten, sind insbesondere die natürlichen α-Aminosäuren bevorzugt, wobei ihre Aminogruppe mit Aminoschutzgruppen derivatisiert sein kann.

Unter Aminoschutzgruppen versteht man insbesondere solche Gruppen, die in R. Geiger und W. König "The Peptides" Volume 3, "Protection of Functional Groups in Peptide Synthesis", E. G. Gross, J. Meienhofer Edit, Academic Press, New York (1981), insbesondere Seiten 7 - 46, beschrieben sind.

Ebenso werden solche Gruppen in A. Hubbuch, Schutzgruppen in der Peptidsynthese, Kontakte 3/79, Seiten 14-23 beschrieben.

Insbesondere bevorzugt sind folgende Aminoschutzgruppen:
Acetamidomethyl,
1-Adamantyloxycarbonyl,
1-(1-Adamantyl)-1-methyl-ethoxycarbonyl,
Allyloxycarbonyl,
tert.-Butyloxycarbonyl,
1-(4-Biphenylyl)-1-methyl-ethoxycarbonyl,
α,α-Dimethyl-3,5-dimethoxybenzyloxycarbonyl,
4-Dihydroxyborylbenzyloxycarbonyl,
9-Fluorenylmethyloxycarbonyl,
Isobornyloxycarbonyl,
1-Methyl-cyclobutyloxycarbonyl,
4-Methoxybenzyloxycarbonyl,
Methylsulfonylethyloxycarbonyl,
4-Pyridylmethyloxycarbonyl,
2,2,2-Trichloro-tert.-butyloxycarbonyl,
Benzyloxycarbonyl,
halogensubstituiertes Benzyloxycarbonyl,
4-Nitro-benzyloxycarbonyl,
2-Phosphonoethyloxycarbonyl,
Phenylsulfonylethoxycarbonyl,
Toluolsulfonylethoxycarbonyl,
2,3,5-Trimethyl-4-methoxy-phenylsulfonyl,
Bevorzugt sind Verbindungen der allgemeinen Formel I,
worin
- X: eine Einfachbindung und Y -SO₂- oder -CO- oder
- X: -CO- und Y -SO₂-, -CO- oder -CONR⁹ bedeutet, wobei
- R¹, R² und R³: gleich oder verschieden sind und Wasserstoff, (C₁-C₆)-Alkyl, (C₁-C₆)-Alkoxy, Halogen, insbesondere Fluor und Chlor, Hydroxy, Amino, Hydroxy-(C₁-C₄)-alkyl,
- R⁶: Wasserstoff, (C₁-C₂₀)-Alkyl oder eine N-Schutzgruppe wie Acyl, (C₁-C₂₀)-Alkanoyl, (C₁-C₂₀)-Alkenoyl, (C₁-C₂₀)-Alkinoyl, (C₇-C₂₀)-Aralkanoyl, (C₆-C₁₈)-Aroyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein physiologisch verwendbares Kation, wie Na⊕, K⊕, Mg²⊕, Ca²⊕, Al³⊕ oder ein Ammoniumion, ggf. 1-3fach substituiert mit (C₁-C₁₆)Alkyl, (C₁-C₁₆)-Hydroxyalkyl, (C₁-C₈)-Alkoxy-(C₁-C₁₆)-alkyl, Phenyl, Benzyl oder (C₁-C₁₆)-Alkyl, welches 1 bis 3-fach substituiert sein kann mit Hydroxy oder (C₁-C₈)-Alkoxy, oder ein Kation eines basischen Aminosäurederivates, wobei ein vorhandener Arylteil gegebenenfalls substituiert sein kann,
- R⁷: einen Rest Y R⁸ bedeutet,
in welchem
- Y: in der obigen Bedeutung steht
und
- R⁸ und R⁹: [C-U]ᵣ-D-W bedeuten, worin
- C: eine Bindung, oder
einen verzweigten oder unverzweigten aliphatischen (C₁-C₁₂)-Alkandiyl-rest, oder
einen verzweigten oder unverzweigten (C₂-C₁₂)-Alkendiyl-Rest, einen (C₂-C₁₂)-Alkindiyl-Rest oder einen (C₂-C₁₂)-Alkenindiyl-Rest bedeutet, der eine oder mehrere C-C-Mehrfachbindungen enthalten kann,
- U: eine Bindung oder
Wasserstoff oder einen Rest aus der Reihe folgender Heteroatomgruppierungen
-(CO)NR-, -NR(CO)-, -O-, -SO-, -SO₂-, worin R (C₁-C₃)Alkyl, (C₁-C₈)Alkanoyl, (C₇-C₁₀)Aralkanoyl, (C₆-C₁₂)Aroyl oder Wasserstoff bedeutet,
- r: 1 oder 2 ist,
- D: eine Bindung oder
Wasserstoff oder
einen verzweigten oder unverzweigten aliphatischen (C₁-C₈)-Alkandiyl-Rest, oder
einen verzweigten oder unverzweigten (C₂-C₈)-Alkendiyl-Rest oder (C₂-C₈)-Alkindiyl-Rest und
- W: Wasserstoff oder
einen (C₃-C₁₀)-cycloaliphatischen Alkyl-, Alkenyl-, Alkinyl- oder Alkeninyl-Rest oder
einen (C₆-C₁₆)-Arylrest oder einen 5- oder 6-gliedrigen Heteroarylrest, wobei mindestens eine der Variablen C oder D oder W nicht eine Bindung bedeuten und U nur dann in der Bedeutung einer Heteratomgruppierung steht, wenn C nicht eine Bindung bedeutet und
- C, D und/oder W,: sofern diese keine Bindung oder Wasserstoff bedeuten, vorzugsweise ihrerseits substituiert sind durch eine Kombination von bis zu 5 gleichen oder verschiedenen Substituenten aus der Reihe
Hydroxy, Halogen, Cyano, Trifluormethyl, Nitro, Carboxyl, (C₁-C₁₂)-Alkyl,(C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, - OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂) Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
Carbamoyl, N-(C₁-C₁₂)-Alkylcarbamoyl, N,N-Di-(C₁-C₁₂)alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)alkyl)carbamoyl,
N-((C₆-C₁₆)-Aryloxy-(C₁-C₁₀)alkyl)carbamoyl,
N-((C₇-C₁₆)-Aralkyloxy-(C₁-C₁₀)alkyl)carbamoyl,
N-(C₁-C₁₀)-Alkyl-N-((C₁-C₁₀)-Alkoxy-(C₁-C₁₀)alkyl)carbamoyl,
N-(C₁-C₁₀)-Alkyl-N-((C₆-C₁₆)-Aryloxy-(C₁-C₁₀)alkyl)carbamoyl,
N-(C₁-C₁₀)-Alkyl-N-((C₇-C₁₆)-Aralkyloxy-(C₁-C₁₀)alkyl)carbamoyl,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-(C₁-C₅)-Alkyl-N(C₇-C₁₀)-Aralkylamino, N-(C₁-C₅)-Alkyl-N(C₆-C₁₂)-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)alkylamino-(C₁-C₁₀)alkyl, N,N-Di(C₁-C₁₀)alkylamino-(C₁-C₁₀)alkyl, (C₃-C₈)cycloalkylamino-(C₁-C₁₀)alkyl,
(C₁-C₁₂)-Alkylmercapto, (C₁-C₁₂)-Alkylsulfinyl, (C₁-C₁₂)-Alkyl-sulfonyl,
(C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
wobei die Reste, die einen Arylrest enthalten, ihrerseits am Aryl substituiert sein können mit 1, 2, 3, 4 oder 5 gleichen oder verschiedenen Substituenten aus der Reihe Wasserstoff, Hydroxy, Halogen, Cyano, Trifluormethyl, Nitro, Carboxyl, (C₁-C₁₂)-Alkyl,(C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, -OCF₂-CHFCl, (C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂) Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)alkyl)carbamoyl,
N-((C₆-C₁₆)-Aryloxy-(C₁-C₁₀)alkyl)carbamoyl,
N-((C₇-C₁₆)Aralkyloxy-(C₁-C₁₀)alkyl)carbamoyl,
N-(C₁-C₁₀)-Alkyl-N-((C₁-C₁₀)-Alkoxy-(C₁-C₁₀)alkyl)carbamoyl,
N-(C₁-C₁₀)-Alkyl-N-((C₆-C₁₆)-Aryloxy-(C₁-C₁₀)alkyl)carbamoyl,
N-(C₁-C₁₀)Alkyl-N-((C₇-C₁₆)-Aralkyloxy-(C₁-C₁₀)alkyl)carbamoyl,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-(C₁-C₅)-Alkyl-(C₇-C₁₀)-Aralkylamino, N-(C₁-C₅)-Alkyl-(C₆-C₁₂)-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)alkylamino-(C₁-C₁₀)alkyl, N,N-Di(C₁-C₁₀)alkylamino-(C₁-C₁₀)alkyl, (C₃-C₈)cycloalkylamino-(C₁-C₁₀)alkyl,
(C₁-C₁₂)-Alkylmercapto, (C₁-C₁₂)-Alkylsulfinyl, (C₁-C₁₂)-Alkylsulfonyl,
(C₆-C₁₆)-Arylmercapto, (C₆-C₁₆)-Arylsulfinyl, (C₆-C₁₆)-Arylsulfonyl,
(C₇-C₁₆)-Aralkylmercapto, (C₇-C₁₆)-Aralkylsulfinyl, (C₇-C₁₆)-Aralkylsulfonyl,
oder
- R⁸ und R⁹: auch über 3 - 6 C-Atome verknüpft sind, wobei -NR⁸R⁹ einen heterocyclischen Ring bilden, mit N als Heteroatom,
oder
- R⁹: in der Bedeutung von R⁶ steht,
und für [C-U]ᵣ-D-W die Bedeutung -SO₂H ausgenommen ist, sowie
- R⁴: eine Methylgruppe oder - CH₂OR⁵ bedeutet, worin
- R⁵: Wasserstoff oder den Rest einer physiologisch vertraglichen, vorzugsweise in der Leber unter physiologischen Bedingungen abspaltbaren Alkoholschutzgruppe bedeutet,
und
- n: 0 oder 1,
- f: 1 bis 8, vorzugsweise 1 bis 5,
- g: 0, 1 bis (2f+ 1) und
- x: 0 bis 8, vorzugsweise 0 oder 1 bedeuten.

Insbesondere bevorzugt sind folgende Schutzgruppen:
(C₁-C₂₀)-Alkanoyl, (C₁-C₂₀)-Alkenoyl, (C₁-C₂₀)-Alkinoyl, (C₁-C₁₈)-Alkylcarbamoyl, Di-(C₁-C₁₈)-alkylcarbamoyl, N-(C₃-C₈)-cycloalkylcarbamoyl, (C₁-C₁₈)-Alkoxycarbonyl, (C₆-C₁₂)-Aryloxycarbonyl, (C₇-C₁₁)Aralkyloxycarbonyl, insbesondere Benzyloxycarbonyl, (C₆-C₁₂)-Arylcarbonyl, (C₇-C₁₁)-Aralkylcarbonyl, (C₁-C₁₈)-Alkyl, gegebenenfalls substituiertes Benzyl, (C₁-C₆)-Alkoxy-(C₁-C₁₂)-alkyl, Carbamoyl-(C₁-C₁₂)-alkylester, (C₁-C₁₈)-Acyloxy-(C₁-C₆)-alkyl, vorzugsweise (C₁-C₁₈)-Alkanoyloxy-(C₁-C₆)-alkyl, Benzyloxy-(C₁-C₆)-alkyl, Benzyloxycarbonyloxy(C₁-C₆)-alkyl, (C₁-C₁₈)-Alkoxycarbonyloxy-(C₁-C₆)-alkyl, wobei diese Gruppen gegebenenfalls weitere Substituenten tragen können, Tetrahydropyranyl, Aminosäureester oder Ester mit Polypeptiden/Polypeptid-Derivaten, vorzugsweise Di- und Tripeptiden, die im besonderen über ihre terminale Carboxylgruppe verestert sind.

Insbesondere bevorzugt sind Verbindungen der allgemeinen Formel I, in der
- A: R³ und B -XNR⁶R⁷,
- X: eine Einfachbindung und Y -SO₂- oder CO bedeuten, oder
- X: CO und Y -SO₂ oder -CONR⁹ bedeuten, und
- R¹, R² und R³: gleich oder verschieden sind und Wasserstoff oder (C₁-C₃)-Alkyl, (C₁-C₃)-Alkoxy, Hydroxy, Fluor oder Chlor,
- R⁶: Wasserstoff, (C₁-C₁₂)-Alkyl oder eine N-Schutzgruppe wie Acyl, (C₁-C₁₈)-Alkanoyl, (C₁-C₁₈)-Alkenoyl, gegebenenfalls substituiertes Benzoyl, ein 1, 2, 3 oder 4-wertiges physiologisch verwendbares Kation, insbesondere Na⊕, K⊕, Ca²⊕, Mg²⊕, Al³⊕ oder ein Ammoniumion, ggf. 1-3 fach substituiert mit (C₁-C₁₂)-Alkyl, (C₁-C₁₂)-Hydroxyalkyl, (C₁-C₄)-Alkoxy-(C₁-C₁₀)-alkyl, Phenyl, Benzyl oder (C₁-C₁₂)-Alkyl, welches 1 bis 3-fach substituiert sein kann mit Hydroxy oder (C₁-C₈)-Alkoxy, wobei ein Arylrest gegebenenfalls substituiert sein kann oder ein Kation eines basischen Aminosäurederivatives,
- R⁷: einen Rest Y R⁸ bedeutet,
in welchem
- Y: in der obigen Bedeutung steht
und
- R⁸: [C-U]ᵣ-D-W bedeuten, worin
- C: eine Bindung oder
einen (C₁-C₆)-Alkandiyl-Rest,
- U: eine Bindung oder
Wasserstoff oder -O- bedeutet,
- r: 1 ist,
- D: eine Bindung oder
Wasserstoff oder
einen unverzweigten aliphatischen (C₁-C₈)-Alkandiyl-Rest, und
- W: Wasserstoff, einen (C₆-C₁₂)-Arylrest oder einen 5- oder 6-gliedrigen Heteroarylrest, wobei mindestens eine der Variablen C oder D oder W nicht eine Bindung bedeuten und U nur dann in der Bedeutung einer Heteratomgruppierung steht, wenn C nicht eine Bindung bedeutet und W sofern W nicht Wasserstoff bedeutet, vorzugsweise seinerseits substituiert ist mit bis zu 3 gleichen oder verschiedenen Substituenten aus der Reihe Hydroxy, Halogen, Cyano, Trifluormethyl, Nitro, Carboxyl, (C₁-C₈)-Alkyl,(C₃-C₈)-Cycloalkyl, (C₆-C₁₂)-Aryl, (C₇-C₁₆)-Aralkyl, (C₃-C₈)-Alkenyl, (C₃-C₈)-Alkinyl, (C₁-C₈)-Alkoxy, (C₁-C₈)-Alkoxy-(C₁-C₈)alkyl, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g},
(C₁-C₈)-Alkoxycarbonyl, (C₆-C₁₂)-Aryloxycarbonyl, (C₇-C₁₄)-Aralkoxycarbonyl, (C₃-C₈)-Cycloalkoxycarbonyl,
(C₁-C₈)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)alkyl)carbamoyl,
N-((C₆-C₁₆)-Aryloxy-(C₁-C₁₀)alkyl)carbamoyl,
N-((C₇-C₁₆)-Aralkyloxy-(C₁-C₁₀)alkyl)carbamoyl,
N-(C₁-C₁₀)-Alkyl-N-((C₁-C₁₀)-Alkoxy-(C₁-C₁₀)alkyl)carbamoyl,
N-(C₁-C₁₀)-Alkyl-N-((C₆-C₁₆)-Aryloxy-(C₁-C₁₀)alkyl)carbamoyl,
N-(C₁-C₁₀)-Alkyl-N-((C₇-C₁₆)-Aralkyloxy-(C₁-C₁₀)alkyl)carbamoyl,
Amino, (C₁-C₈)-Alkylamino, Di-(C₁-C₈)alkylamino, (C₃-C₈)-Cycloalkylamino, N-(C₆-C₁₂)-Arylamino, N-(C₇-C₁₁)-Aralkylamino, N-(C₁-C₃)-Alkyl-N-(C₇-C₁₁)-Aralkylamino,
(C₁-C₁₀)-Alkanoylamino, (C₃-C₈)-Cycloalkanoylamino, (C₆-C₁₂)-Aroylamino, (C₇-C₁₆)-Aralkanoylamino, (C₁-C₁₀)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₆)-alkyl, (C₃-C₈)-Cycloalkanoylamino-(C₁-C₈)alkyl, (C₆-C₁₆)-Aroylamino-(C₁-C₆)-alkyl, (C₇-C₁₆)-Aralkanoylamino-(C₁-C₆)-alkyl,
(C₁-C₈)-Alkylmercapto, (C₁-C₈)-Alkylsulfinyl, (C₁-C₈)-Alkylsulfonyl, (C₆-C₁₂)-Arylmercapto, (C₆-C₁₂)-Alkylsulfinyl, (C₆-C₁₂)-Arylsulfonyl, (C₇-C₁₄)-Aralkylmercapto, (C₇-C₁₄)-Aralkylsulfinyl, (C₇-C₁₄)-Aralkylsulfonyl,
wobei die Reste, die einen Arylrest enthalten, ihrerseits am Aryl substituiert sein können mit 1, 2, 3, 4 oder 5 gleichen oder verschiedenen Substituenten aus der Reihe Wasserstoff, Hydroxy, Halogen, Cyano, Trifluormethyl, Nitro, Carboxyl, (C₁-C₈)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₈)-Alkoxy, (C₁-C₆)-Alkoxy-(C₁-C₆)alkyl, (C₆-C₁₂)-Aryloxy, (C₇-C₁₆)-Aralkyloxy,
-O-[CH₂]_{X}C_{f}H_{(2f+1-g)}F_{g},
(C₁-C₁₂)-Alkoxycarbonyl, (C₃-C₈)-Cycloalkoxycarbonyl,
(C₁-C₁₂)-Alkylcarbonyloxy, (C₃-C₈)-Cycloalkylcarbonyloxy,
Carbamoyl, N-(C₁-C₈)-Alkylcarbamoyl, N,N-Di-(C₁-C₈)-alkylcarbamoyl,
N-(C₃-C₈)-Cycloalkylcarbamoyl, N-(C₆-C₁₆)-Arylcarbamoyl, N-(C₇-C₁₆)-Aralkylcarbamoyl, N-(C₁-C₈)-Alkyl-N-(C₆-C₁₆)arylcarbamoyl, N-(C₁-C₈)-Alkyl-N-(C₇-C₆)-Aralkylcarbamoyl,
N-((C₁-C₆)-Alkoxy-(C₁-C₆)alkyl)carbamoyl,
N-((C₆-C₆)-Aryloxy-(C₁-C₆)alkyl)carbamoyl,
N-((C₇-C₁₆)-Aralkyloxy-(C₁-C₆)alkyl)carbamoyl,
N-(C₁-C₈)-Alkyl-N-((C₁-C₆)-Alkoxy-(C₁-C₆)alkyl)carbamoyl,
N-(C₁-C₈)-Alkyl-N-((C₆-C₁₆)-Aryloxy-(C₁-C₆)alkyl)carbamoyl,
N-(C₁-C₈)-Alkyl-N-((C₇-C₁₆)-Aralkyloxy-(C₁-C₆)alkyl)carbamoyl,
Amino, (C₁-C₆)-Alkylamino, Di-(C₁-C₆)alkylamino, (C₃-C₈)-Cycloalkylamino, N-(C₆-C₁₂)-Arylamino, N-(C₇-C₁₁)-Aralkylamino, N-(C₁-C₃)-Alkyl-(C₇-C₁₁)-Aralkylamino, N-(C₁-C₃)-Alkyl-(C₆-C₁₂)-Arylamino, (C₁-C₈)-Alkoxy-amino,
(C₁-C₈)-Alkanoylamino, (C₃-C₈)-Cycloalkanoylamino, (C₆-C₁₂)-Aroylamino, (C₇-C₁₂)-Aralkanoylamino, (C₁-C₈)-Alkanoyl-N-(C₁-C₆)-alkylamino, (C₃-C₈)-Cycloalkanoyl-N-(C₁-C₆)-alkylamino, (C₆-C₁₂)-Aroyl-N-(C₁-C₁₀)-alkylamino, (C₇-C₁₁)-Aralkanoyl-N-(C₁-C₆)-alkylamino,
(C₁-C₈)-Alkanoylamino-(C₁-C₄)-alkyl, (C₃-C₈)-Cycloalkanoylamino-(C₁-C₄)alkyl, (C₆-C₁₂)-Aroylamino-(C₁-C₄)-alkyl, (C₇-C₁₂)-Aralkanoylamino-(C₁-C₄)-alkyl,
- R⁹: Wasserstoff, (C₁-C₆)-Alkyl, Phenyl; gegebenenfalls einfach substituiert, bedeutet, oder
- R⁸ und R⁹: über (-CH₂-)ᵢ verknüpft sind, wobei -NR⁸R⁹ einen Cyclus bilden und i 3, 4, 5 und 6 bedeuten kann,
und für [C-U]ᵣ-D-W die Bedeutung -SO₂H ausgenommen ist, sowie
- R⁴: Methyl oder -CH₂OR⁵ bedeutet, worin
- R⁵: Wasserstoff oder den Rest einer physiologisch verträglichen, vorzugsweise in der Leber unter physiologischen Bedingungen abspaltbaren Alkoholschutzgruppe bedeutet,
und
- n: 0 oder 1,
- f: 1 bis 8, vorzugsweise 1 bis 5,
- g: 0, 1 bis (2f+ 1) und
- x: 0 bis 8, vorzugsweise 0 oder 1 bedeuten.

Insbesondere bevorzugt sind folgende Schutzgruppen:
(C₁-C₁₈)-Alkanoyl, (C₁-C₁₈)-Alkenoyl, (C₁-C₁₈)-Alkylcarbamoyl, Di-(C₁-C₈)-alkylcarbamoyl, N-(C₃-C₈)-cycloalkylcarbamoyl, (C₁-C₁₈)-Alkoxycarbonyl, (C₆-C₁₂)-Aryloxycarbonyl, (C₇-C₁₁)Aralkyloxycarbonyl, insbesondere Benzyloxycarbonyl, (C₆-C₁₂)-Arylcarbonyl, (C₇-C₁₁)-Aralkylcarbonyl, (C₁-C₁₂)-Alkyl, (C₁-C₆)-Alkoxy-(C₁-C₆)-alkyl, Carbamoyl-(C₁-C₈)-alkylester, (C₁-C₁₈)-Acyloxy-(C₁-C₆)-alkyl, vorzugsweise (C₁-C₁₈)-Alkanoyloxy-(C₁-C₆)-alkyl, Benzyloxy-(C₁-C₆)-alkyl,
Benzyloxycarbonyloxy(C₁-C₆)-alkyl, (C₁-C₆)-Alkoxycarbonyloxy-(C₁-C₆)-alkyl, wobei diese Gruppen gegebenenfalls weitere Substituenten tragen können,
Aminosäureester oder Tetrahydropyranyl.

Ganz besonders bevorzugt sind Verbindungen der allgemeinen Formel I, in der
- A: R³ und B -X NR⁶R⁷,
- X: eine Einfachbindung und Y -SO₂- bedeutet, oder
- X: -CO- und Y -SO₂- oder -CONR⁹ bedeuten,
- R¹, R² und R³: Wasserstoff,
- R⁶: Wasserstoff, (C₁-C₁₂)-Alkyl oder eine Acylgruppe, wie (C₁-C₁₈)-Alkanoyl, (C₁-C₁₈)-Alkenoyl, gegebenenfalls substituiertes Benzoyl oder ein 1, 2 oder 3-wertiges physiologisch verwendbares Kation, insbesondere Na⊕, K⊕, Mg²⊕, Ca²⊕ oder ein Ammoniumion ggf. 1-3fach substituiert mit (C₁-C₁₂)-Alkyl, (C₁-C₁₂)-Hydroxyalkyl, (C₁-C₄)-Alkoxy-(C₁-C₈)-alkyl, Phenyl, Benzyl oder (C₁-C₁₂)-Alkyl, welches 1 bis 3-fach substituiert sein kann mit Hydroxy oder (C₁-C₄)-Alkoxy, wie z.B. NH₃⊕C(CH₂OH)₃ oder ein Kation eines basischen Aminosäurederivates,
- R⁷: einen Rest Y R⁸, ausgenommen -SO₂H, bedeutet in welchem
- Y: in der obigen Bedeutung steht und
- R⁸: [C-U]ᵣ-D-W bedeutet, wobei
- C: eine Bindung oder
(C₁-C₄)-Alkandiyl,
- U: eine Bindung, Wasserstoff oder
-O- bedeutet,
- r: 1 ist,
- D: eine Bindung, Wasserstoff oder
(C₁-C₄)-Alkandiyl,
- W: Wasserstoff oder
einen Phenylrest bedeutet, wobei mindestens eine der Variablen C oder D oder W nicht eine Bindung bedeuten und U nur dann in der Bedeutung von -O- steht, wenn C nicht eine Bindung bedeutet und W substituiert ist durch Wasserstoff, Fluor, Chlor, (C₁-C₆)-Alkyl, (C₃-C₈)-Cycloalkyl, (C₆-C₁₂)-Aryl, (C₇-C₁₆)-Aralkyl, (C₃-C₁₂)-Alkenyl, Phenyl, (C₁-C₆)-Alkoxy, Phenoxy, -O-[CH₂]_{X}C_{f}H_{(2f+1-g)}F_{g},
Carbamoyl, N-(C₁-C₁₀)-Alkylcarbamoyl, N,N-Di-(C₁-C₈)--alkylcarbamoyl, N-(C₃-C₈)-Cycloalkylcarbamoyl, N-Phenylcarbamoyl, N-(C₇-C₁₁)-Phenylalkylcarbamoyl, N-(C₁-C₈)-Alkyl-N-(C₆-C₁₆)phenylcarbamoyl, N-(C₁-C₈)-Alkyl-N-(C₇-C₁₁)-Phenylalkylcarbamoyl,
N-((C₁-C₁₀)-Alkoxy-(C₁-C₈)alkyl)carbamoyl,
N-(Phenoxy-(C₁-C₈)alkyl)carbamoyl,
N-((C₇-C₁₆)-Phenylalkyloxy-(C₁-C₈)alkyl)carbamoyl,
N-(C₁-C₈)-Alkyl-N-((C₁-C₆)-Alkoxy-(C₁-C₈)alkyl)carbamoyl,
N-(C₁-C₈)-Alkyl-N-((C₆-C₁₂)-Phenoxy-(C₁-C₈)alkyl)carbamoyl,
N-(C₁-C₈)-Alkyl-N-((C₇-C₁₆)-Phenylalkyloxy-(C₁-C₈)alkyl)carbamoyl,
(C₁-C₈)-Alkanoylamino, (C₃-C₈)-Cycloalkanoylamino, (C₆-C₁₂)-Phenylamino, (C₇-C₁₁)-Phenylalkanoylamino, (C₁-C₈)-Alkanoyl-N-(C₁-C₁₀)-alkylamino, (C₃-C₈)-Cycloalkanoyl-N-(C₁-C₆)-alkylamino, Benzoyl-N-(C₁-C₁₀)-alkylamino, (C₇-C₁₁)-Phenylalkanoyl-N-(C₁-C₆)-alkylamino,
(C₁-C₁₀)-Alkanoylamino-(C₁-C₈)-alkyl, (C₃-C₈)-Cycloalkanoylamino-(C₁-C₈)alkyl, Phenylamino-(C₁-C₈)-alkyl, (C₇-C₁₁)-Phenylalkanoylamino-(C₁-C₈)-alkyl,
wobei die Reste, die einen Arylrest enthalten, ihrerseits am Aryl substituiert sein können mit 1, 2, 3, 4 oder 5 gleichen oder verschiedenen Substituenten aus der Reihe Hydroxy, Carboxyl, (C₁-C₄)-Alkoxy, Phenoxy, Benzyloxy, Fluor, Chlor, Trifluormethyl, -O-[CH₂]ₓC_{f}H_{(2f+1-g)}Fg
(C₁-C₉)-Alkoxycarbonyl, Phenoxycarbonyl, (C₇-C₁₁)-Phenylalkoxycarbonyl, (C₃-C₈)-Cycloalkoxycarbonyl,
(C₁-C₁₂)Alkylcarbonyloxy, (C₃-C₈)-Cycloalkylcarbonyloxy, Benzoyloxy, (C₇-C₁₁)-Phenalkylcarbonyloxy,
(C₁-C₈)-Alkoxycarbonyloxy, (C₆-C₁₂)-Phenoxycarbonyloxy, (C₇-C₁₁)-Phenalkyloxycarbonyloxy, (C₃-C₈)-Cycloalkoxycarbonyloxy,
Carbamoyl, N-(C₁-C₆)-Alkylcarbamoyl, N,N-Di-(C₁-C₆)-alkylcarbamoyl, N-(C₃-C₈)-Cycloalkylcarbamoyl, N-Phenylcarbamoyl, N-(C₇-C₁₁)-Phenalkylcarbamoyl, Hydroxy-(C₁-C₄)-alkyl-carbamoyl, Acyloxy-(C₁-C₄)-alkyl-carbamoyl,
Carbamoyloxy, N-(C₁-C₆)-Alkylcarbamoyloxy, N,N-Di-(C₁-C₆)-alkylcarbamoyloxy, N-(C₃-C₈)-Cycloalkylcarbamoyl,
- R⁹: Wasserstoff, (C₁-C₄)-Alkyl, Phenyl,
- R⁴: -CH₂O R⁵, worin
- R⁵: Wasserstoff, (C₁-C₁₂)-Alkyl, ggf. substituiertes Benzyl, (C₁-C₁₈)-Alkanoyl, (C₁-C₁₈)-Alkenoyl, ggf. substituiertes Benzoyl und
- n: 0,
- f: 1 bis 5,
- g: 0, 1 bis (2f + 1) und
- x: 0 oder 1
bedeuten.

Von größter Bedeutung sind Verbindungen der allgemeinen Formel I, in der
- A: R³ und B -X NR⁶R⁷
- X: -CO-
- R¹, R² und R³: Wasserstoff,
- R⁶: Wasserstoff, (C₁-C₆)-Alkyl,(C₁-C₁₈)-Alkanoyl, (C₁-C₁₈)-Alkenoyl, Benzoyl oder ein 1 oder 2-wertiges physiologisch verwendbares Kation, insbesondere Na⊕, K⊕, Mg²⊕ oder Ca²⊕ oder ein Ammoniumion, ggf. 1-3 fach substituiert mit (C₁-C₈)-Alkyl, (C₁-C₈)-Hydroxyalkyl, (C₁-C₄)-Alkoxy-(C₁-C₈)-alkyl, Phenyl, Benzyl oder (C₁-C₈)-Alkyl, welches 1 bis 3-fach substituiert sein kann mit Hydroxy oder (C₁-C₄)-Alkoxy,
- R⁷: einen Rest Y R⁸, ausgenommen -SO₂H, bedeutet
in welchem
- Y: -SO₂- und
- R⁸: [C-U]ᵣ-D-W bedeutet, wobei
- C: eine Bindung oder lineares
(C₁-C₄)-Alkandiyl,
- U: eine Bindung, Wasserstoff oder
-O- bedeutet,
- r: 1 ist,
- D: eine Bindung, Wasserstoff oder lineares
(C₁-C₄)-Alkandiyl,
- W: Wasserstoff oder
einen Phenylrest bedeutet, wobei mindestens eine der Variablen C oder D oder W nicht eine Bindung bedeuten und U nur dann in der Bedeutung von -O- steht, wenn C nicht eine Bindung bedeutet und W substituiert ist durch Wasserstoff, Fluor, Chlor, (C₁-C₆)-Alkyl, (C₃-C₈)-Cycloalkyl, (C₇-C₁₆)-Phenylalkyl, Phenyl, (C₁-C₆)-Alkoxy, Phenoxy, -O-[CH₂]_{X}C_{f}H_{(2f+1-g)}F_{g},
Carbamoyl, N-(C₁-C₁₀)-Alkylcarbamoyl, N,N-Di-(C₁-C₈)-alkylcarbamoyl, N-(C₃-C₈)-Cycloalkylcarbamoyl, N-Phenylcarbamoyl, N-(C₇-C₁₁)-Phenylalkylcarbamoyl, N-(C₁-C₈)-Alkyl-N-(C₆-C₁₆)phenylcarbamoyl, N-(C₁-C₈)-Alkyl-N-(C₇-C₁₁)-Phenylalkylcarbamoyl,
N-((C₁-C₃)-Alkoxy-(C₁-C₄)alkyl)carbamoyl,
N-(Phenoxy-(C₁-C₈)alkyl)carbamoyl,
N-((C₇-C₁₆)-Phenylalkyloxy-(C₁-C₄)alkyl)carbamoyl,
N-(C₁-C₆)-Alkyl-N-((C₁-C₃)-Alkoxy-(C₁-C₄)alkyl)carbamoyl,
N-(C₁-C₆)-Alkyl-N-((C₆-C₁₂)-Phenoxy-(C₁-C₄)alkyl)carbamoyl,
N-(C₁-C₆)-Alkyl-N-((C₇-C₁₆)-Phenylalkyloxy-(C₁-C₄)alkyl)carbamoyl,
(C₁-C₈)-Alkanoylamino, (C₃-C₈)-Cycloalkanoylamino, (C₆-C₁₂)-Phenylamino, (C₇-C₁₁)-Phenylalkanoylamino, (C₁-C₈)-Alkanoyl-N-(C₁-C₆)-alkylamino, (C₃-C₈)-Cycloalkanoyl-N-(C₁-C₆)-alkylamino, Benzoyl-N-(C₁-C₆)-alkylamino, (C₇-C₁₁)-Phenylalkanoyl-N-(C₁-C₆)-alkylamino,
(C₁-C₁₀)-Alkanoylamino-(C₁-C₂)-alkyl, (C₃-C₈)-Cycloalkanoylamino-(C₁-C₂)alkyl, Benzoylamino-(C₁-C₂)-alkyl, (C₇-C₁₁)-Phenylalkanoylamino-(C₁-C₂)-alkyl,
wobei die Reste, die einen Arylrest enthalten, ihrerseits am Aryl substituiert sein können mit 1, 2, 3, 4 oder 5 gleichen oder verschiedenen Substituenten aus der Reihe Hydroxy, Carboxyl, (C₁-C₆)-Alkoxy, Phenoxy, Benzyloxy, Fluor, Chlor, Trifluormethyl,
(C₁-C₉)-Alkoxycarbonyl, Phenoxycarbonyl, (C₇-C₁₁)-Phenylalkoxycarbonyl, (C₃-C₈)-Cycloalkoxycarbonyl,
(C₁-C₁₂)Alkylcarbonyloxy, (C₃-C₈)-Cycloalkylcarbonyloxy, Benzoyloxy, (C₇-C₁₁)-Phenalkylcarbonyloxy,
Carbamoyl, N-(C₁-C₆)-Alkylcarbamoyl, N,N-Di-(C₁-C₆)-alkylcarbamoyl, N-(C₃-C₈)-Cycloalkylcarbamoyl, N-Phenylcarbamoyl, N-(C₇-C₁₁)-Phenalkylcarbamoyl, Hydroxy-(C₁-C₄)-alkyl-carbamoyl, Acyloxy-(C₁-C₄)-alkyl-carbamoyl,
Carbamoyloxy, N-(C₁-C₆)-Alkylcarbamoyloxy, N,N-Di-(C₁-C₆)-alkylcarbamoyloxy, N-(C₃-C₈)-Cycloalkylcarbamoyl,
- R⁴: -CH₂O R⁵, worin
- R⁵: Wasserstoff, (C₁-C₁₂)-Alkyl, ggf. substituiertes Benzyl, (C₁-C₁₈)-Alkanoyl, (C₁-C₁₈)-Alkenoyl, ggf. substituiertes Benzoyl und
- n: 0,
- f: 1 bis 5,
- g: 0, 1 bis (2f + 1) und
- x: 0 oder 1
bedeuten.

Weiterhin betrifft die Erfindung die Verwendung von Verbindungen der allgemeinen Formel I sowie die physiologisch verträglichen Salze zur Herstellung eines Arzneimittels gegen fibrotische Erkrankungen.

Schließlich betrifft die Erfindung die Verbindungen der allgemeinen Formel I zur Verwendung als Arzneimittel.

Insbesondere betrifft die Erfindung die Verbindungen der Formel I zur Anwendung als Fibrosuppressiva.

Weiterhin betrifft die Erfindung ein Verfahren zur Herstellung von Verbindungen der allgemeinen Formel I.

Verbindungen der Formel Ib, in denen X eine Einfachbindung und R⁴ CH₂OR⁵ bedeutet, wurden hergestellt, indem
i) 2-Methylpyridin-N-oxide der Formal 1a' mit Anhydriden im sauren Milieu, umgesetzt wurden, oder
ii) 2-Halomethylpyridine der Formel 3 mit Reagenzien MOR⁵ umgesetzt wurden, oder
iii) Pyridin-2-carbonsäure-Derivate der Formel 5 reduziert wurden, oder
iv) die 2-Hydroxymethylpyridin-Derivate der Formel 4 mit den Sulfonsäure-Derivaten der Formel 2 umgesetzt wurden (vgl. Schema 1).

Schema 1 verdeutlicht die Herstellung von Verbindungen der Formeln Ia, Ia', Ib und Ib', in denen X eine Einfachbindung bedeutet:

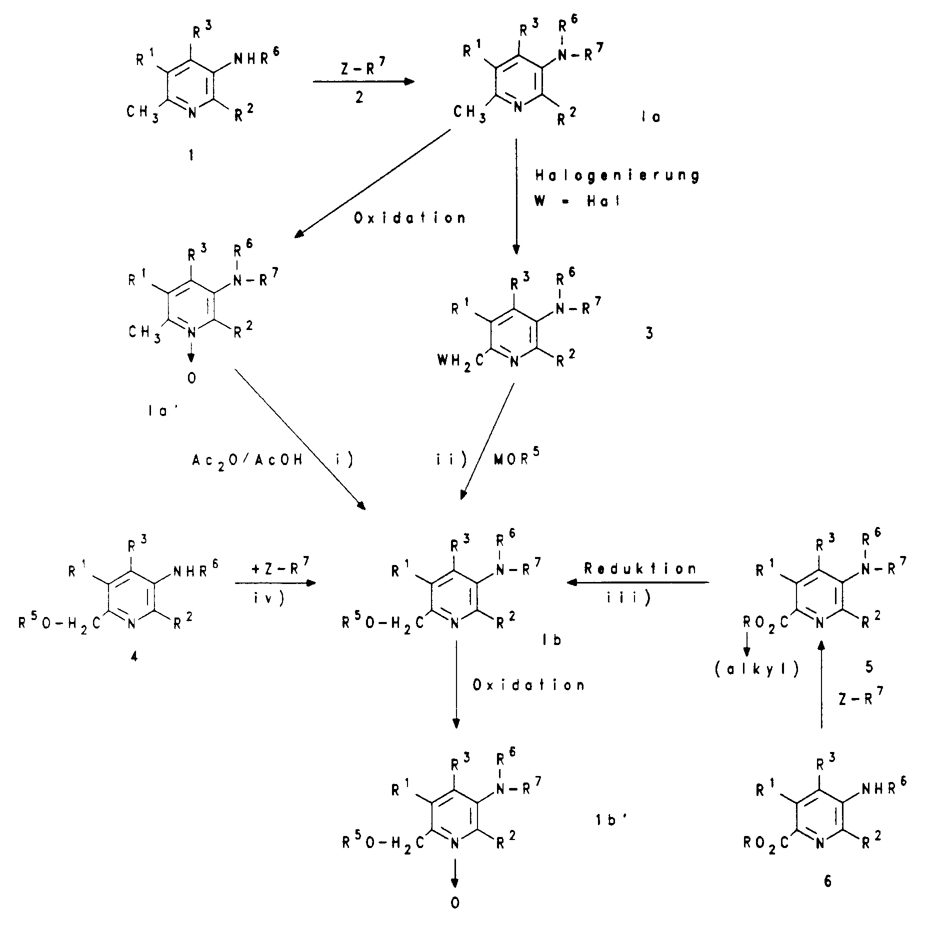

Verbindungen der Formel 1 werden mit Sulfonsäure- bzw. Carbonsäure-Derivaten der Formel 2, in denen Z für eine Abgangsgruppe, die nukleophil ablösbar ist und insbesondere F, Cl, Br, J oder Tosylat bedeutet, zu Verbindungen der Formel Ia umgesetzt, in denen R⁴ Methyl bedeutet. Diese Umsetzung erfolgt in einem dipolar aprotischen organischen Lösungsmittel oder einem Lösemittelgemisch. Insbesondere sind folgende Lösemittel genannt: Dichlormethan, Tetrachlormethan, Butylacetat, Ethylacetat, Toluol, Tetrahydrofuran, Dimethoxyethan, 1,4-Dioxan, Acetonitril, N,N-Dimethylformamid, N,N-Dimethylacetamid, Dimethylsulfoxid, Nitromethan und/oder Pyridin, gegebenenfalls unter Zusatz eines Säurebinders, wie Ammoniak, Triethylamin, Tributylamin, bei einer Reaktionstemperatur von 0° bis 180°C, vorzugsweise von 0° bis 80°C.

Gegebenenfalls erfolgt daran anschließend noch die Oxidation zu den Pyridin-N-oxiden der Formel Ia'.

Eine allgemeine Vorschrift dieser Oxidationsmethode ist auch beispielsweise in "E. Klinsberg, Pyridine and its Derivitives, Interscience Publishers, New York, 1961, Part 2, 93" beschrieben. Die Oxidation mit Wasserstoffperoxid ist beispielsweise in "E. Ochiai, J. Org. Chem. 18, 534 (1953)" beschrieben. Die Verfahrensbedingungen können im Detail der deutschen Patentanmeldung P 38 26 471.4, 38 28 140.6, 39 24 093.2, 40 01 002.3 sowie den DE-A-37 03 959, 37 03 962 und 37 03 963 entnommen werden.

Sofern Verbindungen der allgemeinen Formel I hergestellt werden sollen, in denen R⁴ = -CH₂OR⁵ ist (hier Verbindungen Ib bzw. Ib'), werden Verbindungen der Formel Ia (2-Picoline) mit N-Halogenamiden, wie N-Bromsuccinimid oder N-Chlorphthalimid oder mit Trichlorisocyanursäure halogeniert (Chem. Ber. 120, 649 - 651 (1987)).

Die Umsetzung der 2-Picolylhalogenide der Formel 3 mit Basen der Formel MOR⁵ erfolgt nach dem Fachmann geläufigen Methoden und führt zu Verbindungen der Formel Ib. Das Herstellungsverfahren ist darüberhinaus in der P 41 36 346.9 beschrieben. Daran anschließen kann sich eine Oxidation zu den entsprechenden Pyridin-N-oxiden der Formel Ib' (siehe Oxidation zu Verbindungen der Formel Ia').

Wahlweise lassen sich die Verbindungen der Formel Ib auch durch Umwandlung von Pyridin-N-oxiden der Formel Ia' herstellen (Verfahren i) Schema 1). Die Umwandlung wird mit Trifluoracetanhydrid bei 0°C oder mit Acetanhydrid, eventuell in Gegenwart einer Säure, wie Eisessig, bei 80 - 120°C durchgeführt und man erhält Acetate (Formel Ib, worin R⁵ = Ac). Die Umwandlung wird auch von Katritzky, A.R., Lagowski, J.M. in: Chemistry of the Heterocyclic N-Oxides", Academic Press, New York, 1972, IV. 2, Seiten 352 - 365, beschrieben. Die anschließende Verseifung zu Verbindungen der Formel Ib, worin R⁵ = H, wird nach bekannten Verfahren durchgeführt. Insbesondere werden hierzu Alkalihydroxide oder -carbonate in Methanol, Ethanol, Wasser oder ähnliches verwendet.

Die oben beschriebene Reaktionsfolge gemäß Schema 1 zur Herstellung von in 5-Stellung eine NR⁶R⁸-Gruppe aufweisende Verbindungen der Formeln Ia, Ia',Ib und Ib' läßt sich auch zur Herstellung solcher Verbindungen anwenden, die diese Gruppe in 4-Stellung aufweisen. Begonnen wird dann mit Verbindungen der allgemeinen Formel 1':

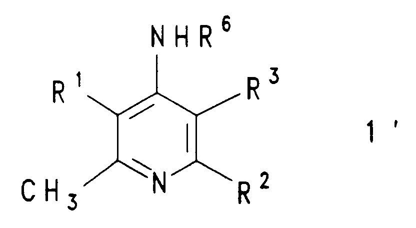

Schema 2 verdeutlicht die Herstellung von Verbindungen der Formeln Ic oder Ic', in denen X = -CO- bedeutet: 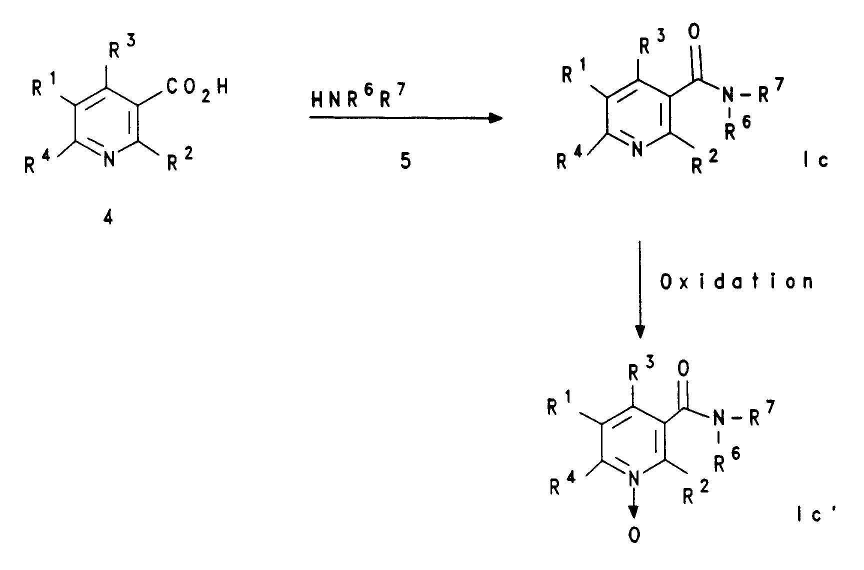
Verbindungen der Formel 4, in denen R⁴ = Methyl und -CH₂-OR⁵ bedeuten, sind von T. Sugiyama et al., Chem. Lett. 1982, 917 - 920 und J.K. Seydel et al., J.Med. Chem. 1976, 19, 483 - 493 bekannt.

Die Umsetzung von Verbindungen der Formel 4 mit Amiden der Formel 5 erfolgt entsprechend den Angaben aus Houben-Weyl: Methoden der organischen Chemie, Band IX, Kapitel 19, Seiten 636 - 637. Es kann dabei zweckmäßig sein, beide Reaktanden mit Hilfsreagenzien zu aktivieren.

Als Hilfsreagenzien zur Carbonsäurereaktivierung können die dem Fachmann bekannten Substanzen, wie Thionylchlorid, Oxalylchlorid, Pivaloylchlorid, N,N'-Carbonyldiimidazol oder Chlorameisensäureester-Derivate Verwendung finden. Es ist nicht immer notwendig, diese aktivierten Derivate der Verbindungen der Formel 4 zu isolieren. Meist ist es zweckmäßig, sie nach Herstellung in situ oder als Rohprodukt mit den Amiden der Formel 5 umzusetzen.

Zweckmäßigerweise werden die Verbindungen der Formel 5 zunächst mit einer anorganischen oder organischen Base, wie z. B. Natrium- oder Kaliumhydroxid, Kalium-tert. -butylat, -carbonat, -alkoxid, -hydrid, -amid, Ammoniak, Triethylamin, Tributylamin, Pyridin bei -20° bis + 150° C, vorzugsweise bei 0° - 80° C zur Reaktion gebracht und dieses Reaktionsgemisch mit einer Verbindung der Formel 4 oder dessen aktivierter Form umgesetzt. Die Umsetzung erfolgt in einem inerten Lösungsmittel, wie z.B. Methylenchlorid, Methanol, Ethanol, Aceton, Essigsäureethylester, Toluol, Tetrahydrofuran, Acetonitril, N,N-Dimethylformamid, N,N-Dimethylacetatamid, Nitromethan, Dimethylsulfoxid oder Gemischen dieser Lösungsmittel.

Als weitere Methode zur Herstellung der erfindungsgemäßen Verbindungen der Formel Ic kann die Kondensation von Verbindungen der Formel 4 mit den Sulfonamid- bzw. Carbonsäureamid- Derivaten der Formel 5 durch N,N'-Dicyclohexylcarbodiimid und 4-N,N-Dimethylaminopyridin, wie in J.Med.Chem. 1992, 35, 800-804 bei der Herstellung von 5-Sulfonylimiden des Pyridin-2,5-dicarbonsäure-2-methylesters beschrieben, Verwendung finden.

Die weiteren aus der Peptidchemie bekannten Kondensationsmethoden und Kondensationsreagenzien können ebenfalls angewendet werden.

Verbindungen der Formel I, in denen R⁴ -CH₂OR⁵
- X: -CO-und
- Y: SO₂ bedeutet,
wurden hergestellt, indem
i) die 6-Halomethylpyridin-Derivate der Formel 12 mit den Reagenzien MOR⁵ umgesetzt wurden, oder
ii) die 6-Hydroxymethylpyridin-Derivate der Formel 13 mit den SulfonamidDerivaten der Formel 9 umgesetzt wurden, oder
iii) die 6-Hydroxymethylpyridin-3-carbonsäureamid-Derivate der Formel 15 mit den Sulfonsäure-Derivaten der Formel 2 umgesetzt wurden, oder
iv) Pyridin-2-carbonsäure(ester) der Formel 14 reduziert wurden (vgl. Schema 3),
wobei die Verbindungen der Formeln 13 bzw. 15 ihrerseits aus den Verbindungen der Formel 6 nach den bekannten Methoden hergestellt wurden.

Schema 3 verdeutlicht die Herstellung von Verbindungen der Formel Id oder Id', in denen X = -CO- und Y = SO₂ bedeutet:

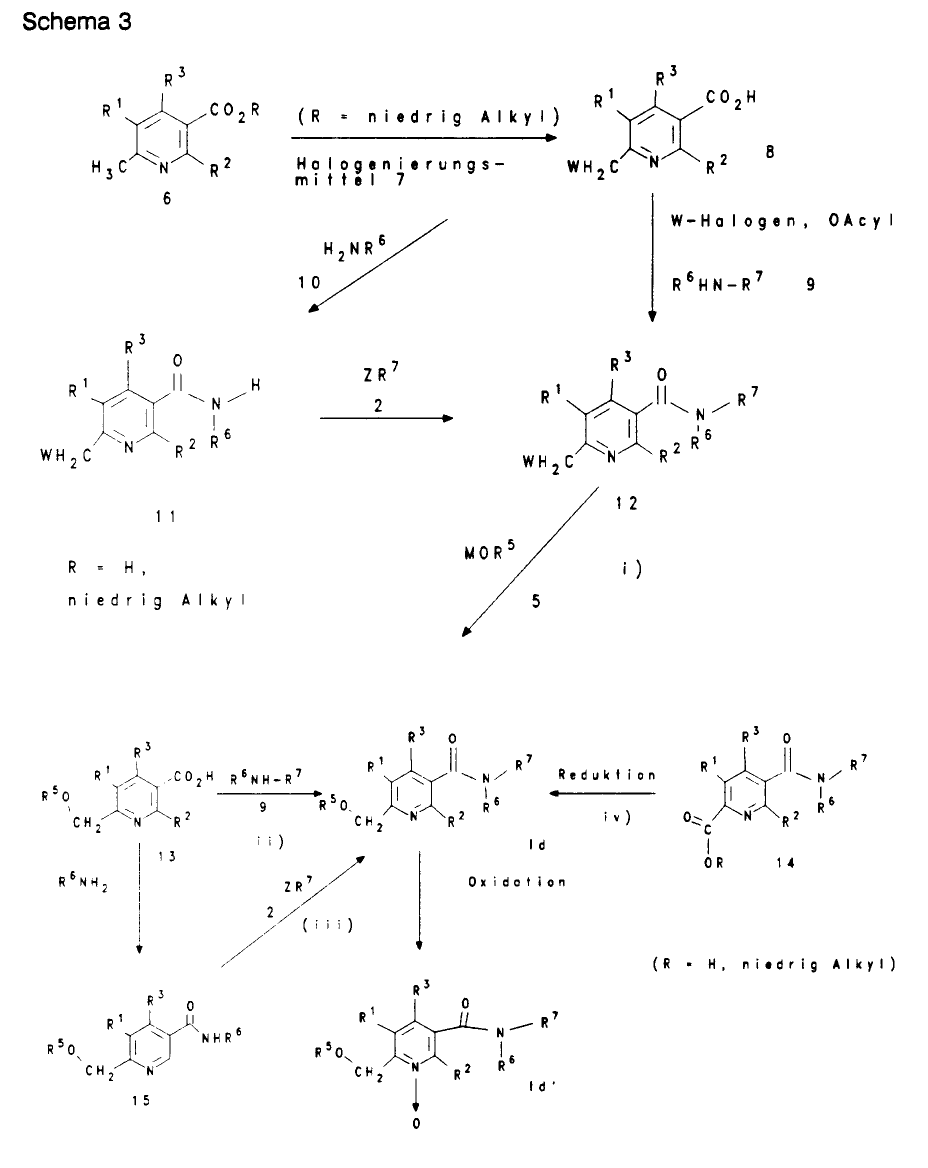

Die weitere Umsetzung zu den Pyriden-N-Oxiden der Formel Ia' ist bereits in Zusammenhang mit Schema 1 erläutert.

Die oben beschriebene Reaktionsfolge gemaß Schema 2 zur Herstellung von in 5-Stellung eine -CO-NR⁶-YR⁸-Gruppe aufweisenden Verbindungen der Formeln Ic bzw. Ic' läßt sich auch zur Herstellung solcher Verbindungen anwenden, die diese Gruppe in 4-Stellung aufweisen. Begonnen wird dann mit Verbindungen der allgemeinen Formel 4:

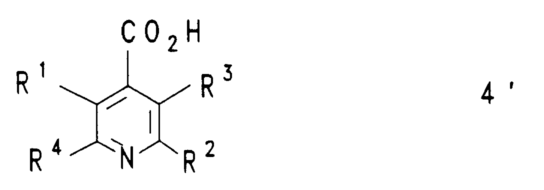

Zur Herstellung von erfindungsgemaßen Verbindungen gemäß der allgemeinen Formel I (Ia,Ia',Ib, Ib', Ic, Ic') nach den Schemata 1 und 2 werden Verbindungen eingesetzt, in denen R⁶ Wasserstoff bedeutet. Die Salzbildung, nach der R⁶ ein physiologisch verwendbares Kation bedeutet, erfolgt bevorzugt im Anschluß. Als Salzbildner kommen bevorzugt N-Alkylamine, (Hydroxyalkyl)amine und (Alkoxyalkyl)amine, wie z.B. 2-Ethanolamin, 3-Propanolamin, 2-Methoxyethylamin, 2-Ethoxyethylamin und α,α,α-Tris-(hydroxymethyl)-methylamin (= Trispuffer, Tromethan) oder auch basische Aminosäuren, wie z.B. Histidin, Arginin und Lysin in Frage.

Schema 4 verdeutlicht die Herstellung der Verbindungen der Formel Id, in denen
- X: CO und
- Y: CONR⁹ bedeuten.

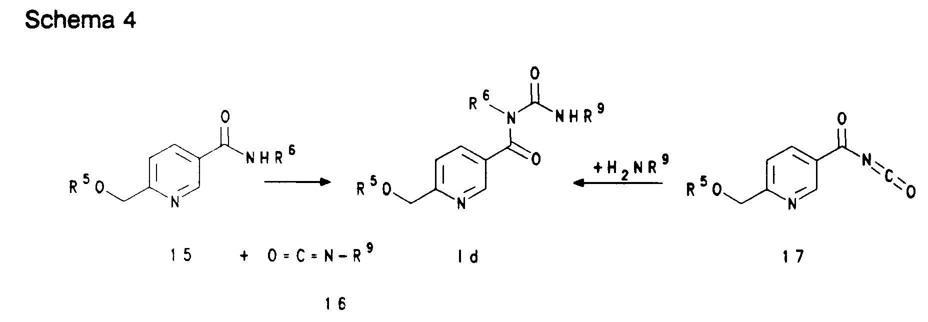

Ihre Synthese erfolgt
i) durch Umsetzung der Amide der Formel 15 mit entsprechenden Isocyanaten der Formel 16, oder
ii) durch Reaktion der Carbonylisocyanate der Formel 17 mit den Aminen H₂NR⁹
und ggf. anschließender Oxidation zum N-Oxid (Id').

Die erfindungsgemäßen Verbindungen der Formel I besitzen wertvolle pharmakologische Eigenschaften und zeigen antifibrotische Wirksamkeit; insbesondere in der Leber.

Die antifibrotische Wirkung kann im Modell der Tetrachlorkohlenstoff-induzierten Leberfibrose bestimmt werden. Dazu werden Ratten mit CCl₄ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben- analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentration 1 - 100 mg/kg wirksam.

Die Aktivität der Fibrogenese kann durch radioimmunologische Bestimmung des N-terminalen Propeptids des Kollagens Typ-III oder der N- bzw. C-terminalen Quervernetzungsdomäne des Kollagens-Typ-IV (7s-Kollagen bzw. Typ-IV-Kollagen NC₁) im Serum bestimmt werden.

Zu diesem Zweck wurden die Hydroxyprolin-, Prokollagen-III-Peptid-, 7s-Kollagen- und Typ-IV-Kollagen-NC-Konzentrationen in der Leber von
a) unbehandelten Ratten (Kontrolle)
b) Ratten, denen Tetrachlorkohlenstoff verabreicht wurde (CCl₄-Kontrolle)
c) Ratten, denen zunächst CCl₄ und anschließend eine erfindungsgemäße Verbindung verabreicht wurde
gemessen (diese Testmethode wird beschrieben von Rouiller, C., experimental toxic injury of the liver; in The Liver, C. Rouiller, Vol. 2, 5. 335-476, New York, Academic Press, 1964).

Die Verbindungen der Formel I können als Medikamente in Form pharmazeutischer Präparate Verwendung finden, welche sie gegebenenfalls zusammen mit verträglichen pharmazeutischen Trägern enthalten. Die Verbindungen können als Heilmittel, z. B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z. B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 - 25 mg/kg/Tag, vorzugsweise 1 - 5 mg/kg/Tag oder parenteral in Dosen von 0,01 - 5 mg/kg/Tag, vorzugsweise 0,01 - 2,5 mg/kg/Tag, insbesondere 0,5 - 1,0 mg/kg/Tag, appliziert werden. Die

Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Stoffwechselstörungen eingesetzt werden.

Ein weiterer Gegenstand der Erfindung sind Arzneimitel, die ein oder mehrere erfindungsgemäße Verbindungen der Formel I und/oder deren physiologisch verträgliche Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägestoffe für die gewünschte Arzneimittelformulierung sind beispielsweise neben Lösemitteln, Gelbildner, Suppositoriengrundlagen, Tabletten-Hilfsstoffen und anderen Wirkstoffträgern auch Antioxidantien, Dispergiermittel, Emulgatoren, Entschäumer, Geschmachskorrigentien, Konservierungsmittel, Lösungsvermittler oder Farbstoffe.

Die folgenden Beispiele sollen die Erfindung erläutern.

### Beispiel 1

5-[(4-Fluorphenylsulfonyl)amino]-2-hydroxymethyl-pyridin
a) 5-[(4-Fluorphenylsulfonyl)amino]-pyridin-2-carbonsäuremethylester
   3,8 g (25 mmol) 5-Amino-pyridin-2-carbonsäuremethylester werden in 75 ml wasserfreiem Pyridin gelöst und portionsweise mit 5,8 g (30 mmol) 4-Fluorbenzolsulfonsäurechlorid versetzt, wobei sich die Temperatur der Reaktionslösung auf 35 °C erhöht. Nach 1 h wird im Vakuum eingeengt, der Rückstand mit Wasser verrieben, abgesaugt, mit Wasser gewaschen und getrocknet. Man erhält 7,3 g Produkt, Fp. 183-185° C.
b) 2,6 g (8,4 mmol) der vorstehenden Verbindung werden in 100 ml wasserfreiem 1,4-Dioxan gelöst und unter Rühren bei 15 - 20 °C mit 20 ml einer 1 m Lösung von Lithiumaluminiumhydrid in Tetrahydrofuran versetzt. Das Reaktionsgemisch wird langsam mit Ethylacetat, dann mit ges. wäßriger NH₄Cl-Lösung versetzt und auf pH 3 eingestellt. Die organische Phase wird abdekantiert, der Rückstand mehrfach mit Ethylacetat gewaschen, die vereinigten organischen Phasen getrocknet, eingeengt und das ölige Rohrprodukt mit Ethylacetat/Methanol (19:1) an Kieselgel (70 - 200 µ) chromatographiert. Aus entsprechenden Fraktionen erhält man 1,6 g der Titelverbindungen in Form farbloser Kristalle, Fp. 88 - 90 °C.

### Beispiel 2

4-[((Phenylsulfonyl)amino)carbonyl]-6-benzyloxymethyl-pyridin
a) 6-Chlormethylpyridin-4-carbonsäuremethylester-hydrochlorid
   Unter Rühren werden zu einer Lösung von 16,7 g (0,01 mol) 2-Hydroxymethylpyridin-4-carbonsäuremethylester in 500 ml Dichlormethan unter Eiskühlung 30 ml Thionylchlorid, gelöst in 70 ml Dichlormethan, zugetropft. Nach Erwärmen auf Raumtemperatur wird nach 1 h gerührt, im Vakuum eingeengt, der Rückstand mit Diethylester behandelt und 23 g des Produktes in Form farbloser Kristalle gewonnen, Fp. 116 - 118 °C.
b) 6-Benzyloxymethylpyridin-4-carbonsäure
   60 ml (0,6 mol) Benzylalkohol werden in 500 ml N,N-Dimethylacetamid portionsweise mit 14,4 g (ca. 0,5 mol) 80 % Natriumhydrid versetzt. Man rührt 30 min bei 60°C, kühlt auf 20°C, gibt portionsweise 23 g (0,1 mol) der Verbindung aus Beispiel 2a zu, rührt 2 h bei 75 °C. Anschließend gibt man 300 ml Wasser zu, engt die dunkle Lösung im Vakuum ein, gibt weitere 100 ml Wasser zu, versetzt mit 2N NaOH bis zur klaren Lösung, filtriert, und bringt das Filtrat mit 2N HCl auf pH 4, saugt den Niederschlag ab, wäscht mit Wasser nach, trocknet und erhält 10,8 g Produkt, Fp. 173 - 175°C.
c) 2,4 g (10 mmol) der vorstehenden Verbindung werden in 50 ml wasserfreiem Tetrahydrofuran suspendiert, bei 0°C mit 1,5 ml (1,1 g, 11 mmol) Triethylamin versetzt. Nach 30 min tropft man bei 0°C 1,35 ml (1,32 g, 11 mmol) Pivaloylchlorid zu und rührt 2 h bei 0°C.

In einem zweitem Reaktionsgefäß werden 1,7g (11 mmol) Benzolsulfonsäureamid in 50 ml wasserfreiem Tetrahydrofuran mit 1,4 g (11 mmol) Kalium-tert.butylat versetzt. Man erwärmt auf 50°C, rührt 30 min bei dieser Temperatur und kühlt auf 20°C ab. Diese Suspension wird über einen Tropftrichter zur obigen Reaktionslösung gegeben, 1 h bei 20°C, 2 h bei 50°C und 2 h bei 70°C gerührt. Das Lösungsmittel wird im Vakuum abdestilliert, der Rückstand mit gesättigter wäßriger NaHCO₃-Lösung versetzt und mit Dichlormethan extrahiert. Aus der wäßrigen Phase erhält man nach Ansäuern 0,4 g Edukt zurück. Die organische Phase wird eingeengt und der Rückstand mit Ethylacetat/Methanol an Kieselgel chromatographiert. Der Rückstand entsprechender Fraktionen wird mit Diethylether zur Kristallisation gebracht und man erhält 1,7 g der Titelverbindung als kristalline Substanz, Fp. ca. 80°C.

### Beispiel 3

5-[4-(Chlorphenylsulfonyl)amino]-2-methyl-pyridin
Zu 4,5 g (41,7 mmol) 5-Amino-2-methyl-pyridin werden in 40 ml wasserfreiem Pyridin unter Rühren 11,4 g (54 mmol) 4-Chlorbenzolsulfonsäurechlorid, gelöst in 15 ml Pyridin, hinzugetropft, wobei die Temperatur auf 35 - 40°C ansteigt. Nach 30 min wird im Vakuum eingeengt, der Rückstand mit Wasser behandelt, das kristalline Produkt abgesaugt und aus Ethanol/Wasser (2 : 1) umkristallisiert. Man erhält 10, 2 g der Titelverbindung.
Fp. 158 - 160°C.

### Beispiel 4

5-[(4-Chlorphenylsulfonyl)amino]-2-hydroxymethyl-pyridin
9,9 g (35 mmol) der Verbindung aus Beispiel 3 werden in 400 ml Dichlormethan unter Rühren portionsweise mit 9,4 g (39 mmol) 3-Chlorperbenzoesäure versetzt. Nach 30 min bei 20°C wird Ammoniak-Gas eingeleitet und das 5-[4-Chlorphenylsulfonyl)amino]-2-methyl-pyridin-N-oxid im Gemisch mit 3-Chlorbenzoesäureammoniumsalz kristallin erhalten, abgesaugt und getrocknet.

Dieser Rückstand wird in 60 ml Eisessig gelöst, bei 80°C unter Rühren 70 ml Acetanhydrid hinzugetropft, 1 h bei 100 - 105°C gerührt, auf 80°C abgekühlt, 100 ml Methanol zugetropft, im Vakuum eingeengt, der Rückstand in wenig Methanol gelöst und in 250 ml 1,5 N methanolische Natronlauge eingetragen. Anschließend wird im Vakuum eingeengt, mit 200 ml Wasser versetzt, mit wäßriger HCl auf pH 6 angesäuert, harzige Nebenprodukte abgetrennt, und mit Dichlormethan extrahiert. Der Rückstand der organischen Phase (ca. 4g) wird mit Ethylacetat/Methanol (10 : 1) an Kieselgel chromatographiert. Das Produkt wird aus entsprechenden Fraktionen mit Ethylacetat zur Kristallisation gebracht. Man erhält 2,2 g des Produktes, Fp. 156 - 157°C.

### Beispiel 5

5-[N-(4-Chlorphenylsulfonyl)-N-(2-methyl-benzoyl)amino]-2-(2-methylbenzoyloxymethyl)pyridin
0,45 g (15 mmol) der Titelverbindung aus Beispiel 4 werden bei 20°C in 100 ml wasserfreiem Dichlormethan unter Rühren mit 0,22 ml (1,65 mmol) Triethylamin und 0,1 g 4-N,N-Dimethylaminopyridin versetzt. Nach 15 min tropft man 0,4 ml (465 mg; 3,0 mmol) 2-Methylbenzoylchlorid zu, rührt 30 min, engt ein, behandelt den Rückstand mit Wasser, nimmt mit Ethylacetat auf, trocknet, engt ein, chromatographiert mit Ethylacetat an Kieselgel und bringt den Rückstand entsprechender Fraktionen mit Diisopropylether/ Petrolether zur Kristallisation. Man erhält 600 mg der Titelverbindung, Fp. 113 - 115°C.

### Beispiel 6

5-[(4-Fluorphenylsulfonyl)amino]-2-methyl-pyridin
wird analog Beispiel 3 erhalten, Fp. 176 - 180°C (aus Wasser)

### Beispiel 7

5-[(4-Fluorphenylsulfonyl)amino]-2-methyl-pyridin-N-oxid
wird aus der Verbindung von Beispiel 6 und m-Chlorperbenzoesäure erhalten
Fp. 123 - 124°C (aus Diethylether)

### Beispiel 8

5-[(4-Methoxyphenylsulfonyl)amino]-2-methyl-pyridin
wird analog Beispiel 3 erhalten, Fp. 112 - 114°C (aus Wasser)

### Beispiel 9

5-[(4-Methoxyphenylsulfonyl)amino]-2-hydroxymethyl-pyridin
wird analog Beispiel 4 aus der in Beispiel 8 beschriebenen Verbindung erhalten, Fp. 119- 121°C
Die Beispiele 10 - 15 werden analog Beispiel 3 aus 5-Amino-2-methylpyridin und den entsprechenden Sulfonsäurechloriden erhalten. Die Produkte werden jeweils aus Wasser isoliert:

### Beispiel 10

5-[(4-Trifluormethylphenylsulfonyl)amino]-2-methyl-pyridin,
Fp. 131 - 133°C

### Beispiel 11

5-[(4-Trifluormethoxyphenylsulfonyl)amino]-2-methyl-pyridin,
Fp. 68 - 70°C

### Beispiel 12

5-[(4-(2,2,2-Trifluorethyloxy)phenyl-sulfonyl)amino]-2-methyl-pyridin,
Fp. 116 - 118°C

### Beispiel 13

5-[(3-Fluorphenylsulfonyl)amino]-2-methyl-pyridin,
Fp. 146 - 148°C

### Beispiel 14

5-[(3,5-Bis-[trifluormethyl]phenylsulfonyl)amino]-2-methyl-pyridin,
Fp. 194 - 197°C

### Beispiel 15

5-[(3-Trifluormethylphenylsulfonyl)amino]-2-methyl-pyridin,
Fp. 95 - 97°C
Die Beispiele 16 - 20 werden analog Beispiel 4 aus den Verbindungen der Beispiele 10 - 12, 14 und 15 erhalten.

### Beispiel 16

5-[(4-Trifluormethylphenylsulfonyl)amino]-2-hydroxymethyl-pyridin,
Fp. 156 - 158°C; aus Diethylether

### Beispiel 17

5-[(4-Trifluormethoxyphenylsulfonyl)amino]-2-hydroxymethyl-pyridin,
Fp. 115 - 117°C; aus Diethylether

### Beispiel 18

5-[(4-(2,2,2-trifluorethyloxy)phenylsulfonyl)amino]-2-hydroxymethyl-pyridin,
Fp. 122 -123°C; aus Diethylether

### Beispiel 19

5-[(3,5-Bis[trifluormethyl]phenylsulfonyl)amino]-2-hydroxymethyl-pyridin,
Fp. 173 - 174°C; aus Diethylether

### Beispiel 20

5-[(3-Trifluormethylphenylsulfonyl)amino]-2-hydroxymethyl-pyridin,
Fp. 115 - 116°C; aus Diethylether
Folgende Verbindungen der Formel I, in denen X = -CO- bedeutet, sind analog den genannten Beispielen und entsprechend den Schemata 2 und 3 hergestellt.

### Beispiel 21

3-[((Phenylsulfonyl)amino)carbonyl]-6-benzyloxymethyl-pyridin
a) 6-Benzyloxymethylpyridin-3-carbonsäure
   Unter Stickstoffatmosphäre werden 0,93 g (40,5 mmol) Natrium bei 50°C in 60 ml Benzylalkohol gelöst. Man läßt auf 0°C abkühlen, gibt 3,0 g (16,2 mmol) 6-Chlormethyl-pyridin-3-carbonsäuremethylester (hergestellt durch Chlorierung von 6-Methyl-pyridin-3-carbonsäuremethylester mit Trichlorisocyanursäure), gelöst in 20 ml Benzylalkohol, zu und gibt 0,243 g (1,62 mmol) Natriumjodid zu und erhitzt die Mischung 8 h auf 80 - 90°C.
   Nach dem Abkühlen gießt man auf Wasser, läßt 8 h bei 50°C rühren, gibt 200 ml Ethylacetat hinzu, extrahiert die organische Phase 2x mit verdünnter NaOH. Die vereinigte wäßrige Phase wird bei 0°C mit konz. HCl angesäuert, das Produkt abgesaugt, mit Wasser gewaschen und bei 60°C im Vakuum getrocknet. Es werden 1,4 g der Verbindung (Fp. 121 - 122°C) erhalten.
b) 1,22 g (5 mmol) der vorstehenden Verbindung werden in 100 ml wasserfreiem Dichlormethan mit 0,79 (5mmol) Benzolsulfonsäureamid, 1,03 g (5 mmol) N, N'-Dicyclohexylcarbodimid und 0,61 g (5 mmol) 4-N, N-Dimethylaminopyridin versetzt und der Ansatz 20 h bei 20°C gerührt. Dann wird der Feststoff abgetrennt, das Filtrat mit wäßriger NaHCO₃-Lösung behandelt, die wäßrige Phase 2x mit Diethylether gewaschen und mit konz. wäßriger HCl auf pH 2 - 3 gebracht. Nach Absaugen, Waschen mit Wasser und Trocknen werden 0,9 g der Titelverbindung erhalten, Fp. 178 - 179°C.

### Beispiel 22

3-[((Phenylsulfonyl)amino)carbonyl]-6-acetoxymethyl-pyridin

### Beispiel 23

3-[((Phenylsulfonyl)amino)carbonyl]-6-(2-methylpropionyl)oxymethyl-pyridin

### Beispiel 24

3-[((Phenylsulfonyl)amino)carbonyl]-6-cyclohexanoyl oxymethyl-pyridin

### Beispiel 25

3-[((Phenylsulfonyl)amino)carbonyl]-6-benzoyloxymethyl-pyridin

### Beispiel 26

3-[((Phenylsulfonyl)amino)carbonyl]-6-hydroxymethyl-pyridin

### Beispiel 27

3-[((Phenylsulfonyl)amino)carbonyl)-6-(2-methylbenzoyl)oxymethyl-pyridin

### Beispiel 28

3-[((Methylsulfonyl)amino)carbonyl]-6-hydroxymethyl-pyridin

### Beispiel 29

3-[(Methylsulfonyl)amino)carbonyl]-6-methoxymethyl-pyridin

### Beispiel 30

3-[((Methylsulfonyl)amino)carbonyl]-6-ethyloxymethyl-pyridin

### Beispiel 31

3-[((Methylsulfonyl)amino)carbonyl]-6-benzoyloxymethyl-pyridin

### Beispiel 32

3-[((Methylsulfonyl)amino)carbonyl]-6-(2-methylbenzoyl)oxymethyl-pyridin

### Beispiel 33

3-[((n-Butylsulfonyl)amino)carbonyl]-6-(2-methylbenzoyl)oxymethyl-pyridin

### Beispiel 34

3-[((n-Butylsulfonyl)amino)carbonyl]-6-acetoxymethyl-pyridin

### Beispiel 35

3-[((4-[2,2,2-Trifluorethyloxy]phenylsulfonyl)amino)carbonyl]-6-hydroxymethyl-pyridin

### Beispiel 36

3-[((4-[2,2,2-Trifluorethyloxy]phenylsulfonyl)amino)carbonyl]-6-methoxymethyl-pyridin

### Beispiel 37

3-[((4-2,2,2-Trifluorethyloxy]phenylsulfonyl)amino)carbonyl]-6-ethyloxymethyl-pyridin

### Beispiel 38

3-[((4-[2,2,2-Trifluorethyloxy]phenylsulfonyl)amino)carbonyl]-6-benzyloxymethyl-pyridin

### Beispiel 39

3-[((4-[2,2,2-Trifluorethyloxy]phenylsulfonyl)amino)carbonyl]-6-isopropyloxymethyl-pyridin

### Beispiel 40

3-[((4-[2,2,2-Trifluorethyloxy]phenylsulfonyl)amino)carbonyl]-6-acetoxymethyl-pyridin

### Beispiel 41

3-[((4-[2,2,3,3,4,4,4-Heptafluorbutyloxy]phenylsulfonyl)amino)carbonyl]-6-hydroxymethyl-pyridin

### Beispiel 42

3-[((4-[2,2,3,3,4,4,4-Heptafluorbutyloxy]phenylsulfonyl)amino)carbonyl]-6-ethyloxymethyl-pyridin

### Beispiel 43

3-[((4-Methoxyphenylsulfonyl)amino)carbonyl]-6-ethyloxymethyl-pyridin

### Beispiel 44

3-[((4-Methoxyphenylsulfonyl)amino)carbonyl]-6-benzyloxymethyl-pyridin

### Beispiel 45

3-[((4-Methoxyphenylsulfonyl)amino)carbonyl]-6-acetoxymethyl-pyridin

### Beispiel 46

3-[((4-Methoxyphenylsulfonyl)amino)carbonyl]-6-benzoyloxymethyl-pyridin

### Beispiel 47

3-[((4-[2,2,3,3,4,4,4-Heptafluorbutyloxy]phenylsulfonyl)amino)carbonyl]-6-(2-methylbenzoyl)oxymethyl-pyridin

### Beispiel 48

3-[((4-[2,2,3,3,4,4,4-Heptafluorbutyloxy]phenylsulfonyl)amino)carbonyl]-6-isopropyloxymethyl-pyridin

### Beispiel 49

3-[((4-Phenoxy-phenylsulfonyl)amino)carbonyl]-6-hydroxymethyl-pyridin
a) 6-Chlormethyl-pyridin-3-carbonsäure
   1,28 g (32 mmol) feste Natronlauge -Plätzchen werden bei 0°C in 250 ml Methanol gelöst und 5,92 g (32 mmol) 6-Chlormethyl-pyridin-3-carbonsäure-methylester (hergestellt durch Chlorierung von 6-Methyl-pyridin-3-carbonsäure-methylester mit Trichlorisocyanursäure) hinzugegeben. Nachdem 18 h gerührt wurde, engt man ein, nimmt mit gesättigter, wäßriger Na-bicarbonat-lösung auf, extrahiert mit Ethylacetat, säuert die wäßrige Phase mit conc. Salzsäure an und saugt 2,43 g als erste Produktfraktion ab.
   Fp. = 157 -159°C.
   Die wäßrige Phase wird dreimal mit Ethylacetat extrahiert, die organische Phase getrocknet, eingeengt und man isoliert 0,9 g weiteres Produkt, Fp. 159-161°C. Ausbeute: 3,33 g
b) Die Titelverbindung wird in Analogie zu Beispiel 92 erhalten.
   1,5 g (8,8 mmol) der vorstehenden Verbindung wurden unter N₂-Atmosphäre in 300 ml Dichlormethan mit 2,19 g (8,8 mmol) 4-Phenoxybenzolsulfonsäureamid, 1,81 g (8,8 mmol) N,N'-Dicyclohexylcarbodiimid und 1,07 g (8,8 mmol) 4-N,N-Dimethylaminopyridin 24 h bei 20°C gerührt. Man filtrierte ab, wusch das Filtrat mit 2 n wäßriger HCl, engte die organische Phase ein und erwärmte den Rückstand unter Rühren in einem Gemisch aus 200 ml 1,4-Dioxan und 200 ml 1n wäßriger NaOH-Lösung 5 h auf 80°C (DC-Kontrolle).
   Dann wurde eingeengt, in Wasser aufgenommen, mit Diethylether gewaschen und mit conc. wäßriger HCl aufgesäuert. Das erhaltene Rohprodukt wurde mit Ethylacetat/n-Heptan/MeOH = 5:3:2 an Kieselgel chromatographiert und man erhielt aus entsprechenden Fraktionen 0,8 g der Titelverbindung als farblose Kristalle, Fp. 253-254°C.

### Beispiel 50

3-[((n-Butylsulfonyl)amino)carbonyl]-6-hydroxymethyl-pyridin

### Beispiel 51

3-[((n-Butylsulfonyl)amino)carbonyl]-6-ethyloxymethyl-pyridin

### Beispiel 52

3-[((n-Butylsulfonyl)amino)carbonyl]-6-benzyloxymethyl-pyridin
Folgende Verbindungen, in denen X eine Einfachbindung bedeutet, sind analog Schema 1 hergestellt.

### Beispiel 53

5-((Phenylsulfonyl)amino)-2-acetoxymethyl-pyridin

### Beispiel 54

5-((Phenylsulfonyl)amino)-2-(2-methylpropionyl)oxymethyl-pyridin

### Beispiel 55

5-((Phenylsulfonyl)amino)-2-cyclohexanoyloxymethyl-pyridin

### Beispiel 56

5-((Phenylsulfonyl)amino)-2-benzoyloxymethyl-pyridin

### Beispiel 57

5-((Phenylsulfonyl)amino)-2-benzyloxymethyl-pyridin

### Beispiel 58

5-((Phenylsulfonyl)amino)-2-(2-methylbenzoyl)oxymethyl-pyridin

### Beispiel 59

5-((Methylsulfonyl)amino)-2-hydroxymethyl-pyridin

### Beispiel 60

5-((Methylsulfonyl)amino)-2-methoxymethyl-pyridin

### Beispiel 61

5-((Methylsulfonyl)amino)-2-ethyloxymethyl-pyridin

### Beispiel 62

5-((Methylsulfonyl)amino)-2-benzoyloxymethyl-pyridin

### Beispiel 63

5-((Methylsulfonyl)amino)-2-(2-methylbenzoyl)oxymethyl-pyridin

### Beispiel 64

5-((n-Butylsulfonyl)amino)2-(2-methylbenzoyl)oxymethyl-pyridin

### Beispiel 65

5-((n-Butylsulfonyl)amino)-2-acetoxymethyl-pyridin
Im folgenden sind Verbindungen, in denen X = -CO- und Y = -CONR⁹- bedeuten zusammengestellt. Diese Verbindungen sind analog der in J. Agr. Food Chem. Vol. 21, NO. 3, 1973, S. 348 und der dort zitierten Literatur für die Herstellung von Benzoyl-3-phenyl-harnstoffen aus den entsprechenden Pyridin-4(5)-carbonsäureamid-6-hydroxymethyl-Derivaten hergestellt.

### Beispiel 66

3-[(((N-Phenylamino)carbonyl)amino)carbonyl]-6-methoxymethylpyridin
a) 6-Methoxymethylpyridin-3-carbonsäure-methylester
   52 g (0,28 mol) 6-Chlormethyl-pyridin-3-carbonsäuremethylester werden in 500 ml Methanol mit 54 ml (ca. 0,3 mol) 30%iger Natriummethylat-Lösung und 4,5 g Natriumjodid versetzt und 3 h zum Rückfluß erhitzt. Das Reaktionsgemisch wird i.Vak. eingeengt, der Rückstand mit warmem Diisopropylether extrahiert und mit n-Heptan-Ethylacetat (2:1) an Kieselgel chromatographiert. 42,8 g Produkt, Fp. 34-36°C (aus Petrolether)
b) 6-Methoxymethylpyridin-3-carbonsäureamid
   13,6 g (75 mmol) des vorstehenden Esters werden in 100 ml konzentrierter wäßriger Ammoniaklösung 1 h bei 25°C gerührt. Dann wird unter Eiskühlung mit konzentrierter wäßriger Salzsäure neutralisiert und bis zur beginnenden Kristallisation eingeengt, 6,7 g Produkt, Fp. 150-152°C (aus Wasser)
c) 2,5 g (15mmol) 6-Methoxymethylpyridin-3-carbonsäureamid werden in 50 ml wasserfreiem Xylol suspendiert, mit 2,0 g (1,85 ml; 17 mmol) Phenylisocyanat versetzt und 18 h unter Rühren auf 130°C erwärmt. Dann wird die Reaktionsmischung abgekühlt, vom dunklen Harz abdekantiert, mit 10 ml Methanol versetzt, eingeengt und der Rückstand mit Ethylacetat zur Kristallisation gebracht. Das so erhaltene Rohprodukt wird mit Dichlormethan - im Verlauf der Chromatographie bis zu 10% Methanolanteil- an Kieselgel (70 - 200 µm) chromatographiert. Aus entsprechenden Fraktionen wird die Titelverbindung mit Dichlormethan zur Kristallisation gebracht, 2,3 g Produkt, Fp. 194-196°C.

### Beispiel 67

3-[(((N-Phenylamino)carbonyl)amino)carbonyl]-6-ethyloxymethylpyridin

### Beispiel 68

3-[(((N-n-Butylamino)carbonyl)amino)carbonyl]-6-methoxymethylpyridin
3,3 g (20 mmol) 6-Methoxymethylpyridin-3-carbonsäureamid (zur Herstellung vgl. Beispiel 66 b)) werden in 100 ml wasserfreiem Xylol mit 2,5 g (2,8 ml, 25 mmol) n-Butylisocyanat analog Beispiel 66 c) 8 h auf 130°C erhitzt. Nach DC-Kontrolle gibt man 1 ml weiteres n-Butylisocyanat zu und erwärmt noch 2 h. Vom Ungelösten wird heiß abfiltriert, das Filtrat eingeengt und der Rückstand mit Diisopropylether zur Kristallisation gebracht. Dieses Rohprodukt wird mit Ethylacetat an Kieselgel chromatographiert. Entsprechende Fraktionen werden eingeengt und mit Diisopropylether zur Kristallisation gebracht. Man erhält 2,3 g der Titelverbindung, Fp. 116-118°C.

### Beispiel 69

3-[(((N-Propylamino)carbonyl)amino)carbonyl]-6-benzyloxymethyl-pyridin

### Beispiel 70

3-[(((N-Phenyl-N-methylamino)carbonyl)amino)carbonyl]-6-methoxymethyl-pyridin

### Beispiel 71

3-[(((N-Phenyl-N-ethylamino)carbonyl)amino)carbonyl]-6-ethyloxymethyl-pyridin

### Beispiel 72

3-[(((N-Cyclohexylamino)carbonyl)amino)carbonyl]-6-ethyloxymethyl-pyridin

### Beispiel 73

3-[(((N-Cyclohexyl-N-ethylamino)carbonyl)amino)carbonyl]-6-ethyloxymethyl-pyridin

### Beispiel 74

3-[(((N-n-Butylamino)carbonyl)amino)carbonyl]-6-acetoxymethyl-pyridin

### Beispiel 75

3-[(((N-n-Butylamino)carbonyl)amino)carbonyl]-6-hydroxymethyl-pyridin

### Beispiel 76

3-[(((N-Ethylamino)carbonyl)amino)carbonyl]-6-acetoxymethyl-pyridin

### Beispiel 77

3-[(((N-Ethylamino)carbonyl)amino)carbonyl]-6-hydroxymethyl-pyridin

### Beispiel 78

3-[(((N-[4-Fluorphenyl]amino)carbonyl)amino)carbonyl]-6-hydroxymethyl-pyridin

### Beispiel 79

3-[(((N-[4-Fluorphenyl]amino)carbonyl)amino)carbonyl]-6-ethyloxymethyl-pyridin

### Beispiel 80

3-[(((N-[4-Fluorphenyl]-N-methylamino)carbonyl)amino)carbonyl]-6-hydroxymethyl-pyridin

### Beispiel 81

3-[(((N-[4-Methoxyphenyl]amino)carbonyl)amino)carbonyl]-6-ethyloxymethyl-pyridin

### Beispiel 82

3-[(((N-[4-Fluorphenyl]amino)carbonyl)amino)carbonyl]-6-benzyloxymethyl-pyridin

### Beispiel 83

3-[(((N-Ethylamino)carbonyl)amino)carbonyl]-6-benzyloxymethyl-pyridin

### Beispiel 84

3-[(((N-Phenylamino)carbonyl)amino)carbonyl]-6-acetoxymethyl-pyridin

### Beispiel 85

3-[(((N-Phenylamino)carbonyl)amino)carbonyl]-6-hydroxymethyl-pyridin

### Beispiel 86

3-[(((N-Phenylamino)carbonyl)amino)carbonyl]-6-(2-methylbenzoyl)methyl-pyridin

### Beispiel 87

3-[(((N-Cyclohexylamino)carbonyl)amino)carbonyl]-6-acetoxymethyl-pyridin

### Beispiel 88

3-[(((N-Cyclohexylamino)carbonyl)amino)carbonyl]-6-hydroxymethyl-pyridin

### Beispiel 89

3-[(((N-4-Fluorphenylamino)carbonyl)amino)carbonyl]-6-methoxymethylpyridin
1,7 g (10 mmol) 6-Methoxymethylpyridin-3-carbonsäureamid wird analog den Beispielen 66 und 68 mit 1,8 g (13 mmol) 4-Fluorphenylisocyanat 8 h bei 120°C umgesetzt. Beim Abkühlen ausgefallene gelbe Kristalle werden abgesaugt, mit 50 ml Methanol erhitzt und bei 20°C abgesaugt, gewaschen und getrocknet. Man erhält 2,6 g Produkt, Fp. 222-224°C (aus Methanol)

### Beispiel 90

3-[((Phenylsulfonyl)amino)carbonyl]-6-ethyloxymethyl-pyridin
a) 6- Ethyloxymethyl-pyridin-3-carbonsäure
   Die Verbindung wird analog Beispiel 21 a) aus Natrium, Ethanol, 5 g (27 mmol) 6-Chlormethyl-pyridin-3-carbonsäure-methylester und 0,4 g (2,7 mmol) Natriumjodid erhalten. Man erhält 4,8 g Produkt, Fp. 95-96°C (aus Ethylacetat)
b) Analog Beispiel 49) werden 1,09 g (6 mmol) 6-Ethyloxymethyl-pyridin-3-carbonsäure in 100 ml wasserfreiem Dichlormethan mit 0,94 g (6 mmol) Benzolsulfonsäureamid, 1,24 g (6 mmol) N,N'-Dicyclohexylcarbodiimid und 0,73 g (6 mmol) 4-N,N-Dimethylaminopyridin 20 h bei 20°C gerührt. Man filtriert vom entstandenen N,N'-Dicyclohexyl-harnstoff ab, engt ein, nimmt mit wäßriger Na-bicarbonat Lösung auf, filtriert vom Ungelösten ab und säuert den Rückstand mit conc. Salzsäure an. Nach Absaugen, Nachwaschen mit Wasser und Trocknen erhält man 1,51 g der Titelverbindung, Fp. 180-182°C.

### Beispiel 91

3-[((4-n-Butyloxyphenylsulfonyl)amino)carbonyl]-6-methoxymethyl-pyridin
0,9 g (5,26 mmol) 6-Chlormethyl-pyridin-3-carbonsäure (Herstellung vgl. Beispiel 49 a)) wurden unter N₂-Atmosphäre in 200 ml Dichlormethan mit 1,2 g (5,26 mmol) 4-n-Butyloxybenzolsulfonsäureamid, 1,09 g (5,26 mmol) N,N'-Dicyclohexylcarbodiimid und 0,64 g (5,26 mmol) 4-N,N-Dimethylaminopyridin versetzt und 20 h bei 20°C gerührt.
Man filtriert den N,N'-Dicyclohexylharnstoff ab, engt i. Vak. ein, behandelt 15 min. mit heißem Methanol, engt ein und chromatographiert den Rückstand mit Ethylacetat/Methanol (9:1) an Kieselgel. Aus entsprechenden Fraktionen werden 0,88 g der Titelverbindung kristallin erhalten, Fp. 218-220°C.

### Beispiel 92

3-[((4-n-Butyloxyphenylsulfonyl)amino)carbonyl]-6-hydroxymethyl-pyridin
0,9 g (5,26 mmol) 6-Chlormethyl-pyridin-3-carbonsäure (Herstellung vgl. Beispiel 49 a)) wurden unter N₂-Atmosphäre in 200 ml Dichlormethan suspendiert und mit 1,2 g (5,26 mmol) 4-n-Butyloxybenzolsulfonsäureamid, 1,09 g (5,26 mmol) N,N'-Dicyclohexylcarbodiimid und 0,64 g (5,26 mmol) 4-N,N-Dimethylaminopyridin versetzt. Nach 10 min. entsteht eine klare Lösung, die sich nach 1 h trübt. Das Reaktionsgemisch wird weitere 19 h bei 20°C gerührt. Der ausgefallene N,N'-Dicyclohexylharnstoff wird abfiltriert, das Filtrat eingeengt, der Rückstand in 100 ml 1 n wäßriger Natronlauge 4 h bei 50°C und 2 h bei 80°C gerührt.Man engt i. Vak. ein, gibt Wasser zu, extrahiert zweimal mit Diethylether. Dann wird die wäßrige Phase mit konz. wäßriger HCl angesäuert, eingeengt, mit Toluol versetzt, i. Vak. eingeengt und der Rückstand mit Ethylacetat/Methanol (1:1, 0,75 ml Essigsäure-Zusatz pro Liter) an Kieselgel chromatographiert. Aus entsprechenden Fraktionen erhält man 0,86 g der Titelverbindung, die mit Ethylacetat zur Kristallisation gebracht wird, Fp. 178-179°C.

Die folgenden Beispiele werden analog zu den Beispielen 49 und 92 hergestellt:

### Beispiel 93

3-[((4-Methoxyphenylsulfonyl)amino)carbonyl]-6-hydroxymethyl-pyridin

### Beispiel 94

3-[((4-Ethyloxyphenylsulfonyl)amino)carbonyl]-6-hydroxymethyl-pyridin

### Beispiel 95

3-[((4-i-Propyloxyphenylsulfonyl)amino)carbonyl]-6-hydroxymethyl-pyridin

### Beispiel 96

3-[((4-n-Pentyloxyphenylsulfonyl)amino)carbonyl]-6-hydroxymethyl-pyridin

### Beispiel 97

3-[((4-n-Hexyloxyphenylsulfonyl)amino)carbonyl]-6-hydroxymethyl-pyridin

### Beispiel 98

3-[((4-n-Octyloxyphenylsulfonyl)amino)carbonyl]-6-hydroxymethyl-pyridin

### Beispiel 99

3-[((4-c-Hexyloxyphenylsufonyl)amino)carbonyl]-6-hydroxymethyl-pyridin

### Beispiel 100

3-[((4-(c-Hexylmethyloxy)phenylsulfonyl)amino)carbonyl]-6-hydroxymethyl-pyridin

### Beispiel 101

3-[((4-Methylphenylsulfonyl)amino)carbonyl]-6-hydroxymethyl-pyridin

### Beispiel 102

3-[((4-Ethylphenylsulfonyl)amino)carbonyl]-6-hydroxymethyl-pyridin

### Beispiel 103

3-[((4-n-Propylphenylsulfonyl)amino)carbonyl]-6-hydroxymethyl-pyridin

### Beispiel 104

3-[((4-Biphenylsulfonyl)amino)carbonyl]-6-hydroxymethyl-pyridin

### Beispiel 105

3-[((2-Biphenylsulfonyl)amino)carbonyl]-6-hydroxymethyl-pyridin

### Beispiel 106

3-[((4-(4-Fluorphenoxy)phenylsulfonyl)amino)carbonyl]-6-hydroxymethyl-pyridin

### Beispiel 107

3-(((4-(3-Fluorphenoxy)phenylsulfonyl)amino)carbonyl]-6-hydroxymethyl-pyridin

### Beispiel 108

3-[((4-(4-Chlorphenoxy)phenylsulfonyl)amino)carbonyl]-6-hydroxymethyl-pyridin

### Beispiel 109

3-[((4-(3,5-Dichlorphenoxy)phenylsulfonyl)amino)carbonyl]-6-hydroxymethyl-pyridin

### Beispiel 110

3-[((4-(3-Trifluormethylphenoxy)phenylsulfonyl)amino)carbonyl]-6-hydroxymethyl-pyridin

### Beispiel 111

3-[((4-(4-Trifluormethylphenyloxy)phenylsulfonyl)amino)carbonyl]-6-hydroxymethyl-pyridin

### Beispiel 112

3-[((3-(4-Fluorphenoxy)phenylsulfonyl)amino)carbonyl]-6-hydroxymethyl-pyridin

### Beispiel 113

3-[((3-(3-Fluorphenoxy)phenylsulfonyl)amino)carbonyl]-6-hydroxymethyl-pyridin

### Beispiel 114

3-[((3-(4-Chlorphenoxy)phenylsulfonyl)amino)carbonyl]-6-hydroxymethyl-pyridin

### Beispiel 115

3-[((3-(3,5-Dichlorphenoxy)phenylsulfonyl)amino)carbonyl]-6-hydroxymethyl-pyridin

### Beispiel 116

3-[((4-(2-(Phenyloxy)ethyloxy)phenylsulfonyl)amino)carbonyl]-6-hydroxymethyl-pyridin

### Beispiel 117

3-[((4-(2-(n-Butyloxy)ethyloxy)phenylsulfonyl)amino)carbonyl]-6-hydroxymethyl-pyridin

### Beispiel 118

3-[((4-((4-Phenyl-n-butyl)aminocarbonyl)phenylsulfonyl)amino)carbonyl]-6-hydroxymethyl-pyridin

### Beispiel 119

3-[((4-(Ethylaminocarbonyl)phenylsulfonyl)amino)carbonyl]-6-hydroxymethyl-pyridin

### Beispiel 120

3-[((4-(n-Propylaminocarbonyl)phenylsulfonyl)amino)carbonyl]-6-hydroxymethyl-pyridin

### Beispiel 121

3-[((4-(n-Hexylaminocarbonyl)phenylsulfonyl)amino)carbonyl]-6-hydroxymethyl-pyridin

### Beispiel 122

3-[((4-(N,N-Di-n-butylaminocarbonyl)phenylsulfonyl)amino)carbonyl]-6-hydroxymethyl-pyridin

### Beispiel 123

3-[((4-(Cyclohexylaminocarbonyl)phenylsulfonyl)amino)carbonyl]-6-hydroxymethyl-pyridin

### Beispiel 124

3-[((4-(Benzylaminocarbonyl)phenylsulfonyl)amino)carbonyl]-6-hydroxymethyl-pyridin

### Beispiel 125

3-[((4-((2-Phenylethyl)aminocarbonyl)phenylsulfonyl)amino)carbonyl]-6-hydroxymethyl-pyridin-ammonium-salz
3,42 g (20 mmol) 6-Chlormethyl-pyridin-3-carbonsäure wurden unter N₂-Atmosphäre in 200 ml Dichlormethan mit 6,1 g (20 mmol) 4-((2-Phenylethyl)aminocarbonyl)benzolsulfonamid, 2,45 g (20 mmol) 4-N,N-Dimethylaminopyridin und 4,12 g (20 mmol) N,N'-Dicyclohexylcarbodiimid versetzt und 20 h bei 20°C gerührt. Anschließend wurde der N,N'-Dicyclohexylharnstoff abfiltriert, mit Dichlormethan gewaschen, das Filtrat eingeengt, mit 200 ml 1 n HCl versetzt, das ausgefallene Produkt abgesaugt und mit Wasser gewaschen.
Das so erhaltene Zwischenprodukt (13 g) wurde in 200 ml 1,4-Dioxan und 300 ml 1 n NaOH 5 h bei 80°C gerührt. Anschließend wurde eingeengt, mit Wasser aufgenommen, mit Diethylether extrahiert und die wäßrige Phase mit conc. wäßriger HCl angesäuert.
Das ausgefallene Produkt wurde abgesaugt, sodann in Methanol gelöst und mit methanolischer Ammoniaklösung versetzt.
Nach Zugabe von Diethylether wurde kristallines Produkt abgesaugt, mit Ether gewaschen und 350 mg der Titelverbindung erhalten, Fp. 188 - 190°C. Aus der Mutterlauge (7 g) konnte nach Chromatographie weiteres Produkt isoliert werden.

### Beispiel 126

3-[((4-((3-Phenylpropyl)aminocarbonyl)phenylsulfonyl)amino)carbonyl]-6-hydroxymethyl-pyridin

### Beispiel 127

3-[((4-((2-Methoxyethyl)aminocarbonyl)phenylsulfonyl)amino)carbonyl]-6-hydroxymethyl-pyridin

### Beispiel 128

3-[((4-((2-Ethoxyethyl)aminocarbonyl)phenylsulfonyl)amino)carbonyl]-6-hydroxymethyl-pyridin

### Beispiel 129

3-[((4-((3-Ethoxypropyl)aminocarbonyl)phenylsulfonyl)amino)carbonyl]-6-hydroxymethyl-pyridin

### Beispiel 130

3-[((3-(4-Phenyl-n-butyl)aminocarbonyl)phenylsulfonyl)amino)carbonyl]-6-hydroxymethyl-pyridin

### Beispiel 131

3-[((3-(Ethylaminocarbonyl)phenylsulfonyl)amino)carbonyl]-6-hydroxymethyl-pyridin

### Beispiel 132

3-[((3-(n-Propylaminocarbonyl)phenylsulfonyl)amino)carbonyl]-6-hydroxymethyl-pyridin

### Beispiel 133

3-[((3-(n-Hexylaminocarbonyl)phenylsulfonyl)amino)carbonyl]-6-hydroxymethyl-pyridin

### Beispiel 134

3-[((3-(N,N-Di-n-butylaminocarbonyl)phenylsulfonyl)amino)carbonyl]-6-hydroxymethyl-pyridin

### Beispiel 135

3-[((3-(Cyclohexylaminocarbonyl)phenylsulfonyl)amino)carbonyl]-6-hydroxymethyl-pyridin

### Beispiel 136

3-[((3-(Benzylaminocarbonyl)phenylsulfonyl)amino)carbonyl]-6-hydroxymethyl-pyridin

### Beispiel 137

3-[((3-(2-Phenylethyl)aminocarbonyl)phenylsulfonyl)amino)carbonyl]-6-hydroxymethyl-pyridin

### Beispiel 138

3-[((3-(3-Phenylpropyl)aminocarbonyl)phenylsulfonyl)amino)carbonyl]-6-hydroxymethyl-pyridin

### Beispiel 139

3-[((3-(2-Methoxyethyl)aminocarbonyl)phenylsulfonyl)amino)carbonyl]-6-hydroxymethyl-pyridin

### Beispiel 140

3-[((3-(2-Ethoxyethyl)aminocarbonyl)phenylsulfonyl)amino)carbonyl]-6-hydroxymethyl-pyridin

### Beispiel 141

3-[((3-(3-Ethoxypropyl)aminocarbonyl)phenylsulfonyl)amino)carbonyl]-6-hydroxymethyl-pyridin

### Beispiel 142

3-[((4-(4-Phenyl-n-butanoylamino)phenylsulfonyl)amino)carbonyl]-6-hydroxymethyl-pyridin

### Beispiel 143

3-[((4-(Acetylamino)phenylsulfonyl)amino)carbonyl]-6-hydroxymethyl-pyridin

### Beispiel 144

3-[((4-(n-Propionylamino)phenylsulfonyl)amino)carbonyl]-6-hydroxymethyl-pyridin

### Beispiel 145

3-[((4-(n-Hexanoylamino)phenylsulfonyl)amino)carbonyl]-6-hydroxymethyl-pyridin

### Beispiel 146

3-[((4-(Benzoylamino)phenylsulfonyl)amino)carbonyl]-6-hydroxymethyl-pyridin

### Beispiel 147

3-[((4-(3-(Phenylpropionylamino)phenylsulfonyl)amino)carbonyl]-6-hydroxymethyl-pyridin

### Beispiel 148

3-[((4-(2-Phenylacetylamino)phenylsulfonyl)amino)carbonyl]-6-hydroxymethyl-pyridin

### Beispiel 149

3-[((4-(2-(Acetylamino)ethyl)phenylsulfonyl)amino)carbonyl]-6-hydroxymethyl-pyridin

### Beispiel 150

3-[((4-(2-(Butanoylamino)ethyl)phenylsulfonyl)amino)carbonyl]-6-hydroxymethyl-pyridin

### Beispiel 151

3-[((4-(2-(Benzoylamino)ethyl)phenylsulfonyl)amino)carbonyl]-6-hydroxymethyl-pyridin

### Beispiel 152

3-[((4-(2-((2-Chlor-5-methoxybenzoyl)amino)ethyl)phenylsulfonyl)amino)carbonyl]-6-hydroxymethyl-pyridin-natrium-salz
a) 3-[((4-(2-((2-Chlor-5-methoxybenzoyl)amino)ethyl)phenylsulfonyl)amino)carbonyl]-6-chlormethyl-pyridin-natrium-salz
   5,5 g (15 mmol) 4-(2-((2-Chlor-5-methoxybenzoyl)amino)ethyl)benzolsulfonamid wurden in 300 ml Tetrahydrofuran unter Rühren bei 20°C mit 1,85 g (16,5 mmol) Kalium-tert.butylat versetzt und 30 min bei 50°C gerührt. Zu dieser Lösung tropfte man nach dem Abkühlen eine Lösung von 2,57 g 6-Chlormethylpyridin-3-carbonsäure und 2,7 g (16,5 mmol) N,N'-Carbonyldiimidazol in 70 ml wasserfreiem Tetrahydrofuran, die zuvor ebenfalls für 30 min bei 50°C gerührt worden war.
   Man rührte das Reaktionsgemisch 2 h bei 40 - 50°C, engte nach dem Abkühlen im Vakuum ein, behandelte den festen Rückstand mit Wasser, mit 200 ml gesättigter wäßriger Na-bicarbonat-Lösung und mit 200 ml Dichlormethan und erhielt 7,3 g Produkt, Fp. 192 - 193°C.
b) 2,7 g (5 mmol) der vorstehenden Verbindung wurden in 60 ml 1 n NaOH suspendiert und unter Rühren bei 80°C mit 30 ml 1,4-Dioxan versetzt bis eine klare Lösung entstand. Nach 1 h bei 80°C (DC-Kontrolle) wurde abgekühlt, im Vakuum eingeengt, mit 30 ml Wasser versetzt, einmal mit 100 ml Dichlormethan extrahiert, die wäßrige Phase mit conc. wäßriger HCl auf pH 1 gestellt. Das so erhaltene halbkristalline Produkt wurde in Methanol gelöst, mit wäßriger Na-bicarbonat-Lösung versetzt, eingeengt und der Rückstand mit Ethylacetat/Methanol (5:1) an Kieselgel chromatographiert.
   Aus entsprechenden Fraktionen erhielt man nach Behandeln mit Ethylacetat 0,44 g der farblos kristallinen Titelverbindung, Fp. ab 120°C (unter Schäumen).

### Beispiel 153

3-[(((3-(4-Phenyl-n-butanoylamino)phenylsulfonyl)amino)carbonyl]-6-hydroxymethyl-pyridin

### Beispiel 154

3-[((3-(Acetylamino)phenylsulfonyl)amino)carbonyl]-6-hydroxymethyl-pyridin

### Beispiel 155

3-[((3-(Propionylamino)phenylsulfonyl)amino)carbonyl]-6-hydroxymethyl-pyridin

### Beispiel 156

3-[((3-(n-Hexanoylamino)phenylsulfonyl)amino)carbonyl]-6-hydroxymethyl-pyridin

### Beispiel 157

3-[((3-(Benzoylamino)phenylsulfonyl)amino)carbonyl]-6-hydroxymethyl-pyridin

### Beispiel 158

3-[((3-(3-Phenylpropionylamino)phenylsulfonyl)amino)carbonyl]-6-hydroxymethyl-pyridin

### Beispiel 159

3-[((2-(2-Phenylacetylamino)phenylsulfonyl)amino)carbonyl]-6-hydroxymethyl-pyridin

### Beispiel 160

3-[((3-(2-Acetylamino)ethyl)phenylsulfonyl)amino)carbonyl]-6-hydroxymethyl-pyridin

### Beispiel 161

3-[((3-(2-Butanoylamino)ethyl)phenylsulfonyl)amino)carbonyl]-6-hydroxymethyl-pyridin

### Beispiel 162

3-[((3-(2-Benzoylamino)ethyl)phenylsulfonyl)amino)carbonyl]-6-hydroxymethyl-pyridin

### Beispiel 163

3-[((3-(2-((2-Chlor-5-methoxy-benzoyl)amino)ethyl)phenylsulfonyl)amino)carbonyl]-6-hydroxymethyl-pyridin

### Beispiel 164

3-[((((4-Fluorphenyl)-2-oxyethyl)sulfonyl)amino)carbonyl]-6-hydroxymethyl-pyridin

### Beispiel 165

3-[((((3-Fluorphenyl)-2-oxyethyl)sulfonyl)amino)carbonyl]-6-hydroxymethyl-pyridin

### Beispiel 166

3-[((((2,4-Difluorphenyl)-2-oxyethyl)sulfonyl)amino)carbonyl]-6-hydroxymethyl-pyridin

### Beispiel 167

3-[((((4-Chlorphenyl)-2-oxyethyl)sulfonyl)amino)carbonyl]-6-hydroxymethyl-pyridin

### Beispiel 168

3-[((((3,5-Dichlorphenyl)-2-oxyethyl)sulfonyl)amino)carbonyl]-6-hydroxymethyl-pyridin

### Beispiel 169

3-[((((3-Trifluormethylphenyl)-2-oxyethyl)sulfonyl)amino)carbonyl]-6-hydroxymethyl-pyridin

### Beispiel 170

3-[((((4-Trifluormethylphenyl)-2-oxyethyl)sulfonyl)amino)carbonyl]-6-hydroxymethyl-pyridin

### Beispiel 171

3-[((((4-n-Butyloxyphenyl)-2-oxyethyl)sulfonyl)amino)carbonyl]-6-hydroxymethyl-pyridin

### Beispiel 172

3-[((((2-Ethyloxyphenyl)-2-oxyethyl)sulfonyl)amino)carbonyl]-6-hydroxymethyl-pyridin

### Beispiel 173

3-[(((2-Methoxyethylsulfonyl)amino)carbonyl]-6-hydroxymethyl-pyridin

### Beispiel 174

3-[(((2-Ethyloxyethyl)sulfonyl)amino)carbonyl]-6-hydroxymethyl-pyridin

### Beispiel 175

3-[(((2-Isopropyloxyethyl)sulfonyl)amino)carbonyl]-6-hydroxymethyl-pyridin

### Beispiel 176

3-[(((2-n-Butyloxyethyl)sulfonyl)amino)carbonyl]-6-hydroxymethyl-pyridin

### Beispiel 177

3-[(((2-Benzyloxyethyl)sulfonyl)amino)carbonyl]-6-hydroxymethyl-pyridin

### Beispiel 178

3-[(((2-Phenylethyl)-2-oxyethyl)sulfonyl)amino)carbonyl]-6-hydroxymethyl-pyridin

### Beispiel 179

3-[((1-Naphthylsulfonyl)amino)carbonyl]-6-hydroxymethyl-pyridin

### Beispiel 180

3-[(((4-Methoxy-1-naphthyl)sulfonyl)amino)carbonyl]-6-hydroxymethyl-pyridin

### Beispiel 181

3-[(((5-Ethoxy-1-naphthyl)sulfonyl)amino)carbonyl]-6-hydroxymethyl-pyridin

### Beispiel 182

3-[((2-Biphenylsulfonyl)amino)carbonyl]-6-hydroxymethyl-pyridin

### Beispiel 183

3-[(((4-Methoxy-2-biphenyl)sulfonyl)amino)carbonyl]-6-hydroxymethyl-pyridin

### Beispiel 184

3-[((((4-Fluorphenyl)-3-oxypropyl)sulfonyl)amino)carbonyl]-6-hydroxymethyl-pyridin

### Beispiel 185

3-[((((3-Fluorphenyl)-3-oxypropyl)sulfonyl)amino)carbonyl]-6-hydroxymethyl-pyridin

### Beispiel 186

3-[((((2,4-Difluorphenyl)-3-oxypropyl)sulfonyl)amino)carbonyl]-6-hydroxymethyl-pyridin

### Beispiel 187

3-[((((4-Chlorphenyl)-3-oxypropyl)sulfonyl)amino)carbonyl]-6-hydroxymethyl-pyridin

### Beispiel 188

3-[((((3,5-Dichlorphenyl)-3-oxypropyl)sulfonyl)amino)carbonyl]-6-hydroxymethyl-pyridin

### Beispiel 189

3-[((((3-Trifluormethylphenyl)-3-oxypropyl)sulfonyl)amino)carbonyl]-6-hydroxymethyl-pyridin

### Beispiel 190

3-[((((4-Trifluormethylphenyl)-3-oxypropyl)sulfonyl)amino)carbonyl]-6-hydroxymethyl-pyridin

### Beispiel 191

3-[(((2-Phenylethyl)-2-oxypropyl)sulfonyl)amino)carbonyl]-6-hydroxymethyl-pyridin

### Beispiel 192

3-[((((4-Fluorphenyl)-4-oxy-n-butyl)sulfonyl)amino)carbonyl]-6-hydroxymethyl-pyridin

### Beispiel 193

3-[((((3-Fluorphenyl)-4-oxy-n-butyl)sulfonyl)amino)carbonyl]-6-hydroxymethyl-pyridin

### Beispiel 194

3-[((((2-4-Difluorphenyl)-4-oxy-n-butyl)sulfonyl)amino)carbonyl]-6-hydroxymethyl-pyridin

### Beispiel 195

3-[((((4-Chlorphenyl)-4-oxy-n-butyl)sulfonyl)amino)carbonyl]-6-hydroxymethyl-pyridin

### Beispiel 196

3-[((((3,5-Dichlorphenyl)-4-oxy-n-butyl)sulfonyl)amino)carbonyl]-6-hydroxymethyl-pyridin

### Beispiel 197

3-[((((3-Trifluormethylphenyl)-4-oxy-n-butyl)sulfonyl)amino)carbonyl]-6-hydroxymethyl-pyridin

### Beispiel 198

3-[((((4-Trifluormethylphenyl)-4-oxy-n-butyl)sulfonyl)amino)carbonyl]-6-hydroxymethyl-pyridin

### Beispiel 199

3-[((((4-n-Butyloxyphenyl)-4-oxy-n-butyl)sulfonyl)amino)carbonyl]-6-hydroxymethyl-pyridin

### Beispiel 200

3-[((((2-Ethyloxyphenyl)-4-oxy-n-butyl)sulfonyl)amino)carbonyl]-6-hydroxymethyl-pyridin

### Beispiel 201

3-[(((4-Methoxy-n-butylsulfonyl)amino)carbonyl]-6-hydroxymethyl-pyridin

### Beispiel 202

3-[(((4-Ethyloxy-n-butyl)sulfonyl)amino)carbonyl]-6-hydroxymethyl-pyridin

### Beispiel 203

3-[(((4-Isopropyloxy-n-butyl)sulfonyl)amino)carbonyl]-6-hydroxymethyl-pyridin

### Beispiel 204

3-[(((4-n-Butyloxy-n-butyl)sulfonyl)amino)carbonyl]-6-hydroxymethyl-pyridin

### Beispiel 205

3-[(((4-Benzyloxy-n-butyl)sulfonyl)amino)carbonyl]-6-hydroxymethyl-pyridin

### Beispiel 206

3-[(((2-Phenylethyl)-4-oxy-n-butyl)sulfonyl)amino)carbonyl]-6-hydroxymethyl-pyridin

### Beispiel 207

3-[((2-Naphthylsulfonyl)amino)carbonyl]-6-hydroxymethyl-pyridin

### Beispiel 208

3-[(((4-Methoxy-2-naphthyl)sulfonyl)amino)carbonyl]-6-hydroxymethyl-pyridin

### Beispiel 209

3-[((((4-Fluorphenyl)-2-oxyethyl)sulfonyl)amino)carbonyl]-6-ethyloxymethyl-pyridin

### Beispiel 210

3-[((((3-Fluorphenyl)-2-oxyethyl)sulfonyl)amino)carbonyl]-6-ethyloxymethyl-pyridin

### Beispiel 211

3-[((((2,4-Difluorphenyl)-2-oxyethyl)sulfonyl)amino)carbonyl]-6-ethyloxymethyl-pyridin

### Beispiel 213

3-[((((4-Chlorphenyl)-2-oxyethyl)sulfonyl)amino)carbonyl]-6-ethyloxymethyl-pyridin

### Beispiel 214

3-[((((3,5-Dichlorphenyl)-2-oxyethyl)sulfonyl)amino)carbonyl]-6-ethyloxymethyl-pyridin

### Beispiel 215

3-[((((3-Trifluormethylphenyl)-2-oxyethyl)sulfonyl)amino)carbonyl]-6-ethyloxymethyl-pyridin

### Beispiel 216

3-[((((4-Trifluormethylphenyl)-2-oxyethyl)sulfonyl)amino)carbonyl]-6-ethyloxymethyl-pyridin

### Beispiel 217

3-[((((4-n-Butyloxyphenyl)-2-oxyethyl)sulfonyl)amino)carbonyl]-6-ethyloxymethyl-pyridin

### Beispiel 218

3-[((((2-Ethyloxyphenyl)-2-oxyethyl)sulfonyl)amino)carbonyl]-6-ethyloxymethyl-pyridin

### Beispiel 219

3-[(((2-Methoxyethylsulfonyl)amino)carbonyl]-6-ethyloxymethyl-pyridin

### Beispiel 220

3-[(((2-Ethyloxyethyl)sulfonyl)amino)carbonyl]-6-ethyloxymethyl-pyridin

### Beispiel 221

3-[(((2-Isopropyloxyethyl)sulfonyl)amino)carbonyl]-6-ethyloxymethyl-pyridin

### Beispiel 222

3-[(((2-n-Butyloxyethyl)sulfonyl)amino)carbonyl]-6-ethyloxymethyl-pyridin

### Beispiel 223

3-[(((2-Benzyloxyethyl)sulfonyl)amino)carbonyl]-6-ethyloxymethyl-pyridin

### Beispiel 224

3-[(((2-Phenylethyl)-2-oxyethyl)sulfonyl)amino)carbonyl]-6-ethyloxymethyl-pyridin

### Beispiel 225

3-[((1-Naphthylsulfonyl)amino)carbonyl]-6-ethyloxymethyl-pyridin

### Beispiel 226

3-[(((4-Methoxy-1-naphthyl)sulfonyl)amino)carbonyl]-6-ethyloxymethyl-pyridin

### Beispiel 227

3-[(((5-Ethoxy-1-naphthyl)sulfonyl)amino)carbonyl]-6-ethyloxymethyl-pyridin

### Beispiel 228

3-[((2-Biphenylsulfonyl)amino)carbonyl]-6-ethyloxymethyl-pyridin

### Beispiel 229

3-[(((4-Methoxy-2-biphenyl)sulfonyl)amino)carbonyl]-6-ethyloxymethyl-pyridin

### Beispiel 230

3-[((4-(4-Fluorphenoxy)phenylsulfonyl)amino)carbonyl)-6-ethyloxymethyl-pyridin

### Beispiel 231

3-[((4-(3-Fluorphenoxy)phenylsulfonyl)amino)carbonyl)-6-ethyloxymethyl-pyridin

### Beispiel 232

3-[((4-(4-Chlorphenoxy)phenylsulfonyl)amino)carbonyl)-6-ethyloxymethyl-pyridin

### Beispiel 233

3-[((4-(3,5-Dichlorphenoxy)phenylsulfonyl)amino)carbonyl)-6-ethyloxymethyl-pyridin

### Beispiel 234

3-[((((4-Fluorphenyl)-2-oxyethyl)sulfonyl)amino)carbonyl]-6-acetoxymethyl-pyridin

### Beispiel 235

3-[((((3-Fluorphenyl)-2-oxyethyl)sulfonyl)amino)carbonyl]-6-acetoxymethyl-pyridin

### Beispiel 236

3-[((((2,4-Difluorphenyl)-2-oxyethyl)sulfonyl)amino)carbonyl]-6-acetoxymethyl-pyridin

### Beispiel 237

3-[((((4-Chlorphenyl)-2-oxyethyl)sulfonyl)amino)carbonyl]-6-acetoxymethyl-pyridin

### Beispiel 238

3-[((((3,5-Dichlorphenyl)-2-oxyethyl)sulfonyl)amino)carbonyl]-6-acetoxymethyl-pyridin

### Beispiel 239

3-[((((3-Trifluormethylphenyl)-2-oxyethyl)sulfonyl)amino)carbonyl]-6-acetoxymethyl-pyridin

### Beispiel 240

3-[((((4-Trifluormethylphenyl)-2-oxyethyl)sulfonyl)amino)carbonyl]-6-acetoxymethyl-pyridin

### Beispiel 241

3-[((((4-n-Butyloxyphenyl)-2-oxyethyl)sulfonyl)amino)carbonyl]-6-acetoxymethyl-pyridin

### Beispiel 242

3-[((((2-Ethyloxyphenyl)-2-oxyethyl)sulfonyl)amino)carbonyl]-6-acetoxymethyl-pyridin

### Beispiel 243

3-[(((2-Methoxyethylsulfonyl)amino)carbonyl]-6-acetoxymethyl-pyridin

### Beispiel 244

3-[(((2-Ethyloxyethyl)sulfonyl)amino)carbonyl]-6-acetoxymethyl-pyridin

### Beispiel 245

3-[(((2-Isopropyloxyethyl)sulfonyl)amino)carbonyl]-6-acetoxymethyl-pyridin

### Beispiel 246

3-[(((2-n-Butyloxyethyl)sulfonyl)amino)carbonyl)-6-acetoxymethyl-pyridin

### Beispiel 247

3-[(((2-Benzyloxyethyl)sulfonyl)amino)carbonyl]-6-acetoxymethyl-pyridin

### Beispiel 248

3-[(((2-Phenylethyl)-2-oxyethyl)sulfonyl)amino)carbonyl]-6-acetoxymethyl-pyridin

### Beispiel 249

3-[((1-Naphthylsulfonyl)amino)carbonyl]-6-acetoxymethyl-pyridin

### Beispiel 250

3-[(((4-Methoxy-1-naphthyl)sulfonyl)amino)carbonyl]-6-acetoxymethyl-pyridin

### Beispiel 251

3-[(((5-Ethoxy-1-naphthyl)sulfonyl)amino)carbonyl]-6-acetoxymethyl-pyridin

### Beispiel 252

3-[((2-Biphenylsulfonyl)amino)carbonyl]-6-acetoxymethyl-pyridin

### Beispiel 253

3-[(((4-Methoxy-2-biphenyl)sulfonyl)amino)carbonyl]-6-acetoxymethyl-pyridin

### Beispiel 254

3-[((4-(4-Phenyl-n-butylaminocarbonyl)phenylsulfonyl)amino)carbonyl]-6-acetoxymethyl-pyridin

### Beispiel 255

3-[((4-(2-Phenylethylaminocarbonyl)phenylsulfonyl)amino)carbonyl]-6-acetoxymethyl-pyridin

### Beispiel 256

3-[((4-(3-Phenylpropylaminocarbonyl)phenylsulfonyl)amino)carbonyl]-6-acetoxymethyl-pyridin

### Beispiel 257

3-[((4-(2-Methoxyethyl)aminocarbonyl)phenylsulfonyl)amino)carbonyl]-6-acetoxymethyl-pyridin

### Beispiel 258

3-[((4-(2-Ethoxyethyl)aminocarbonyl)phenylsulfonyl)amino)carbonyl]-6-acetoxymethyl-pyridin

### Beispiel 259

3-[((4-(3-Ethoxypropyl)aminocarbonyl)phenylsulfonyl)amino)carbonyl]-6-acetoxymethyl-pyridin

### Beispiel 260

3-[((4-Ethylaminocarbonyl)phenylsulfonyl)amino)carbonyl]-6-benzyloxymethyl-pyridin

### Beispiel 261

3-[((4-(n-Propylaminocarbonyl)phenylsulfonyl)amino)carbonyl]-6-benzyloxymethyl-pyridin

### Beispiel 262

3-[((4-(n-Hexylaminocarbonyl)phenylsulfonyl)amino)carbonyl]-6-n-butanoyloxymethyl-pyridin

### Beispiel 263

3-[((4-(N,N-Di-n-butylaminocarbonyl)phenylsulfonyl)amino)carbonyl]-6-benzoyloxymethyl-pyridin

### Beispiel 264

3-[((4-(Cyclohexylaminocarbonyl)phenylsulfonyl)amino)carbonyl]-6-acetoxymethyl-pyridin

### Beispiel 265

3-[((4-(Benzylaminocarbonyl)phenylsulfonyl)amino)carbonyl]-6-acetoxymethyl-pyridin

### Beispiel 266

3-[(((4-(4-Phenylbutanoylamino)phenylsulfonyl)amino)carbonyl]-6-acetoxymethyl-pyridin

### Beispiel 267

3-[((4-[2-Acetylamino]ethyl)phenylsulfonyl)amino)carbonyl]-6-benzyloxymethyl-pyridin

### Beispiel 268

3-[((4-[2-Butanoylamino]ethyl)phenyl)sulfonyl)amino)carbonyl]-6-(2-methylpropionyl)oxymethyl-pyridin

### Beispiel 269

3-[((4-[2-Benzoylamino]ethyl)phenyl)sulfonyl)amino)carbonyl]-6-benzoyloxymethyl-pyridin

### Beispiel 270

3-[((4-[2-(2-Chlor-5-methoxy-benzoyl)amino]ethyl)phenylsulfonyl)amino)carbonyl]-6 n-butanoyloxymethyl-pyridin

### Beispiel 271

3-[((4-Acetylamino)phenylsulfonyl)amino)carbonyl]-6-ethyloxymethyl-pyridin

### Beispiel 272

3-[(((4-Propionylamino)phenylsulfonyl)amino)carbonyl]-6-ethyloxymethyl-pyridin

### Beispiel 273

3-[(((4-n-Hexanoylamino)phenylsulfonyl)amino)carbonyl]-6-ethyloxymethyl-pyridin

### Beispiel 274

3-[(((4-Benzoylamino)phenylsulfonyl)amino)carbonyl]-6-ethyloxymethyl-pyridin

### Beuspiel 275

3-[(((4-[3-Phenylpropionylamino]phenylsulfonyl)amino)carbonyl]-6-ethyloxymethyl-pyridin

### Beispiel 276

3-[(((4-(2-Phenylacetylamino)phenylsulfonyl)amino)carbonyl]-6-benzyloxymethyl-pyridin

### Beispiel 277

3-[((((4-Fluorphenyl)-2-oxyethyl)sulfonyl)amino)carbonyl]-6-benzoyloxymethyl-pyridin

### Beispiel 278

3-[((((3-Fluorphenyl)-2-oxyethyl)sulfonyl)amino)carbonyl]-6-(2-methylbenzoyl)oxymethyl-pyridin

### Beispiel 279

3-[((((2,4-Difluorphenyl)-2-oxyethyl)sulfonyl)amino)carbonyl]-6-(2-methylpropionyl)oxymethyl-pyridin

### Beispiel 280

3-[((((4-Chlorphenyl)-2-oxyethyl)sulfonyl)amino)carbonyl]-6-benzyloxymethyl-pyridin

### Beispiel 281

3-[((((3,5-Dichlorphenyl)-2-oxyethyl)sulfonyl)amino)carbonyl]-6-benzyloxymethyl-pyridin

### Beispiel 282

3-[((((3-Trifluormethylphenyl)-2-oxyethyl)sulfonyl)amino)carbonyl]-6-benzyloxymethyl-pyridin

### Beispiel 283

3-[((((4-Trifluormethylphenyl)-2-oxyethyl)sulfonyl)amino)carbonyl]-6-benzyloxymethyl-pyridin

### Beispiel 284

3-[((((4-n-Butyloxyphenyl)-2-oxyethyl)sulfonyl)amino)carbonyl]-6-(2-methylpropionyl)oxymethyl-pyridin

### Beispiel 285

3-[((((2-Ethyloxyphenyl)-2-oxyethyl)sulfonyl)amino)carbonyl]-6-propionyloxymethyl-pyridin

### Beispiel 286

3-[(((2-Methoxyethylsulfonyl)amino)carbonyl]-6-benzyloxymethyl-pyridin

### Beispiel 287

3-[(((2-Ethyloxyethyl)sulfonyl)amino)carbonyl]-6-benzyloxymethyl-pyridin

### Beispiel 288

3-[(((2-Isopropyloxyethyl)sulfonyl)amino)carbonyl]-6-benzoyloxymethyl-pyridin

### Beispiel 289

3-[(((2-n-Butyloxyethyl)sulfonyl)amino)carbonyl]-6-(2-methylpropionyl)oxymethyl-pyridin

### Beispiel 290

3-[(((2-Benzyloxyethyl)sulfonyl)amino)carbonyl]-6-n-butanoyloxymethyl-pyridin

### Beispiel 291

3-[(((2-Phenylethyl)-2-oxyethyl)sulfonyl)amino)carbonyl]-6-benzyloxymethyl-pyridin

### Beispiel 292

3-[((1-Naphthylsulfonyl)amino)carbonyl]-6-(2-methylpropionyl)oxymethyl-pyridin

### Beispiel 293

3-[(((4-Methoxy-1-naphthyl)sulfonyl)amino)carbonyl]-6-benzoyloxymethyl-pyridin

### Beispiel 294

3-[(((5-Ethoxy-1-naphthyl)sulfonyl)amino)carbonyl]-6-(2-methylpropionyl)oxymethyl-pyridin

### Beispiel 295

3-[((2-Biphenylsulfonyl)amino)carbonyl]-6-methoxymethyl-pyridin

### Beispiel 296

3-[(((4-Methoxy-2-biphenyl)sulfonyl)amino)carbonyl]-6-methoxymethyl-pyridin

### Beispiel 297

3-[((((4-n-Butyloxyphenyl)-3-oxypropyl)sulfonyl)amino)carbonyl]-6-ethyloxymethyl-pyridin

### Beispiel 298

3-[((((2-Ethyloxyphenyl)-3-oxypropyl)sulfonyl)amino)carbonyl]-6-ethyloxymethyl-pyridin

### Beispiel 299

3-[(((3-Methoxypropylsulfonyl)amino)carbonyl]-6-acetoxymethyl-pyridin

### Beispiel 300

3-[(((3-Ethyloxypropyl)sulfonyl)amino)carbonyl]-6-acetoxymethyl-pyridin

### Beispiel 301

3-[(((3-Isopropyloxypropyl)sulfonyl)amino)carbonyl]-6-ethyloxymethyl-pyridin

### Beispiel 302

3-[(((3-n-Butyloxypropyl)sulfonyl)amino)carbonyl]-6-(2-methylpropionyl)oxymethyl-pyridin

### Beispiel 303

3-[(((3-Benzyloxypropyl)sulfonyl)amino)carbonyl]-6-acetoxymethyl-pyridin

### Beispiel 304

3-[((1-Naphthylsulfonyl)amino)carbonyl]-6-methoxymethyl-pyridin

### Beispiel 305

3-[(((4-Methoxy-1-naphthyl)sulfonyl)amino)carbonyl]-6-methoxymethyl-pyridin

### Beispiel 306

3-[(((5-Ethoxy-1-naphthyl)sulfonyl)amino)carbonyl]-6-methoxymethyl-pyridin

### Beispiel 307

3-[((2-Biphenylsulfonyl)amino)carbonyl]-6-(2-methylpropionyl)oxymethyl-pyridin

### Beispiel 308

3-[(((4-Methoxy-2-biphenyl)sulfonyl)amino)carbonyl]-6-benzyloxymethyl-pyridin

Die folgenden Beispiele 309 - 318 wurden aus 6-Benzyloxymethylpyridin-3-carbonsäure (vgl. Beispiel 21a)) bzw. 6-Ethyoxymethylpyridin-3-carbonsäure (vgl. Beispiel 90a)) und den entsprechenden Sulfonamid-Derivaten nach der in den Beispielen 21b) und 90b) beschriebenen Methode hergestellt.

### Beispiel 309

3-[((4-((n-Butylamino)carbonyl)phenylsulfonyl)amino)carbonyl]-6-benzyloxymethyl-pyridin
Fp. 189 - 191°C (aus Methanol)

### Beispiel 310

3-[((4-(2-((2-Methylpropionylamino)ethyl)phenylsulfonyl)amino)carbonyl]-6-benzyloxymethyl-pyridin
Fp. 156 - 158°C (nach Chromatogrphie mit Ethylacetat/n-Heptan/Methanol = 5:3:2 an Kieselgel)

### Beispiel 311

3-[((4-(2-((4-Methylpentanoyl)amino)ethyl)phenylsulfonyl)amino)carbonyl]-6-benyloxymethyl-pyridin
Fp. 109 - 111°C (nach Chromatographie mit Ethylacetat/n-Heptan/Methanol = 5:3:2 an Kieselgel)

### Beispiel 312

3-[((4-((2-(Phenylethyl)amino)carbonyl)phenylsulfonyl)amino)carbonyl]-6-benzyloxymethyl-pyridin
Fp. 175 - 176°C (aus Methanol)

### Beispiel 313

3-{((4-((2-Phenylethyl)amino)carbonyl)phenylsulfonyl)amino)carbonyl]-6-ehtyloxymethyl-pyridin
Fp. 190 - 191°C (aus Methanol)

### Beispiel 314

3-[((4-(2-((Cyclohexylacetyl)amino)ethyl)phenylsulfonyl)amino)carbonyl]-6-ethyloxymethyl-pyridin
Fp. 201 - 202°C (aus Methanol/Diisopropylether)

### Beispiel 315

3-[((4-(2-(Hexanoylamino)ethyl)phenylsulfonyl)amino)carbonyl]-6-ethyloxymethyl-pyridin
Fp. 175 - 177°C (aus wäßriger Salzsäure)

### Beispiel 316

3-[((4-(2-((4-Methylpentanoyl)amino)ethyl)phenylsulfonyl)amino)carbonyl]-6-ethyloxymethyl-pyridin
Fp. 156 - 158°C (aus wäßriger Salzsäure)

### Beispiel 317

3-[((4-((n-Butylamino)carbonyl)phenylsulfonyl)amino)carbonyl]-6-ethyloxymethyl-pyridin
Fp. 206 - 208°C (aus wäßriger Salzsäure)

### Beispiel 318

3-[((3-((n-Butylamino)carbonyl)phenylsulfonyl)amino)carbonyl]-6-ethyloxymethyl-pyridin
Fp. 214 - 216°C (nach Chromatographie mit Ethylacetat/n-Heptan/Methanol = 5:3:2 an Kieselgel)

### Beispiel 319

3-[((2-Chlor-5-(((2-(4-fluorphenyl)ethyl)amino)carbonyl)phenylsulfonyl)amino)-carbonyl]-6-hydroxymethyl-pyridin-ammonium-salz
hell bräunliche amorphe Substanz nach Chromatographie mit Dichlormethan/Methanol/wäßr. Ammoniak (10:4:0,25);
Herstellung in Analogie zu Beispiel 125.

### Beispiel 320

3-[((4-((2-(4-Methoxyphenyl)ethyl)aminocarbonyl)phenylsulfonyl)amino)carbonyl]-6-hydroxymethyl-pyridin

### Beispiel 321

3-[((4-((2-(3,4-Dimethoxyphenyl)ethyl)aminocarbonyl)phenylsulfonyl)amino)carbonyl] -6-hydroxymethyl-pyridin

### Beispiel 322

3-[((4-((2-(2-Methoxyphenyl)ethyl)aminocarbonyl)phenylsulfonyl)amino)carbonyl]-6-hydroxymethyl-pyridin

### Beispiel 323

3-[((4-((2-(4-Fluorphenyl)ethyl)aminocarbonyl)phenylsulfonyl)amino)carbonyl]-6-hydroxymethyl-pyridin

### Beispiel 324

3-[((4-((2-(4-Chlorphenyl)ethyl)aminocarbonyl)phenylsulfonyl)amino)carbonyl]-6-hydroxymethyl-pyridin

### Beispiel 325

3-[((4-(2-((5-Chlor-2-methoxybenzoyl)amino)ethyl)phenylsulfonyl)amino)carbonyl]-6-hydroxymethyl-pyridin

### Beispiel 326

3-[((4-(2-((3,4-Dimethoxyphenyl-propionyl)amino)ethyl)phenylsulfonyl)amino)carbonyl]-6-hydroxymethyl-pyridin

### Beispiel 327

3-[((4-(2-((2-Phenylacetyl)amino)ethyl)phenylsulfonyl)amino)carbonyl]-6-hydroxymethyl-pyridin

### Beispiel 328

3-[((4-(2-((Phenoxyacetyl)amino)ethyl)phenylsulfonyl)amino)carbonyl]-6-hydroxymethyl-pyridin

### Beispiel 329

3-[((4-(2-((4-Fluorbenzoyl)amino)ethyl)phenylsulfonyl)amino)carbonyl]-6-hydroxymethyl-pyridin

### Beispiel 330

3-[((4-(2-((4-Methylpentanoyl)amino)ethyl)phenylsulfonyl)amino)carbonyl]-6-hydroxymethyl-pyridin

### Beispiel 331

3-[((4-(2-((Cyclohexanoyl)amino)ethyl)phenylsulfonyl)amino)carbonyl]-6-hydroxymethyl-pyridin

### Beispiel 332

3-[((4-(2-((Cyclohexylacetyl)amino)ethyl)phenylsulfonyl)amino)carbonyl]-6-hydroxymethyl-pyridin-ammoniumsalz
Aus 2,46 g (14,4 mmol) 6-Chlormethylpyridin-3-carbonsäure wurden in Analogie zu Beispiel 125 1,73 g der Titelverbindung erhalten.
Fp. 140 - 142°C; nach Chromatographie mit Dichlormethan/Methanol/wäßr. Ammoniak (10:4:0.2) an Kieselgel.

### Beispiel 333

3-[((4-(2-((2-Methylpropionyl)amino)ethyl)phenylsulfonyl)amino)carbonyl]-6 hydroxymethyl-pyridin-ammoniumsalz
Aus 2,46 g (14,4 mmol) 6-Chlormethylpyridin-3-carbonsäure wurden in Analogie zu Beispiel 125 2,48 g der Titelverbindung erhalten.
Fp. 178 - 180°C; nach Chromatographie analog zu Beispiel 332.

### Beispiel 334

3-[((4-(2-((3,4-Dimethoxybenzoyl)amino)ethyl)phenylsulfonyl)amino)carbonyl]-6-hydroxymethyl-pyridin

### Beispiel 335

3-[((4-(2-((3,4-Diethoxybenzoyl)amino)ethyl)phenylsulfonyl)amino)carbonyl]-6-hydroxymethyl-pyridin

### Beispiel 336

3-[((4-(2-((4-n-Butyloxybenzoyl)amino)ethyl)phenylsulfonyl)amino)carbonyl]-6-hydroxymethyl-pyridin

### Beispiel 337

3-[((4-((5-Chlor-2-methoxybenzoyl)amino)methyl)phenylsulfonyl)amino)carbonyl]-6-hydroxymethyl-pyridin

### Beispiel 338

3-[((4-((3,4-Dimethoxyphenyl-propionyl)amino)methyl)phenylsulfonyl)amino)carbonyl]-6-hydroxymethyl-pyridin

### Beispiel 339

3-[((4-((2-Phenylacetyl)amino)methyl)phenylsulfonyl)amino)carbonyl]-6-hydroxymethyl-pyridin

### Beispiel 340

3-[((4-((Phenoxyacetyl)amino)methyl)phenylsulfonyl)carbonyl]-6-hydroxymethyl-pyridin

### Beispiel 341

3-[((4-((4-Fluorbenzoyl)amino)methyl)phenylsulfonyl)amino)carbonyl]-6-hydroxymethyl-pyridin

### Beispiel 342

3-[((4-((4-Methylpentanoyl)amino)methyl)phenylsulfonyl)amino)carbonyl]-6-hydroxymethyl-pyridin

### Beispiel 343

3-[((4-((Cyclohexanoyl)amino)methyl)phenylsulfonyl)amino)carbonyl]-6-hydroxymethyl-pyridin

### Beispiel 344

3-[((4-((Cyclohexylacetyl)amino)methyl)phenylsulfonyl)amino)carbonyl]-6-hydroxymethyl-pyridin

### Beispiel 345

3-[((4-((2-Methylpropionyl)amino)methyl)phenylsulfonyl)amino)carbonyl]-6-hydroxymethyl-pyridin

### Beispiel 346

3-[((4-((3,4-Dimethoxybenzoyl)amino)methyl)phenylsulfonyl)amino)carbonyl]-6 hydroxymethyl-pyridin

### Beispiel 347

3-[((4-((3,4-Diethoxybenzoyl)amino)methyl)phenylsulfonyl)amino)carbonyl]-6-hydroxymethyl-pyridin

### Beispiel 348

3-[((4-((4-n-Butyloxybenzoyl)amino)methyl)phenylsulfonyl)amino)carbonyl]-6-hydroxymethyl-pyridin

### Beispiel 349

3-[((4-((2-Phenylethyl)aminocarbonyl)phenylsulfonyl)amino)carbonyl]-6-caproyloxymethyl-pyridin

### Beispiel 350

3-[((4-((2-(4-Methoxyphenyl)ethyl)aminocarbonyl)phenylsulfonyl)amino)carbonyl]-6-caproyloxymethyl-pyridin

### Beispiel 351

3-[((4-((2-(3,4-Dimethoxyphenyl)ethyl)aminocarbonyl)phenylsulfonyl)amino)carbonyl] -6-caproyloxymethyl-pyridin

### Beispiel 352

3-[((4-((2-(2-Methoxyphenyl)ethyl)aminocarbonyl)phenylsulfonyl)amino)carbonyl]-6 caproyloxymethyl-pyridin

### Beispiel 353

3-[((4-((2-(4-Fluorphenyl)ethyl)aminocarbonyl)phenylsulfonyl)amino)carbonyl]-6-caproyloxymethyl-pyridin

### Beispiel 354

3-[((4-((2-(4-Chlorphenyl)ethyl)aminocarbonyl)phenylsulfonyl)amino)carbonyl]-6-caproyloxymethyl-pyridin

### Beispiel 355

3-[((4-((2-(4-Methoxyphenyl)ethyl)aminocarbonyl)phenylsulfonyl)amino)carbonyl]-6-lauroyloxymethyl-pyridin

### Beispiel 356

3-[((4-((2-(3,4-Dimethoxyphenyl-propionyl)amino)ethyl)phenylsulfonyl)amino)carbonyl]-6-lauroyloxymethyl-pyridin

### Beispiel 357

3-[((4-((2-(2-Methoxyphenyl)ethyl)aminocarbonyl)phenylsulfonyl)amino)carbonyl]-6-lauroyloxymethyl-pyridin

### Beispiel 358

3-[((4-((2-(4-Fluorphenyl)ethyl)aminocarbonyl)phenylsulfonyl)amino)carbonyl]-6-lauroyloxymethyl-pyridin

### Beispiel 359

3-[((4-((2-(4-Chlorphenyl)ethyl)aminocarbonyl)phenylsulfonyl)amino)carbonyl]-6-lauroyloxymethyl-pyridin

### Beispiel 360

3-[((4-((2-(Cyclohexylacetyl)amino)ethyl)phenylsulfonyl)amino)carbonyl]-6-lauroyloxymethyl-pyridin

### Beispiel 361

3-[((4-(2-((2-Methylpropionyl)amino)ethyl)phenylsulfonyl)amino)carbonyl]-6-lauroyloxymethyl-pyridin

### Beispiel 362

3-[((4-(2-((3,4-Dimethoxybenzoyl)amino)ethyl)phenylsulfonyl)amino)carbonyl]-6-lauroyloxymethyl-pyridin

### Beispiel 363

3-[((4-(2-((3,4-Diethoxybenzoyl)amino)ethyl)phenylsulfonyl)amino)carbonyl]-6-lauroyloxymethyl-pyridin

### Beispiel 364

3-[((4-(2-((4-n-Butyloxybenzoyl)amino)ethyl)phenylsulfonyl)amino)carbonyl]-6-lauroyloxymethyl-pyridin

### Beispiel 365

3-[((4-((2-(4-Methoxyphenyl)ethyl)aminocarbonyl)phenylsulfonyl)amino)carbonyl]-6-palimitoyloxymethyl-pyridin

### Beispiel 366

3-[((4-((2-(3,4-Dimethoxyphenyl)ethyl)aminocarbonyl)phenylsulfonyl)amino)carbonyl]-6-palimitoyloxymethyl-pyridin

### Beispiel 367

3-[((4-((2-(2-Methoxyphenyl)ethyl)aminocarbonyl)phenylsulfonyl)amino)carbonyl]-6-palimitoyloxymethyl-pyridin

### Beispiel 368

3-[((4-((2-(4-Fluorphenyl)ethyl)aminocarbonyl)phenylsulfonyl)amino)carbonyl]-6-palimitoyloxymethyl-pyridin

### Beispiel 369

3-[((4-((2-(4-Chlorphenyl)ethyl)aminocarbonyl)phenylsulfonyl)amino)carbonyl]-6-palmitoyloxymethyl-pyridin

### Beispiel 370

3-[((4-(2-((Cyclohexylacetyl)amino)ethyl)phenylsulfonyl)amino)carbonyl-6-palimitoyloxymethyl-pyridin

### Beispiel 371

3-[((4-(2-((2-Methylpropionyl)amino)ethyl)phenylsulfonyl)amino)carbonyl]-6-palimitoyloxymethyl-pyridin

### Beispiel 372

3-[((4-(2-((3,4-Dimethoxybenzoyl)amino)ethyl)phenylsulfonyl)amino)carbonyl]-6-palimitoyloxymethyl-pyridin

### Beispiel 373

3-[((4-(2-((3,4-Diethoxybenzoyl)amino)ethyl)phenylsulfonyl)amino)carbonyl]-6-palimitoyloxymethyl-pyridin

### Beispiel 374

3-[((4-(2-((4-n-Butyloxybenzoyl)amino)ethyl)phenylsulfonyl)amino)carbonyl]-6-palimitoyloxymethyl-pyridin

### Beispiel 375

3-[((4-((2-(4-Methoxyphenyl)ethyl)aminocarbonyl)phenylsulfonyl)amino)carbonyl]-6-stearoyloxymethyl-pyridin

### Beispiel 376

3-[((4-((2-(3,4-Dimethoxyphenyl)ethyl)aminocarbonyl)phenylsulfonyl)amino)carbonyl]-6-stearoyloxymethyl-pyridin

### Beispiel 377

3-[((4-((2-Phenylethyl)aminocarbonyl)phenylsulfonyl)amino)carbonyl]-6-stearoyloxymethyl-pyridin

### Beispiel 378

3-[((4-((2-(4-Fluorphenyl)ethyl)aminocarbonyl)phenylsulfonyl)amino)carbonyl]-6-stearoyloxymethyl-pyridin

### Beispiel 379

3-[((4-((2-(4-Chlorphenyl)ethyl)aminocarbonyl)phenylsulfonyl)amino)carbonyl]-6-stearoyloxymethyl-pyridin

### Beispiel 380

3-[((4-(2-((Cyclohexylacetyl)amino)ethyl)phenylsulfonyl)amino)carbonyl-6-stearoyloxymethyl-pyridin

### Beispiel 381

3-[((4-(2-((2-Methylpropionyl)amino)ethyl)phenylsulfonyl)amino)carbonyl]-6-stearoyloxymethyl-pyridin

### Beispiel 382

3-[((4-(2-((3,4-Dimethoxybenzoyl)amino)ethyl)phenylsulfonyl)amino)carbonyl]-6-stearoyloxymethyl-pyridin

### Beispiel 383

3-[((4-(2-((3,4-Diethoxybenzoyl)amino)ethyl)phenylsulfonyl)amino)carbonyl]-6-stearoyloxymethyl-pyridin

### Beispiel 384

3-[((4-(2-((4-n-Butyloxybenzoyl)amino)ethyl)phenylsulfonyl)amino)carbonyl]-6-stearoyloxymethyl-pyridin

### Beispiel 385

3-[((4-(2-((2-Chlor-5-methoxybenzoyl)amino)methyl)phenylsulfonyl)amino)carbonyl]-6-linoyloxymethyl-pyridin

### Beispiel 386

3-[((4-(2-((3,4-Dimethoxyphenylpropionyl)amino)ethyl)phenylsulfonyl)amino)carbonyl]-6-linoyloxymethyl-pyridin

### Beispiel 387

3-[((4-(2-((2-Phenylacetyl)amino)ethyl)phenylsulfonyl)amino)carbonyl]-6-linoyloxymethyl-pyridin

### Beispiel 388

3-[((4-(2-((Phenoxyacetyl)amino)ethyl)phenylsulfonyl)amino)carbonyl]-6-linoyloxymethyl-pyridin

### Beispiel 389

3-[((4-(2-((4-Fluorbenzoyl)amino)ethyl)phenylsulfonyl)amino)carbonyl]-6-linoyloxymethyl-pyridin

### Beispiel 390

3-[((4-((2-Phenylethyl)aminocarbonyl)phenylsulfonyl)amino)carbonyl)]-6-(2-ethylbutyryl)oxymethyl-pyridin

### Beispiel 391

3-[((4-((2-(4-Methoxyphenyl)ethyl)aminocarbonyl)phenylsulfonyl)amino)carbonyl]-6-(2-ethylbutyryl)oxymethyl-pyridin

### Beispiel 392

3-[((4-((2-(3,4-Dimethoxyphenyl)ethyl)aminocarbonyl)phenylsulfonyl)amino)carbonyl] -6-(2-ethylbutyryl)oxymethyl-pyridin

### Beispiel 393

3-[((4-((2-(2-Methoxyphenyl)ethyl)aminocarbonyl)phenylsulfonyl)amino)carbonyl]-6-(2-ethylbutyryl)oxymethyl-pyridin

### Beispiel 394

3-[((4-((2-(4-Fluorphenyl)ethyl)aminocarbonyl)phenylsulfonyl)amino)carbonyl]-6-(2-ethylbutyryl)oxymethyl-pyridin

### Beispiel 395

3-[((4-((2-(4-Chlorphenyl)ethyl)aminocarbonyl)phenylsulfonyl)amino)carbonyl]-6-(2-ethylbutyryl)oxymethyl-pyridin

### Beispiel 396

3-[((4-(2-((Cyclohexylacetyl)amino)ethyl)phenylsulfonyl)amino)carbonyl]-6-(2-methylbenzoyl)oxymethyl-pyridin

### Beispiel 397

3-[((4-(2-((2-Methylpropionyl)amino)ethyl)phenylsulfonyl)amino)carbonyl]-6-(2-methylbenzoyl)oxymethyl-pyridin

### Beispiel 398

3-[((4-(2-((3,4-Dimethoxybenzoyl)amino)ethyl)phenylsulfonyl)amino)carbonyl]-6-(2-methylbenzoyl)oxymethyl-pyridin

### Beispiel 399

3-[((4-(2-((3,4-Diethoxybenzoyl)amino)ethyl)phenylsulfonyl)amino)carbonyl]-6-(2-methylbenzoyl)oxymethyl-pyridin

### Beispiel 400

3-[((4-(2-((4-n-Butyloxybenzoyl)amino)ethyl)phenylsulfonyl)amino)carbonyl]-6-(2-methylbenzoyl)oxymethyl-pyridin

### Beispiel 401

3-[((4-((n-Butylamino)carbonyl)phenylsulfonyl)-N-acetylamino)carbonyl]-6-ethyloxymethylpyridin, hergestellt durch Umsetzung von Beispiel 317 mit Natriumhydrid in Tetrahydrofuran und Acetylchlorid.

### Beispiel 402

3-[((4-(2-((2-Chlor-5-methoxybenzoyl)amino)ethyl)phenylsulfonyl)-N-acetylamino)carbonyl]-6-acetoxymethyl-pyridin
Fp. 235°C (u. Zers.) aus Diethylether

### Beispiel 403

3-[((4-(2-((Cyclohexylacetyl)amino)ethyl)phenylsulfonyl)-N-lauroylamino)carbonyl]-6-hydroxymethyl-pyridin

### Beispiel 404

3-[((4-(2-((Cyclohexylacetyl)amino)ethyl)phenylsulfonyl)-N-caproylamino)carbonyl]-6-caproyloxymethyl-pyridin

### Beispiel 405

3-[((4-(2-((2-Chlor-5-methoxybenzoyl)amino)ethyl)phenylsulfonyl)-N-stearoylamino)carbonyl]-6-hydroxymethyl-pyridin

### Beispiel 406

3-[((4-((2-Phenylethyl)aminocarbonyl)phenylsulfonyl)-N-lauroylamino)carbonyl]-6-hydroxymethyl-pyridin

### Beispiel 407

3-[((4-((2-Phenylethyl)aminocarbonyl)phenylsulfonyl)-N-palmitoylamino)carbonyl]-6-hydroxymethyl-pyridin

### Beispiel 408

3-[((4-((2-Phenylethyl)aminocarbonyl)phenylsulfonyl)-N-lauroylamino)carbonyl]-6-acetoxymethyl-pyridin

### Beispiel 409

3-[((4-((2-Phenylethyl)aminocarbonyl)phenylsulfonyl)-N-stearoylamino)carbonyl]-6-hydroxymethyl-pyridin

## Patentansprüche

1. 4- oder 5-(Sulf)imido- und (Sulfon)amidopyridine und verwandte Verbindungen sowie ihre Pyridin-N-oxide der allgemeinen Formel I 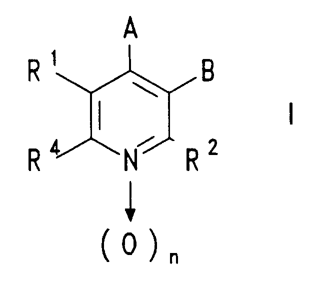 worin
A = R³ und B = -XNR⁶R⁷ oder
B = R³ und A = -XNR⁶R⁷ und
X eine Einfachbindung oder -CO-,
bedeutet und
R¹, R² und R³ gleich oder verschieden sind und Wasserstoff, unsubstituiertes oder substituiertes (C₁-C₆)-Alkyl, (C₁-C₆)-Alkoxy, Halogen, insbesondere Fluor, Chlor oder Brom, Nitril, Hydroxy, Amino,
R⁶ Wasserstoff, (C₁-C₂₀)-Alkyl oder eine N-Schutzgruppe wie Acyl, (C₁-C₂₀)-Alkanoyl, (C₁-C₂₀)-Alkenoyl, (C₁-C₂₀)-Alkinoyl, (C₇-C₂₀)-Aralkanoyl, (C₆-C₁₈)-Aroyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein physiologisch verwendbares Kation, wie Na⊕, K⊕, Mg²⊕, Ca²⊕, Al³⊕ oder ein Ammoniumion, ggf. 1-3 fach substituiert mit (C₁-C₁₆)Alkyl, (C₁-C₁₆)-Hydroxyalkyl, (C₁-C₈)-Alkoxy-(C₁-C₁₆)-alkyl, Phenyl, Benzyl oder (C₁-C₁₆)-Alkyl, welches 1 bis 3-fach substituiert sein kann mit Hydroxy oder (C₁-C₈)-Alkoxy, oder ein Kation eines basischen Aminosäurederivates, wobei ein vorhandener Arylteil gegebenenfalls substituiert sein kann,
R⁷ einen Rest Y R⁸ bedeutet,
in welchem
Y -SO₂-, -CO-, -CONR⁹ oder -SO₂NR⁹
und
R⁸ und R⁹ [C-U]ᵣ-D-W bedeuten, worin
C eine Bindung oder
einen verzweigten oder unverzweigten aliphatischen (C₁-C₁₆)-Alkandiyl oder cycloaliphatischen (C₃-C₁₀)-Alkandiylrest oder
einen verzweigten oder unverzweigten (C₂-C₁₆)-Alkendiyl- oder
Cycloalkendiyl-Rest, oder einen (C₂-C₁₆)-Alkindiyl-Rest oder einen (C₂-C₁₆)-Alkenindiyl-Rest bedeutet, der jeweils eine oder mehrere C-C-Mehrfachbindungen enthalten kann,
U eine Bindung oder
Wasserstoff oder
einen Rest aus der Reihe folgender Heteroatomgruppierungen -CO-, -O(CO)-, -(CO)-O-, -(CO)NR-, -NR(CO)-, -O-, -SO-, -SO₂-, -NR, worin R (C₁-C₃)Alkyl, (C₁-C₈)-Alkanoyl, (C₇-C₁₆)-Aralkanoyl, (C₆-C₁₂)-Aroyl oder Wasserstoff bedeutet,
r 1, 2, 3 oder 4 ist,
D eine Bindung oder Wasserstoff oder
einen verzweigten oder unverzweigten aliphatischen (C₁-C₁₀)-Alkandiyl-Rest, oder
einen verzweigten oder unverzweigten (C₁-C₁₀)-Alkendiyl-Rest, einen (C₂-C₁₀)-Alkindiyl-Rest oder einen (C₂-C₁₀)-Alkenindiyl-Rest, der jeweils eine oder mehrere C-C-Mehrfachbindungen enthalten kann,
W Wasserstoff oder
einen (C₃-C₁₀) cycloaliphatischen Alkyl-, Alkenyl-, Alkinyl- oder Alkeninyl-Rest oder
einen (C₆-C₁₆)-Arylrest oder einen 5- oder 6-gliedrigen Heteroarylrest, wobei mindestens eine der Variablen C oder D oder W nicht eine Bindung bedeuten und U nur dann in der Bedeutung einer Heteratomgruppierung steht, wenn C nicht eine Bindung bedeutet und
C, D und/oder W, sofern diese keine Bindung oder Wasserstoff bedeuten und vorzugsweise ihrerseits substituiert sind
oder
R⁸ und R⁹ auch über 3-6 C-Atome verknüpft sind, wobei -NR⁸R⁹ einen heterocyclischen Ring bilden, mit N als Heteroatom,
oder
R⁹ in der Bedeutung von R⁶ steht,
und
für [C-U]ᵣ-D-W die Bedeutung -SO₂H ausgenommen ist, sowie
R⁴ einen Rest bedeutet, der physiologisch, insbesondere in der Leber, in eine Carboxylatgruppe oder deren Salze umgewandelt werden kann, wobei Ester und Amide ausgenommen sind, und
und
n 0 oder 1,
f 1 bis 8, vorzugsweise 1 bis 5,
g 0, 1 bis (2f+ 1) und
x 0 bis 8, vorzugsweise 0 oder 1 bedeuten.

2. Verbindungen der Formel I, nach Anspruch 1 in denen
X eine Einfachbindung oder -CO-,
R¹, R² und R³ gleich oder verschieden sind und Wasserstoff, (C₁-C₆)-Alkyl, (C₁-C₆)-Alkoxy, Halogen, insbesondere Fluor, Chlor oder Brom, Nitril, Hydroxy, Amino, ggf. mono oder disubstituiert mit (C₁-C₄)-Alkyl, Hydroxy-(C₁-C₄)-alkyl, (C₁-C₆)-Alkylcarbonyloxy,
R⁶ Wasserstoff, (C₁-C₂₀)-Alkyl oder eine N-Schutzgruppe wie Acyl, (C₁-C₂₀)-Alkanoyl, (C₁-C₂₀)-Alkenoyl, (C₁-C₂₀)-Alkinoyl, (C₇-C₂₀)-Aralkanoyl, (C₆-C₁₈)-Aroyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein physiologisch verwendbares Kation, wie Na⊕, K⊕, Mg²⊕, Ca²⊕, Al³⊕ oder ein Ammoniumion, ggf. 1-3fach substituiert mit (C₁-C₁₆)-Alkyl, (C₁-C₁₆)-Hydroxyalkyl, (C₁-C₈)-Alkoxy-(C₁-C₁₆)-alkyl, Phenyl, Benzyl oder (C₁-C₁₆)-Alkyl, welches 1 bis 3-fach substituiert sein kann mit Hydroxy oder (C₁-C₈)-Alkoxy, oder ein Kation eines basischen Aminosäurederivates, wobei ein vorhandener Arylteil gegebenenfalls substituiert sein kann,
R⁷ einen Rest Y R⁸ bedeutet,
in welchem
Y -SO₂-, -CO-, -CONR⁹ oder -SO₂NR⁹
und
R⁸ und R⁹ [C-U]ᵣ-D-W bedeuten, worin
C eine Bindung oder
einen verzweigten oder unverzweigten aliphatischen (C₁-C₁₆)-Alkandiyl oder cycloaliphatischen (C₃-C₁₀)-Alkandiylrest oder
einen verzweigten oder unverzweigten (C₂-C₁₆)-Alkendiyl- oder Cycloalkendiyl-Rest, oder einen (C₂-C₁₆)-Alkindiyl-Rest oder einen (C₂-C₁₆)-Alkenindiyl-Rest bedeutet, der jeweils eine oder mehrere C-C-Mehrfachbindungen enthalten kann,
U eine Bindung oder
Wasserstoff oder
einen Rest aus der Reihe folgender Heteroatomgruppierungen -CO-, -O(CO)-, -(CO)-O-, -(CO)NR-, -NR(CO)-, -O-, -SO-, -SO₂-, -NR, worin R (C₁-C₃)Alkyl, (C₁-C₈)-Alkanoyl, (C₇-C₁₆)-Aralkanoyl, (C₆-C₁₂)-Aroyl oder Wasserstoff bedeutet,
r 1, 2, 3 oder 4 ist,
D eine Bindung oder Wasserstoff oder
einen verzweigten oder unverzweigten aliphatischen (C₁-C₁₀)-Alkandiyl-Rest, oder
einen verzweigten oder unverzweigten (C₁-C₁₀)-Alkendiyl-Rest, einen (C₂-C₁₀)-Alkindiyl-Rest oder einen (C₂-C₁₀)-Alkenindiyl-Rest, der jeweils eine oder mehrere C-C-Mehrfachbindungen enthalten kann,
W Wasserstoff oder einen (C₃-C₁₀) cycloaliphatischen Alkyl-, Alkenyl-, Alkinyl- oder Alkeninyl-Rest oder
einen (C₆-C₁₆)-Arylrest oder einen 5- oder 6-gliedrigen Heteroarylrest, wobei mindestens eine der Variablen C oder D oder W nicht eine Bindung bedeuten und U nur dann in der Bedeutung einer Heteratomgruppierung steht, wenn C nicht eine Bindung bedeutet und
C, D und/oder W, sofern diese keine Bindung oder Wasserstoff bedeuten, vorzugsweise ihrerseits substituiert sind durch eine Kombination von bis zu 5 gleichen oder verschiedenen Substituenten aus der Reihe Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, - OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂) Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂)-Alkinyloxycarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)alkyl)carbamoyl,
N-((C₆-C₁₆)-Aryloxy-(C₁-C₁₀)alkyl)carbamoyl,
N-((C₇-C₁₆)-Aralkyloxy-(C₁-C₁₀)alkyl)carbamoyl,
N-(C₁-C₁₀)-Alkyl-N-((C₁-C₁₀)-Alkoxy-(C₁-C₁₀)alkyl)carbamoyl,
N-(C₁-C₁₀)-Alkyl-N-((C₆-C₁₆)-Aryloxy-(C₁-C₁₀)alkyl)carbamoyl,
N-(C₁-C₁₀)-Alkyl-N-((C₇-C₁₆)-Aralkyloxy-(C₁-C₁₀)alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)arylcarbamoyloxy, N-(C₁-C₁₀)-Alkyl-N-(C₇-C₁₆)-Aralkylcarbamoyloxy
N-((C₁-C₁₀)-Alkoxy-(C₁-C₁₀)alkyl)carbamoyloxy,
N-((C₆-C₁₆)-Aryloxy-(C₁-C₁₀)alkyl)carbamoyloxy,
N-((C₇-C₁₆)-Aralkyloxy-(C₁-C₁₀)alkyl)carbamoyloxy,
N-(C₁-C₁₀)-Alkyl-N-((C₁-C₁₀)-Alkoxy-(C₁-C₁₀)alkyl)carbamoyloxy,
N-(C₁-C₁₀)-Alkyl-N-((C₆-C₁₆)-Aryloxy-(C₁-C₁₀)alkyl)carbamoyloxy,
N-(C₁-C₁₀)-Alkyl-N-((C₇-C₁₆)-Aralkyloxy-(C₁-C₁₀)alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-(C₁-C₁₀)Alkyl-(C₇-C₁₀)Aralkylamino, N-(C₁-C₁₀)Alkyl-N(C₆-C₁₂)Arylamino, (C₁-C₁₂)-Alkoxy-amino, (C₁-C₁₂)-Alkoxy-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)alkylamino-(C₁-C₁₀)alkyl, N,N-Di(C₁-C₁₀)alkylamino-(C₁-C₁₀)alkyl, (C₃-C₈)cycloalkylamino-(C₁-C₁₀)alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈)-cycloalkylsulfamoyl,
N-(C₆-C₁₆)-Arylsulfamoyl,
N-(C₇-C₁₆)-Aralkylsulfamoyl,
N-(C₁-C₁₀)-Alkyl-N-(C₆-C₁₆)-Arylsulfamoyl,
N-(C₁-C₁₀)-Alkyl-N-(C₇-C₁₆)-Aralkylsulfamoyl,
(C₁-C₁₀)-Alkyl-sulfonamido,
N-((C₁-C₁₀)alkyl)-(C₁-C₁₀)alkylsulfonamido, (C₇-C₁₆)-Aralkylsulfonamido,
N-((C₁-C₁₀)alkyl-(C₇-C₁₆)-Aralkylsulfonamido,
wobei die Reste, die einen Arylrest enthalten, ihrerseits am Aryl substituiert sein können mit 1, 2, 3, 4 oder 5 gleichen oder verschiedenen Substituenten aus der Reihe, Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl, -0-[CH₂₋]_{X}C_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂) Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂)-Alkinyloxycarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)alkyl)carbamoyl,
N-((C₆-C₁₆)-Aryloxy-(C₁-C₁₀)alkyl)carbamoyl,
N-((C₇-C₁₆)-Aralkyloxy-(C₁-C₁₀)alkyl)carbamoyl,
N-(C₁-C₁₀)-Alkyl-N-((C₁-C₁₀)-Alkoxy(C₁-C₁₀)alkyl)carbamoyl,
N-(C₁-C₁₀)-Alkyl-N-((C₆-C₁₆)-Aryloxy-(C₁-C₁₀)alkyl)carbamoyl,
N-(C₁-C₁₀)-Alkyl-N-((C₇-C₁₆)-Aralkyloxy-(C₁-C₁₀)alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)arylcarbamoyloxy, N-(C₁-C₁₀)-Alkyl-N-(C₇-C₁₆)-Aralkylcarbamoyloxy N-((C₁-C₁₀)-Alkoxy-(C₁-C₁₀)alkyl)carbamoyloxy,
N-((C₆-C₁₆)-Aryloxy-(C₁-C₁₀)alkyl)carbamoyloxy,
N-((C₇-C₁₆)-Aralkyloxy-(C₁-C₁₀)alkyl)carbamoyloxy,
N-(C₁-C₁₀)-Alkyl-N-((C₁-C₁₀)-Alkoxy-(C₁-C₁₀)alkyl)carbamoyloxy,
N-(C₁-C₁₀)-Alkyl-N-((C₆-C₁₆)-Aryloxy-(C₁-C₁₀)alkyl)carbamoyloxy,
N-(C₁-C₁₀)-Alkyl-N-((C₇-C₁₆)-Aralkyloxy-(C₁-C₁₀)alkyl)carbamoyloxy,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-(C₁-C₁₀)-Alkyl-N-(C₇-C₁₆)-Aralkylamino, N-(C₁-C₁₀)-Alkyl-N-(C₆-C₁₂)-Arylamino, (C₁-C₁₂)-Alkoxy-amino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)alkylamino-(C₁-C₁₀)alkyl, N,N-Di(C₁-C₁₀)alkylamino-(C₁-C₁₀)alkyl, (C₃-C₈)cycloalkylamino-(C₁-C₁₀)alkyl,
(C₁-C₁₂)-Alkylmercapto, (C₁-C₁₂)-Alkylsulfinyl, (C₁-C₁₂)-Alkylsulfonyl,
C₆-C₁₆)-Arylmercapto, (C₆-C₁₆)-Arylsulfinyl, C₆-C₁₆)-Arylsulfonyl,
(C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl,(C₃-C₈)-cycloalkylsulfamoyl,
N-(C₆-C₁₆)-Arylsulfamoyl,
N-(C₇-C₁₆)-Aralkylsulfamoyl,
N-(C₁-C₁₀)-Alkyl-N-(C₆-C₁₆)-Arylsulfamoyl,
N-(C₁-C₁₀)-Alkyl-N-(C₇-C₁₆)-Aralkylsulfamoyl,
(C₁-C₁₀)-Alkyl-sulfonamido,
N-((C₁-C₁₀)alkyl)-(C₁-C₁₀)alkylsulfonamido, (C₇-C₁₆)-Aralkylsulfonamido,
N-((C₁-C₁₀)alkyl-(C₇-C₁₆)-Aralkylsulfonamido,
oder
R⁸ und R⁹ auch über 3 - 6 C-Atome verknüpft sind, wobei -NR⁸R⁹ einen heterocyclischen Ring bilden, mit N als Heteroatom,
oder
R⁹ in der Bedeutung von R⁶ steht,
und für [C-U]ᵣ-D-W die Bedeutung -SO₂H ausgenommen ist, sowie
R⁴ eine (C₁-C₁₀)-Alkylgruppe, die gegebenenfalls mit Hydroxy, (C₁-C₆)-Alkoxy substituiert sein kann, Aldehyd-, Imino-, N(C₁-C₈)-Alkylimino-, Acetal-, ein Thioacetal, eine cyclische Acetalgruppe, eine cyclische Thioacetalgruppe oder eine andere der Aldehydgruppe äquivalente Gruppe oder - CH₂OR⁵ bedeutet, worin
R⁵ Wasserstoff oder den Rest einer physiologisch verträglichen, vorzugsweise in der Leber unter physiologischen Bedingungen abspaltbaren Alkoholschutzgruppe bedeutet,
und
n 0 oder 1,
f 1 bis 8, vorzugsweise 1 bis 5,
g 0, 1 bis (2f+ 1) und
x 0 bis 8, vorzugsweise 0 oder 1 bedeuten.

3. Verbindungen der allgemeinen Formel I, nach den Ansprüchen 1 oder 2 worin
X eine Einfachbindung und Y -SO₂- oder -CO- oder
X -CO- und Y -SO₂-, -CO- oder -CONR⁹ bedeutet, wobei
R¹, R² und R³ gleich oder verschieden sind und Wasserstoff, (C₁-C₆)-Alkyl, (C₁-C₆)-Alkoxy, Halogen, insbesondere Fluor und Chlor, Hydroxy, Amino, Hydroxy-(C₁-C₄)-alkyl,
R⁶ Wasserstoff, (C₁-C₂₀)-Alkyl oder eine N-Schutzgruppe wie Acyl, (C₁-C₂₀)-Alkanoyl, (C₁-C₂₀)-Alkenoyl, (C₁-C₂₀)-Alkinoyl, (C₇-C₂₀)-Aralkanoyl, (C₆-C₁₈)-Aroyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein physiologisch verwendbares Kation, wie Na⊕, K⊕, Mg²⊕, Ca²⊕, Al³⊕ oder ein Ammoniumion, ggf. 1-3fach substituiert mit (C₁-C₁₆)Alkyl, (C₁-C₁₆)-Hydroxyalkyl, (C₁-C₈)-Alkoxy-(C₁-C₁₆)-alkyl, Phenyl, Benzyl oder (C₁-C₁₆)-Alkyl, welches 1 bis 3-fach substituiert sein kann mit Hydroxy oder (C₁-C₈)-Alkoxy, oder ein Kation eines basischen Aminosäurederivates, wobei ein vorhandener Arylteil gegebenenfalls substituiert sein kann,
R⁷ einen Rest Y R⁸ bedeutet,
in welchem
Y in der obigen Bedeutung steht
und
R⁸ und R⁹ [C-U]ᵣ-D-W bedeuten, worin
C eine Bindung, oder
einen verzweigten oder unverzweigten aliphatischen (C₁-C₁₂)-Alkandiyl-rest, oder
einen verzweigten oder unverzweigten (C₂-C₁₂)-Alkendiyl-Rest, einen (C₂-C₁₂)-Alkindiyl-Rest oder einen (C₂-C₁₂)-Alkenindiyl-Rest bedeutet, der eine oder mehrere C-C-Mehrfachbindungen enthalten kann,
U eine Bindung oder
Wasserstoff oder einen Rest aus der Reihe folgender Heteroatomgruppierungen
-(CO)NR-, -NR(CO)-, -O-, -SO-, -SO₂-, worin R (C₁-C₃)Alkyl, (C₁-C₈)Alkanoyl, (C₇-C₁₀)Aralkanoyl, (C₆-C₁₂)Aroyl oder Wasserstoff bedeutet,
r 1 oder 2 ist,
D eine Bindung oder
Wasserstoff oder
einen verzweigten oder unverzweigten aliphatischen (C₁-C₈)-Alkandiyl-Rest, oder
einen verzweigten oder unverzweigten (C₂-C₈)-Alkendiyl-Rest oder (C₂-C₈)-Alkindiyl-Rest und
W Wasserstoff oder
einen (C₃-C₁₀)-cycloaliphatischen Alkyl-, Alkenyl-, Alkinyl- oder Alkeninyl-Rest oder
einen (C₆-C₁₆)-Arylrest oder einen 5- oder 6-gliedrigen Heteroarylrest, wobei mindestens eine der Variablen C oder D oder W nicht eine Bindung bedeuten und U nur dann in der Bedeutung einer Heteratomgruppierung steht, wenn C nicht eine Bindung bedeutet und
C, D und/oder W, sofern diese keine Bindung oder Wasserstoff bedeuten, vorzugsweise ihrerseits substituiert sind durch eine Kombination von bis zu 5 gleichen oder verschiedenen Substituenten aus der Reihe
Hydroxy, Halogen, Cyano, Trifluormethyl, Nitro, Carboxyl, (C₁-C₁₂)-Alkyl,(C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, - OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂) Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)alkyl)carbamoyl,
N-((C₆-C₁₆)-Aryloxy-(C₁-C₁₀)alkyl)carbamoyl,
N-((C₇-C₁₆)-Aralkyloxy-(C₁-C₁₀)alkyl)carbamoyl,
N-(C₁-C₁₀)-Alkyl-N-((C₁-C₁₀)-Alkoxy-(C₁-C₁₀)alkyl)carbamoyl,
N-(C₁-C₁₀)-Alkyl-N-((C₆-C₁₆)-Aryloxy-(C₁-C₁₀)alkyl)carbamoyl,
N-(C₁-C₁₀)-Alkyl-N-(C₇-C₁₆)-Aralkyloxy-(C₁-C₁₀)alkyl)carbamoyl,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-(C₁-C₅)-Alkyl-N(C₇-C₁₀)-Aralkylamino, N-(C₁-C₅)-Alkyl-N(C₆-C₁₂)-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)alkylamino-(C₁-C₁₀)alkyl, N,N-Di(C₁-C₁₀)alkylamino-(C₁-C₁₀)alkyl, (C₃-C₈)cycloalkylamino-(C₁-C₁₀)alkyl,
(C₁-C₁₂)-Alkylmercapto, (C₁-C₁₂)-Alkylsulfinyl, (C₁-C₁₂)-Alkylsulfonyl,
(C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
wobei die Reste, die einen Arylrest enthalten, ihrerseits am Aryl substituiert sein können mit 1, 2, 3, 4 oder 5 gleichen oder verschiedenen Substituenten aus der Reihe Wasserstoff, Hydroxy, Halogen, Cyano, Trifluormethyl, Nitro, Carboxyl, (C₁-C₁₂)-Alkyl,(C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)alkyl, (C₁-C₁₂)-Alkoxy-(C₁-C₁₂)alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂) Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)alkyl)carbamoyl,
N-((C₆-C₁₆)-Aryloxy-(C₁-C₁₀)alkyl)carbamoyl,
N-((C₇-C₁₆)-Aralkyloxy-(C₁-C₁₀)alkyl)carbamoyl,
N-(C₁-C₁₀)-Alkyl-N-((C₁-C₁₀)-Alkoxy-(C₁-C₁₀)alkyl)carbamoyl,
N-(C₁-C₁₀)-Alkyl-N-((C₆-C₁₆)-Aryloxy-(C₁-C₁₀)alkyl)carbamoyl,
N-(C₁-C₁₀)-Alkyl-N-((C₇-C₁₆)-Aralkyloxy-(C₁-C₁₀)alkyl)carbamoyl,
Amino, (C₁-C₁₂)-Alkylamino, Di-(C₁-C₁₂)alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-(C₁-C₅)-Alkyl-(C₇-C₁₀)-Aralkylamino, N-(C₁-C₅)-Alkyl-(C₆-C₁₂)-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)alkylamino-(C₁-C₁₀)alkyl, N,N-Di(C₁-C₁₀)alkylamino-(C₁-C₁₀)alkyl, (C₃-C₈)cycloalkylamino-(C₁-C₁₀)alkyl,
(C₁-C₁₂)-Alkylmercapto, (C₁-C₁₂)-Alkylsulfinyl, (C₁-C₁₂)-Alkylsulfonyl,
(C₆-C₁₆)-Arylmercapto, (C₆-C₁₆)-Arylsulfinyl, (C₆-C₁₆)-Arylsulfonyl,
(C₇-C₁₆)-Aralkylmercapto, (C₇-C₁₆)-Aralkylsulfinyl, (C₇-C₁₆)-Aralkylsulfonyl,
oder
R⁸ und R⁹ auch über 3 - 6 C-Atome verknüpft sind, wobei -NR⁸R⁹ einen heterocyclischen Ring bilden, mit N als Heteroatom,
oder
R⁹ in der Bedeutung von R⁶ steht,
und für [C-U]ᵣ-D-W die Bedeutung -SO₂H ausgenommen ist, sowie
R⁴ eine Methylgruppe oder - CH₂OR⁵ bedeutet, worin
R⁵ Wasserstoff oder den Rest einer physiologisch vertraglichen, vorzugsweise in der Leber unter physiologischen Bedingungen abspaltbaren Alkoholschutzgruppe bedeutet,
und
n 0 oder 1,
f 1 bis 8, vorzugsweise 1 bis 5,
g 0, 1 bis (2f+ 1) und
x 0 bis 8, vorzugsweise 0 oder 1 bedeuten.

4. Verbindungen der allgemeinen Formel I, nach einem oder mehreren der Ansprüche 1 bis 3 in der
A R³ und B -XNR⁶R⁷,
X eine Einfachbindung und Y -SO₂- oder CO bedeuten, oder
X CO und Y -SO₂ oder -CONR⁹ bedeuten, und
R¹, R² und R³ gleich oder verschieden sind und Wasserstoff oder (C₁-C₃)-Alkyl, (C₁-C₃)-Alkoxy, Hydroxy, Fluor oder Chlor,
R⁶ Wasserstoff, (C₁-C₁₂)-Alkyl oder eine N-Schutzgruppe wie Acyl, (C₁-C₁₈)-Alkanoyl, (C₁-C₁₈)-Alkenoyl, gegebenenfalls substituiertes Benzoyl, ein 1, 2, 3 oder 4-wertiges physiologisch verwendbares Kation, insbesondere Na⊕, K⊕, Ca²⊕, Mg²⊕, Al³⊕ oder ein Ammoniumion, ggf. 1-3 fach substituiert mit (C₁-C₁₂)-Alkyl, (C₁-C₁₂)-Hydroxyalkyl, (C₁-C₄)-Alkoxy-(C₁-C₁₀)-alkyl, Phenyl, Benzyl oder (C₁-C₁₂)-Alkyl, welches 1 bis 3-fach substituiert sein kann mit Hydroxy oder (C₁-C₈)-Alkoxy, wobei ein Arylrest gegebenenfalls substituiert sein kann oder ein Kation eines basischen Aminosäurederivatives,
in welchem
Y in der obigen Bedeutung steht
und
R⁸ [C-U]ᵣ-D-W bedeuten, worin
C eine Bindung oder einen (C₁-C₆)-Alkandiyl-Rest,
U eine Bindung oder
Wasserstoff oder -O- bedeutet,
r 1 ist,
D eine Bindung oder
Wasserstoff oder
einen unverzweigten aliphatischen (C₁-C₈)-Alkandiyl-Rest, und
W Wasserstoff, einen (C₆-C₁₂)-Arylrest oder einen 5- oder 6-gliedrigen Heteroarylrest, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und W, sofern W nicht Wasserstoff bedeutet, vorzugsweise seinerseits substituiert ist mit bis zu 3 gleichen oder verschiedenen Substituenten aus der Reihe Hydroxy, Halogen, Cyano, Trifluormethyl, Nitro, Carboxyl, (C₁-C₈)-Alkyl,(C₃-C₈)-Cycloalkyl, (C₆-C₁₂)-Aryl, (C₇-C₁₆)-Aralkyl, (C₃-C₈)-Alkenyl, (C₃-C₈)-Alkinyl, (C₁-C₈)-Alkoxy, (C₁-C₈)-Alkoxy-(C₁-C₈)alkyl, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g},
(C₁-C₈)-Alkoxycarbonyl, (C₆-C₁₂)-Aryloxycarbonyl, (C₇-C₁₄)-Aralkoxycarbonyl, (C₃-C₈)-Cycloalkoxycarbonyl,
(C₁-C₈)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)alkyl)carbamoyl,
N-((C₆-C₁₆)-Aryloxy-(C₁-C₁₀)alkyl)carbamoyl,
N-((C₇-C₁₆)-Aralkyloxy-(C₁-C₁₀)alkyl)carbamoyl,
N-(C₁-C₁₀)-Alkyl-N-((C₁-C₁₀)-Alkoxy-(C₁-C₁₀)alkyl)carbamoyl,
N-(C₁-C₁₀)-Alkyl-N-((C₆-C₁₆)-Aryloxy-(C₁-C₁₀)alkyl)carbamoyl,
N-(C₁-C₁₀)-Alkyl-N-((C₇-C₁₆)-Aralkyloxy-(C₁-C₁₀)alkyl)carbamoyl,
Amino, (C₁-C₈)-Alkylamino, Di-(C₁-C₈)alkylamino, (C₃-C₈)-Cycloalkylamino, N-(C₆-C₁₂)-Arylamino, N-(C₇-C₁₁)-Aralkylamino, N-(C₁-C₃)-Alkyl-N-(C₇-C₁₁)-Aralkylamino,
(C₁-C₁₀)-Alkanoylamino, (C₃-C₈)-Cycloalkanoylamino, (C₆-C₁₂)-Aroylamino, (C₇-C₁₆)-Aralkanoylamino, (C₁-C₁₀)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₆)-alkyl, (C₃-C₈)-Cycloalkanoylamino-(C₁-C₈)alkyl, (C₆-C₁₆)-Aroylamino-(C₁-C₆)-alkyl, (C₇-C₁₆)-Aralkanoylamino-(C₁-C₆)-alkyl,
(C₁-C₈)-Alkylmercapto, (C₁-C₈)-Alkylsulfinyl, (C₁-C₈)-Alkylsulfonyl, (C₆-C₁₂)-Arylmercapto, (C₆-C₁₂)-Arylsulfinyl, (C₆-C₁₂)-Arylsulfonyl, (C₇-C₁₄)-Aralkylmercapto, (C₇-C₁₄)-Aralkylsulfinyl, (C₇-C₁₄)-Aralkylsulfonyl,
wobei die Reste, die einen Arylrest enthalten, ihrerseits am Aryl substituiert sein können mit 1, 2, 3, 4 oder 5 gleichen oder verschiedenen Substituenten aus der Reihe Wasserstoff, Hydroxy, Halogen, Cyano, Trifluormethyl, Nitro, Carboxyl, (C₁-C₈)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₈)-Alkoxy, (C₁-C₆)-Alkoxy-(C₁-C₆)alkyl, (C₆-C₁₂)-Aryloxy, (C₇-C₁₆)-Aralkyloxy,
-O-[CH₂]_{X}C_{f}H_{(2f+1-g)}F_{g},
(C₁-C₁₂)-Alkoxycarbonyl, (C₃-C₈)-Cycloalkoxycarbonyl,
(C₁-C₁₂)-Alkylcarbonyloxy, (C₃-C₈)-Cycloalkylcarbonyloxy,
Carbamoyl, N-(C₁-C₈)-Alkylcarbamoyl, N,N-Di-(C₁-C₈)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₈)-Alkyl-N-(C₆-C₁₆)arylcarbamoyl, N-(C₁-C₈)-Alkyl-N-(C₇-C₆)-Aralkylcarbamoyl,
N-((C₁-C₆)-Alkoxy-(C₁-C₆)alkyl)carbamoyl,
N-((C₆-C₆)-Aryloxy-(C₁-C₆)alkyl)carbamoyl,
N-((C₇-C₁₆)-Aralkyloxy-(C₁-C₆)alkyl)carbamoyl,
N-(C₁-C₈)-Alkyl-N-((C₁-C₆)-Alkoxy-(C₁-C₆)alkyl)carbamoyl,
N-(C₁-C₈)-Alkyl-N-((C₆-C₁₆)-Aryloxy-(C₁-C₆)alkyl)carbamoyl,
N-(C₁-C₈)-Alkyl-N-((C₇-C₁₆)-Aralkyloxy-(C₁-C₆)alkyl)carbamoyl,
Amino, (C₁-C₆)-Alkylamino, Di-(C₁-C₆)alkylamino, (C₃-C₈)-Cycloalkylamino, N-(C₆-C₁₂)-Arylamino, N-(C₇-C₁₁)-Aralkylamino, N-(C₁-C₃)-Alkyl-(C₇-C₁₁)-Aralkylamino, N-(C₁-C₃)-Alkyl-(C₆-C₁₂)-Arylamino, (C₁-C₈)-Alkoxy-amino,
(C₁-C₈)-Alkanoylamino, (C₃-C₈)-Cycloalkanoylamino, (C₆-C₁₂)-Aroylamino, (C₇-C₁₂)-Aralkanoylamino, (C₁-C₈)-Alkanoyl-N-(C₁-C₆)-alkylamino, (C₃-C₈)-Cycloalkanoyl-N-(C₁-C₆)-alkylamino, (C₆-C₁₂)-Aroyl-N-(C₁-C₁₀)-alkylamino, (C₇-C₁₁)-Aralkanoyl-N-(C₁-C₆)-alkylamino,
(C₁-C₈)-Alkanoylamino-(C₁-C₄)-alkyl, (C₃-C₈)-Cycloalkanoylamino-(C₁-C₄)alkyl, (C₆-C₁₂)-Aroylamino-(C₁-C₄)-alkyl, (C₇-C₁₂)-Aralkanoylamino-(C₁-C₄)-alkyl,
R⁹ Wasserstoff, (C₁-C₆)-Alkyl, Phenyl; gegebenenfalls einfach substituiert, bedeutet, oder
R⁸ und R⁹ über (-CH₂-)ᵢ verknüpft sind, wobei -NR⁸R⁹ einen Cyclus bilden und i 3, 4, 5 und 6 bedeuten kann,
und für [C-U]ᵣ-D-W die Bedeutung -SO₂H ausgenommen ist, sowie
R⁴ Methyl oder -CH₂OR⁵ bedeutet, worin
R⁵ Wasserstoff oder den Rest einer physiologisch verträglichen, vorzugsweise in der Leber unter physiologischen Bedingungen abspaltbaren Alkoholschutzgruppe bedeutet,
und
n 0 oder 1,
f 1 bis 8, vorzugsweise 1 bis 5,
g 0, 1 bis (2f+ 1) und
x 0 bis 8, vorzugsweise 0 oder 1 bedeuten.

5. Verbindungen der allgemeinen Formel I, nach einem oder mehreren der Ansprüche 1 bis 4 in der
A R³ und B -X NR⁶R⁷,
X eine Einfachbindung und Y -SO₂- bedeutet, oder
X -CO- und Y -SO₂- oder -CONR⁹ bedeuten,
R¹, R² und R³ Wasserstoff,
R⁶ Wasserstoff, (C₁-C₁₂)-Alkyl oder eine Acylgruppe, wie (C₁-C₁₈)-Alkanoyl, (C₁-C₁₈)-Alkenoyl, gegebenenfalls substituiertes Benzoyl oder ein 1, 2 oder 3-wertiges physiologisch verwendbares Kation, insbesondere Na⊕, K⊕, Mg²⊕, Ca²⊕ oder ein Ammoniumion ggf. 1-3fach substituiert mit (C₁-C₁₂)-Alkyl, (C₁-C₁₂)-Hydroxyalkyl, (C₁-C₄)-Alkoxy-(C₁-C₈)-alkyl, Phenyl, Benzyl oder (C₁-C₁₂)-Alkyl, welches 1 bis 3-fach substituiert sein kann mit Hydroxy oder (C₁-C₄)-Alkoxy, wie z.B. NH₃⊕C(CH₂OH)₃ oder ein Kation eines basischen Aminosäurederivates,
R⁷ einen Rest Y R⁸, ausgenommen -SO₂H, bedeutet
in welchem
Y in der obigen Bedeutung steht und
R⁸ [C-U]ᵣ-D-W bedeutet, wobei
C eine Bindung oder (C₁-C₄)-Alkandiyl,
U eine Bindung, Wasserstoff oder
-O- bedeutet,
r 1 ist,
D eine Bindung, Wasserstoff oder
(C₁-C₄)-Alkandiyl,
W Wasserstoff oder
einen Phenylrest bedeutet, wobei mindestens eine der Variablen C oder D oder W nicht eine Bindung bedeuten und U nur dann in der Bedeutung von -O- steht, wenn C nicht eine Bindung bedeutet, und
W substituiert ist durch Wasserstoff, Fluor, Chlor, (C₁-C₆)-Alkyl, (C₃-C₈)-Cycloalkyl, (C₆-C₁₂)-Aryl, (C₇-C₁₆)-Aralkyl, (C₃-C₁₂)-Alkenyl, Phenyl, (C₁-C₆)-Alkoxy, Phenoxy, -O-[CH₂]_{X}C_{f}H_{(2f+1-g)}F_{g},
Carbamoyl, N-(C₁-C₁₀)-Alkylcarbamoyl, N,N-Di-(C₁-C₈)-alkylcarbamoyl, N-(C₃-C₈)-Cycloalkylcarbamoyl, N-Phenylcarbamoyl, N-(C₇-C₁₁)-Phenylalkylcarbamoyl, N-(C₁-C₈)-Alkyl-N-(C₆-C₁₆)phenylcarbamoyl, N-(C₁-C₈)-Alkyl-N-(C₇-C₁₁)-Phenylalkylcarbamoyl,
N-((C₁-C₁₀)-Alkoxy-(C₁-C₈)alkyl)carbamoyl,
N-(Phenoxy-(C₁-C₈)alkyl)carbamoyl,
N-((C₇-C₁₆)-Phenylalkyloxy-(C₁-C₈)alkyl)carbamoyl,
N-(C₁-C₈)-Alkyl-N-((C₁-C₆)-Alkoxy-(C₁-C₈)alkyl)carbamoyl,
N-(C₁-C₈)-Alkyl-N-((C₆-C₁₂)-Phenoxy-(C₁-C₈)alkyl)carbamoyl,
N-(C₁-C₈)-Alkyl-N-((C₇-C₁₆)-Phenylalkyloxy-(C₁-C₈)alkyl)carbamoyl,
(C₁-C₈)-Alkanoylamino, (C₃-C₈)-Cycloalkanoylamino, (C₆-C₁₂)-Phenylamino, (C₇-C₁₁)-Phenylalkanoylamino, (C₁-C₈)-Alkanoyl-N-(C₁-C₁₀)-alkylamino, (C₃-C₈)-Cycloalkanoyl-N-(C₁-C₆)-alkylamino,
Benzoyl-N-(C₁-C₁₀)-alkylamino, (C₇-C₁₁)-Phenylalkanoyl-N-(C₁-C₆)-alkylamino,
(C₁-C₁₀)-Alkanoylamino-(C₁-C₈)-alkyl, (C₃-C₈)-Cycloalkanoylamino-(C₁-C₈)alkyl, Phenylamino-(C₁-C₈)-alkyl, (C₇-C₁₁)-Phenylalkanoylamino-(C₁-C₈)-alkyl,
wobei die Reste, die einen Arylrest enthalten, ihrerseits am Aryl substituiert sein können mit 1, 2, 3, 4 oder 5 gleichen oder verschiedenen Substituenten aus der Reihe Hydroxy, Carboxyl, (C₁-C₄)-Alkoxy, Phenoxy, Benzyloxy, Fluor, Chlor, Trifluormethyl, O-[CH₂]ₓC_{f}H_{(2f+1-g)}Fg
(C₁-C₉)-Alkoxycarbonyl, Phenoxycarbonyl, (C₇-C₁₁)-Phenylalkoxycarbonyl, (C₃-C₈)-Cycloalkoxycarbonyl,
(C₁-C₁₂)Alkylcarbonyloxy, (C₃-C₈)-Cycloalkylcarbonyloxy, Benzoyloxy, (C₇-C₁₁)-Phenalkylcarbonyloxy,
(C₁-C₈)-Alkoxycarbonyloxy, (C₆-C₁₂)-Phenoxycarbonyloxy, (C₇-C₁₁)-Phenalkyloxycarbonyloxy, (C₃-C₈)-Cycloalkoxycarbonyloxy,
Carbamoyl, N-(C₁-C₆)-Alkylcarbamoyl, N,N-Di-(C₁-C₆)-alkylcarbamoyl, N-(C₃-C₈)-Cycloalkylcarbamoyl, N-Phenylcarbamoyl, N-(C₇-C₁₁)-Phenalkylcarbamoyl, Hydroxy-(C₁-C₄)-alkyl-carbamoyl, Acyloxy-(C₁-C₄)-alkyl-carbamoyl,
Carbamoyloxy, N-(C₁-C₆)-Alkylcarbamoyloxy, N,N-Di-(C₁-C₆)-alkylcarbamoyloxy, N-(C₃-C₈)-Cycloalkylcarbamoyl,
R⁹ Wasserstoff, (C₁-C₄)-Alkyl, Phenyl,
R⁴ -CH₂O R⁵, worin
R⁵ Wasserstoff, (C₁-C₁₂)-Alkyl, ggf. substituiertes Benzyl, (C₁-C₁₈)-Alkanoyl, (C₁-C₁₈)-Alkenoyl, ggf. substituiertes Benzoyl und
n 0,
f 1 bis 5,
g 0, 1 bis (2f + 1) und
x 0 oder 1
bedeuten.

6. Verbindungen der allgemeinen Formel I, nach einem oder mehreren der Ansprüche 1 bis 5 in der
A R³ und B -X NR⁶R⁷
X -CO-
R¹, R² und R³ Wasserstoff,
R⁶ Wasserstoff, (C₁-C₆)-Alkyl, (C₁-C₁₈)-Alkanoyl, (C₁-C₁₈)-Alkenoyl, Benzoyl oder ein 1 oder 2-wertiges physiologisch verwendbares Kation, insbesondere Na⊕, K⊕, Mg²⊕ oder Ca²⊕ oder ein Ammoniumion, ggf. 1-3 fach substituiert mit (C₁-C₈)-Alkyl, (C₁-C₈)-Hydroxyalkyl, (C₁-C₄)-Alkoxy-(C₁-C₈)-alkyl, Phenyl, Benzyl oder (C₁-C₈)-Alkyl, welches 1 bis 3-fach substituiert sein kann mit Hydroxy oder (C₁-C₄)-Alkoxy
R⁷ einen Rest Y R⁸, ausgenommen -SO₂H, bedeutet
in welchem
Y -SO₂- und
R⁸ [C-U]ᵣ-D-W bedeutet, wobei
C eine Bindung oder lineares
(C₁-C₄)-Alkandiyl,
U eine Bindung, Wasserstoff oder
-O- bedeutet,
r 1 ist,
D eine Bindung, Wasserstoff oder lineares
(C₁-C₄)-Alkandiyl,
W Wasserstoff oder
einen Phenylrest bedeutet, wobei mindestens eine der Variablen C oder D oder W nicht eine Bindung bedeuten und U nur dann in der Bedeutung von -O- steht, wenn C nicht eine Bindung bedeutet und
W substituiert ist durch Wasserstoff, Fluor, Chlor, (C₁-C₆)-Alkyl, (C₃-C₈)-Cycloalkyl, (C₇-C₁₆)-Phenylalkyl, Phenyl, (C₁-C₆)-Alkoxy, Phenoxy, O-[CH₂]_{X}C_{f}H_{(2f+1-g)}F_{g},
Carbamoyl, N-(C₁-C₁₀)-Alkylcarbamoyl, N,N-Di-(C₁-C₈)-alkylcarbamoyl, N-(C₃-C₈)-Cycloalkylcarbamoyl, N-Phenylcarbamoyl,
N-(C₇-C₁₁)-Phenylalkylcarbamoyl, N-(C₁-C₈)-Alkyl-N-(C₆-C₁₆)phenylcarbamoyl, N-(C₁-C₈)-Alkyl-N-(C₇-C₁₁)-Phenylalkylcarbamoyl,
N-((C₁-C₃)-Alkoxy-(C₁-C₄)alkyl)carbamoyl,
N-(Phenoxy-(C₁-C₈)alkyl)carbamoyl,
N-((C₇-C₁₆)-Phenylalkyloxy-(C₁-C₄)alkyl)carbamoyl,
N-(C₁-C₆)-Alkyl-N-((C₁-C₃)-Alkoxy-(C₁-C₄)alkyl)carbamoyl,
N-(C₁-C₆)-Alkyl-N-((C₆-C₁₂)-Phenoxy-(C₁-C₄)alkyl)carbamoyl,
N-(C₁-C₆)-Alkyl-N-((C₇-C₁₆)-Phenylalkyloxy-(C₁-C₄)alkyl)carbamoyl,
(C₁-C₈)-Alkanoylamino, (C₃-C₈)-Cycloalkanoylamino, (C₆-C₁₂)-Phenylamino, (C₇-C₁₁)-Phenylalkanoylamino, (C₁-C₈)-Alkanoyl-N-(C₁-C₆)-alkylamino, (C₃-C₈)-Cycloalkanoyl-N-(C₁-C₆)-alkylamino, Benzoyl-N-(C₁-C₆)-alkylamino, (C₇-C₁₁)-Phenylalkanoyl-N-(C₁-C₆)-alkylamino,
(C₁-C₁₀)-Alkanoylamino-(C₁-C₂)-alkyl, (C₃-C₈)-Cycloalkanoylamino-(C₁-C₂)alkyl, Benzoylamino-(C₁-C₂)-alkyl, (C₇-C₁₁)-Phenylalkanoylamino-(C₁-C₂)-alkyl,
wobei die Reste, die einen Arylrest enthalten, ihrerseits am Aryl substituiert sein können mit 1, 2, 3, 4 oder 5 gleichen oder verschiedenen Substituenten aus der Reihe Hydroxy, Carboxyl, (C₁-C₆)-Alkoxy, Phenoxy, Benzyloxy, Fluor, Chlor, Trifluormethyl,
(C₁-C₉)-Alkoxycarbonyl, Phenoxycarbonyl, (C₇-C₁₁)-Phenylalkoxycarbonyl, (C₃-C₈)-Cycloalkoxycarbonyl,
(C₁-C₁₂)Alkylcarbonyloxy, (C₃-C₈)-Cycloalkylcarbonyloxy, Benzoyloxy, (C₇-C₁₁)-Phenalkylcarbonyloxy,
Carbamoyl, N-(C₁-C₆)-Alkylcarbamoyl, N,N-Di-(C₁-C₆)-alkylcarbamoyl, N-(C₃-C₈)-Cycloalkylcarbamoyl, N-Phenylcarbamoyl, N-(C₇-C₁₁)-Phenalkylcarbamoyl, Hydroxy-(C₁-C₄)-alkyl-carbamoyl, Acyloxy-(C₁-C₄)-alkyl-carbamoyl,
Carbamoyloxy, N-(C₁-C₆)-Alkylcarbamoyloxy, N,N-Di-(C₁-C₆)-alkylcarbamoyloxy, N-(C₃-C₈)-Cycloalkylcarbamoyl,
R⁴ -CH₂O R⁵, worin
R⁵ Wasserstoff, (C₁-C₁₂)-Alkyl, ggf. substituiertes Benzyl, (C₁-C₁₈)-Alkanoyl, (C₁-C₁₈)-Alkenoyl, ggf. substituiertes Benzoyl und
n 0,
f 1 bis 5,
g 0, 1 bis (2f + 1) und
x 0 oder 1
bedeuten.

7. Verbindungen nach den Ansprüchen 1 bis 6, dadurch gekennzeichnet, daß es sich um physiologisch verwertbare Salze handelt, wobei die Salzbildung über R⁶ erfolgt.

8. Verbindungen nach den Ansprüchen 1 bis 7 gegen fibrotische Erkrankungen.

9. Verbindungen nach den Ansprüchen 1 bis 7 zur Anwendung als Fibrosuppressiva.

10. Arzneimittel, enthaltend mindestens eine Verbindung der Formel I gemäß Ansprüche 1 bis 7 und ggf. einen vertraglichen pharmazeutischen Träger.

11. Verwendung von Verbindungen der Formel I gemaß Ansprüche 1 bis 7 zur Behandlung von Störungen des Stoffwechsels von Collagen und collagenähnlichen Stoffen.

12. Verwendung von Verbindungen der Formel I gemaß Ansprüche 1 bis 7 zur Behandlung von fibrotischen Erkrankungen.

13. Verwendung von Verbindungen der Formel I gemaß Ansprüche 1 bis 7 zur Behandlung von Leberfibrose.

14. Verfahren zur Herstellung von Arzneimitteln zur Behandlung von fibrotischen Erkrankungen, dadurch gekennzeichnet, daß das Arzneimittel eine Verbindung der Formel I gemaß Ansprüche 1 bis 7 enthält.

15. Verfahren zur Herstellung von Verbindungen nach einem oder mehreren der Ansprüche 1 bis 7, in denen gemaß Formel I X für eine Einfachbindung steht und R¹ bis R⁹ die Bedeutung gemäß den Ansprüchen 1 bis 6 aufweisen, dadurch gekennzeichnet, daß man die Verbindung der Formel 1, 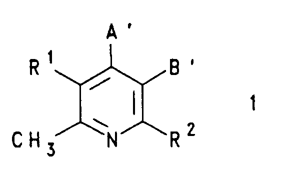 in der
A' = R³ und B' = NHR⁶ oder
A' = NHR⁶ und B' = R³ bedeutet
mit einem Sulfonsäure-Derivat der Formel 2, indem Z für eine Abgangsgruppe steht
Z - R⁷ 2
zu einer Verbindung gemaß Formel Ia umsetzt 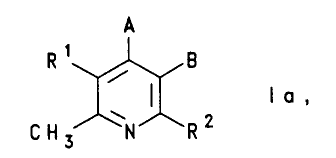 in der
A = R³ und B = -NHR⁶R⁷ oder
A = -NHR⁶R⁷ und B = R³
bedeutet und diese Verbindung ggf. zu einer Verbindung gemäß Formel Ia' oxidiert 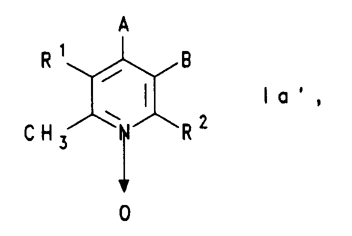 oder die Verbindung gemaß Formel Ia an der Methylgruppe monohalogeniert und anschließend mit HOR⁵ zu einer Verbindung gemaß Formel Ib umsetzt 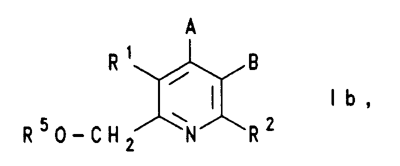 wobei diese anschließend ggf. zu ihrem Pyridin-N-oxid gemäß Formel Ib' 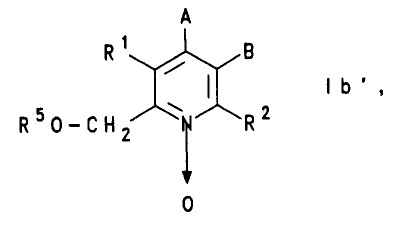 oxidiert wird, oder die Verbindung gemaß Formel Ia' mit einem Anhydrid im sauren pH-Bereich zu einer Verbindung gemaß Formel Ib umgesetzt wird.

16. Verfahren zur Herstellung von Verbindungen nach einem oder mehreren der Ansprüche 1 bis 7, in denen gemäß Formel I X für -CO- steht und R¹ bis R⁷ die Bedeutungen gemäß den Ansprüchen 1 bis 3 aufweisen, dadurch gekennzeichnet, daß man eine Verbindung der Formel 4 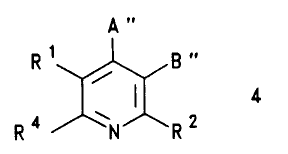 in der
A'' = R³ und B'' = -CO₂H oder
A'' = -CO₂H und B'' = R³ bedeutet
mit einer Verbindung der Formel 5
HNR⁶R⁷ 5
zu einer Verbindung Ic umsetzt 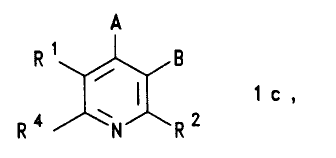 worin
A = R³ und B = -CO-NR⁶R⁷ oder
A = -CO-NHR⁶R⁷ und B = R³
bedeutet, und diese Verbindung ggf. zu ihrem Pyridin-N-oxid gemäß Formel Ic' 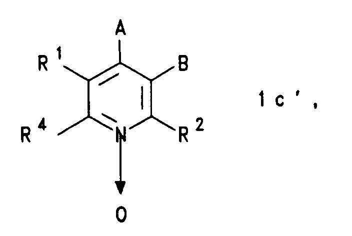 oxidiert.

17. Verfahren zur Herstellung von Verbindungen nach einem oder mehreren der Ansprüche 1 bis 7, in denen gemäß Formel I X für -CO steht und Y SO₂ bedeutet, und R¹ bis R⁹ die Bedeutung gemaß den Ansprüchen 1 bis 6 aufweisen, dadurch gekennzeichnet, daß man die Verbindung der Formel 6, 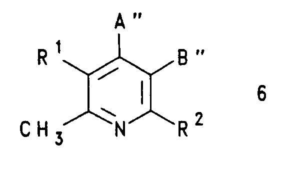 in der
A" = R³ und B" = -CO₂R oder
A" = -CO₂R und B" = R³ bedeutet, wobei R Wasserstoff und (C₁-C₅)-Alkyl bedeutet, mit einem
Halogenierungsmittel 7 für die Methylgruppe umsetzt, die halogenierte Verbindung mit einem Amin der Formel H₂NR⁶ und anschließend mit einem Sulfonsäure-Derivat der Formel 2, indem Z für eine Abgangsgruppe steht,
Z - R⁷ 2
und anschließend mit MOR⁵ zu einer Verbindung gemäß Formel Id umsetzt 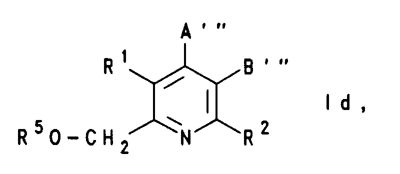 in der
A'" = R³ und B'" = -CONHR⁶R⁷ oder
A'" = -CONHR⁶R⁷ und B'" = R³
bedeutet und diese Verbindung ggf. zu einer Verbindung gemäß Formel Id' oxidiert 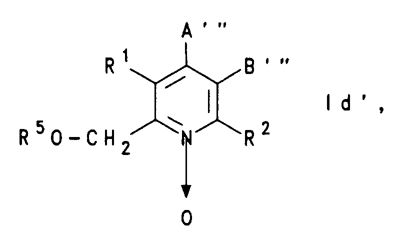 oder die Verbindung gemaß Formel 6 nach der Halogenierung zuerst mit MOR⁵, dann mit einem Amin der Formeln R⁶NH₂ oder HNR⁶R⁷ und anschließend mit einer Verbindung der Formel 2 umsetzt zu einer Verbindung gemäß Formel Id umsetzt 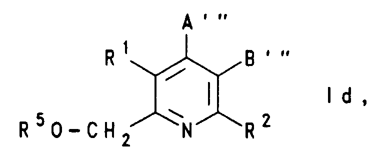 wobei diese anschließend ggf. zu ihrem Pyridin-N-oxid gemäß Formel Ib' 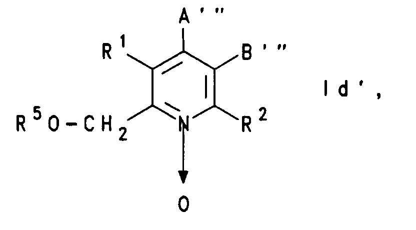 oxidiert wird.
